# EUROPEAN PATENT APPLICATION

(11) **EP 2 390 266 A1**
(43) Date of publication of application: **30.11.2011**
(21) Application number: 10290279.8
(22) Date of filing: 26.05.2010
(51) Int. Cl.: C07K 14/705, G01N 33/68, G06F 19/00

(54) **3-D Conformation of GLIC and allosteric modulation thereof**

(71) Applicant: Institut Pasteur, 75724 Paris Cédex 15 (FR); Centre National de la Recherche Scientifique, 75794 Paris Cedex 16 (FR)
(72) Inventor: Corringer, Pierre-Jean, 95220 Herblay (FR); Delarue, Marc, 75003 Paris (FR); Van Renterghem, Catherine, 75018 Paris (FR); Nury, Hughes, 75020 Paris (FR)
(74) Representative: Paris, Fabienne

(57) **Abstract**

The application relates to the ligand-gated ion channel of *Gloebacter violaceus* (GLIC), more particularly to its 3-D conformation. The application notably relates to crystal(s) comprising at least one GLIC monomer chain and a compound bound thereto, as well as to the biotechnological and medical applications thereof, including methods for screening and screening tools, such as a computer device system. The application describes GLIC allosteric site(s), which is(are) present in the transmembrane domain of each monomer of the pentameric GLIC. This(these) allosteric site(s) is(are), or contains(contain) binding site(s) for compounds which (allosterically) modulate the GLIC pore ion conductance, and therefore (allosterically) modulate cell excitability, more particularly neurotransmission.

SEQUENCE LISTING

<110>  INSTITUT PASTEUR
       C.N.R.S.

<120>  3-D conformation of GLIC and allosteric modulation thereof

<130>  B08840 - FP/BA

<160>  9

<170>  PatentIn version 3.3

<210>  1
<211>  359
<212>  PRT
<213>  Gloeobacter violaceus

<400>  1

Met Phe Pro Thr Gly Trp Arg Pro Lys Leu Ser Glu Ser Ile Ala Ala
1               5                   10                  15

Ser Arg Met Leu Trp Gln Pro Met Ala Ala Val Ala Val Val Gln Ile
            20                  25                  30

Gly Leu Leu Trp Phe Ser Pro Pro Val Trp Gly Gln Asp Met Val Ser
        35                  40                  45

Pro Pro Pro Pro Ile Ala Asp Glu Pro Leu Thr Val Asn Thr Gly Ile
    50                  55                  60

Tyr Leu Ile Glu Cys Tyr Ser Leu Asp Asp Lys Ala Glu Thr Phe Lys
65                  70                  75                  80

Val Asn Ala Phe Leu Ser Leu Ser Trp Lys Asp Arg Arg Leu Ala Phe
                85                  90                  95

Asp Pro Val Arg Ser Gly Val Arg Val Lys Thr Tyr Glu Pro Glu Ala
            100                 105                 110

Ile Trp Ile Pro Glu Ile Arg Phe Val Asn Val Glu Asn Ala Arg Asp
        115                 120                 125

Ala Asp Val Val Asp Ile Ser Val Ser Pro Asp Gly Thr Val Gln Tyr
    130                 135                 140

Leu Glu Arg Phe Ser Ala Arg Val Leu Ser Pro Leu Asp Phe Arg Arg
145                 150                 155                 160

Tyr Pro Phe Asp Ser Gln Thr Leu His Ile Tyr Leu Ile Val Arg Ser
                165                 170                 175

Val Asp Thr Arg Asn Ile Val Leu Ala Val Asp Leu Glu Lys Val Gly
            180                 185                 190

Lys Asn Asp Asp Val Phe Leu Thr Gly Trp Asp Ile Glu Ser Phe Thr
        195                 200                 205

Ala Val Val Lys Pro Ala Asn Phe Ala Leu Glu Asp Arg Leu Glu Ser
    210                 215                 220

Lys Leu Asp Tyr Gln Leu Arg Ile Ser Arg Gln Tyr Phe Ser Tyr Ile
225                 230                 235                 240

Pro Asn Ile Ile Leu Pro Met Leu Phe Ile Leu Phe Ile Ser Trp Thr
                245                 250                 255

Ala Phe Trp Ser Thr Ser Tyr Glu Ala Asn Val Thr Leu Val Val Ser
            260                 265                 270

Thr Leu Ile Ala His Ile Ala Phe Asn Ile Leu Val Glu Thr Asn Leu
        275                 280                 285

Pro Lys Thr Pro Tyr Met Thr Tyr Thr Gly Ala Ile Ile Phe Met Ile
    290                 295                 300

Tyr Leu Phe Tyr Phe Val Ala Val Ile Glu Val Thr Val Gln His Tyr
305                 310                 315                 320

Leu Lys Val Glu Ser Gln Pro Ala Arg Ala Ala Ser Ile Thr Arg Ala
                325                 330                 335

Ser Arg Ile Ala Phe Pro Val Val Phe Leu Leu Ala Asn Ile Ile Leu
            340                 345                 350

Ala Phe Leu Phe Phe Gly Phe
        355

<210>  2
<211>  317
<212>  PRT
<213>  Gloeobacter violaceus

<400>  2

Gly Gln Asp Met Val Ser Pro Pro Pro Pro Ile Ala Asp Glu Pro Leu
1               5                   10                  15

Thr Val Asn Thr Gly Ile Tyr Leu Ile Glu Cys Tyr Ser Leu Asp Asp
            20                  25                  30

Lys Ala Glu Thr Phe Lys Val Asn Ala Phe Leu Ser Leu Ser Trp Lys
        35                  40                  45

Asp Arg Arg Leu Ala Phe Asp Pro Val Arg Ser Gly Val Arg Val Lys
    50                  55                  60

Thr Tyr Glu Pro Glu Ala Ile Trp Ile Pro Glu Ile Arg Phe Val Asn

65                  70                  75                  80

Val Glu Asn Ala Arg Asp Ala Asp Val Val Asp Ile Ser Val Ser Pro
                85                  90                  95

Asp Gly Thr Val Gln Tyr Leu Glu Arg Phe Ser Ala Arg Val Leu Ser
            100                 105                 110

Pro Leu Asp Phe Arg Arg Tyr Pro Phe Asp Ser Gln Thr Leu His Ile
        115                 120                 125

Tyr Leu Ile Val Arg Ser Val Asp Thr Arg Asn Ile Val Leu Ala Val
    130                 135                 140

Asp Leu Glu Lys Val Gly Lys Asn Asp Asp Val Phe Leu Thr Gly Trp
145                 150                 155                 160

Asp Ile Glu Ser Phe Thr Ala Val Val Lys Pro Ala Asn Phe Ala Leu
                165                 170                 175

Glu Asp Arg Leu Glu Ser Lys Leu Asp Tyr Gln Leu Arg Ile Ser Arg
            180                 185                 190

Gln Tyr Phe Ser Tyr Ile Pro Asn Ile Ile Leu Pro Met Leu Phe Ile
        195                 200                 205

Leu Phe Ile Ser Trp Thr Ala Phe Trp Ser Thr Ser Tyr Glu Ala Asn
    210                 215                 220

Val Thr Leu Val Val Ser Thr Leu Ile Ala His Ile Ala Phe Asn Ile
225                 230                 235                 240

Leu Val Glu Thr Asn Leu Pro Lys Thr Pro Tyr Met Thr Tyr Thr Gly
                245                 250                 255

Ala Ile Ile Phe Met Ile Tyr Leu Phe Tyr Phe Val Ala Val Ile Glu
            260                 265                 270

Val Thr Val Gln His Tyr Leu Lys Val Glu Ser Gln Pro Ala Arg Ala
        275                 280                 285

Ala Ser Ile Thr Arg Ala Ser Arg Ile Ala Phe Pro Val Val Phe Leu
    290                 295                 300

Leu Ala Asn Ile Ile Leu Ala Phe Leu Phe Phe Gly Phe
305                 310                 315

<210>  3
<211>  127
<212>  PRT
<213>  Gloeobacter violaceus

<400>  3

Ser Arg Gln Tyr Phe Ser Tyr Ile Pro Asn Ile Ile Leu Pro Met Leu
1               5                   10                  15

Phe Ile Leu Phe Ile Ser Trp Thr Ala Phe Trp Ser Thr Ser Tyr Glu
            20                  25                  30

Ala Asn Val Thr Leu Val Val Ser Thr Leu Ile Ala His Ile Ala Phe
        35                  40                  45

Asn Ile Leu Val Glu Thr Asn Leu Pro Lys Thr Pro Tyr Met Thr Tyr
    50                  55                  60

Thr Gly Ala Ile Ile Phe Met Ile Tyr Leu Phe Tyr Phe Val Ala Val
65                  70                  75                  80

Ile Glu Val Thr Val Gln His Tyr Leu Lys Val Glu Ser Gln Pro Ala
                85                  90                  95

Arg Ala Ala Ser Ile Thr Arg Ala Ser Arg Ile Ala Phe Pro Val Val
            100                 105                 110

Phe Leu Leu Ala Asn Ile Ile Leu Ala Phe Leu Phe Phe Gly Phe
        115                 120                 125

<210>  4
<211>  23
<212>  PRT
<213>  Gloeobacter violaceus

<400>  4

Phe Ser Tyr Ile Pro Asn Ile Ile Leu Pro Met Leu Phe Ile Leu Phe
1               5                   10                  15

Ile Ser Trp Thr Ala Phe Trp
            20

<210>  5
<211>  25
<212>  PRT
<213>  Gloeobacter violaceus

<400>  5

Glu Ala Asn Val Thr Leu Val Val Ser Thr Leu Ile Ala His Ile Ala
1               5                   10                  15

Phe Asn Ile Leu Val Glu Thr Asn Leu
            20                  25

<210>  6
<211>  25
<212>  PRT
<213>  Gloeobacter violaceus

<400>   6

Met Thr Tyr Thr Gly Ala Ile Ile Phe Met Ile Tyr Leu Phe Tyr Phe
1               5                   10                  15

Val Ala Val Ile Glu Val Thr Val Gln
            20                  25

<210>   7
<211>   24
<212>   PRT
<213>   Gloeobacter violaceus

<400>   7

Ala Ser Ile Thr Arg Ala Ser Arg Ile Ala Phe Pro Val Val Phe Leu
1               5                   10                  15

Leu Ala Asn Ile Ile Leu Ala Phe
            20

<210>   8
<211>   118
<212>   PRT
<213>   Gloeobacter violaceus

<400>   8

Phe Ser Tyr Ile Pro Asn Ile Ile Leu Pro Met Leu Phe Ile Leu Phe
1               5                   10                  15

Ile Ser Trp Thr Ala Phe Trp Ser Thr Ser Tyr Glu Ala Asn Val Thr
            20                  25                  30

Leu Val Val Ser Thr Leu Ile Ala His Ile Ala Phe Asn Ile Leu Val
        35                  40                  45

Glu Thr Asn Leu Pro Lys Thr Pro Tyr Met Thr Tyr Thr Gly Ala Ile
    50                  55                  60

Ile Phe Met Ile Tyr Leu Phe Tyr Phe Val Ala Val Ile Glu Val Thr
65                  70                  75                  80

Val Gln His Tyr Leu Lys Val Glu Ser Gln Pro Ala Arg Ala Ala Ser
                85                  90                  95

Ile Thr Arg Ala Ser Arg Ile Ala Phe Pro Val Val Phe Leu Leu Ala
            100                 105                 110

Asn Ile Ile Leu Ala Phe
        115

<210>   9
<211>   5
<212>   PRT

<213>  Gloeobacter violaceus

<400>  9

Arg Tyr Pro Phe Asp
1               5

## Description

### FIELD OF THE INVENTION

The application relates to the ligand-gated ion channel of *Gloebacter violaceus* (GLIC), more particularly to its 3-D conformation. The application notably relates to crystal(s) comprising at least one GLIC monomer chain and a compound bound thereto, as well as to the biotechnological and medical applications thereof, including methods for screening and screening tools, such as a computer device system.

### BACKGROUND OF THE INVENTION

The cloning, expression and functional identification of the ligand-gated ion channel of *Gloebacter violaceus* (GLIC) has been described in Bocquet *et al.* 2007. It was expressed as a homo-oligomer in HEK 293 cells and *Xenopus oocytes,* generating a transmembrane cationic channel that is opened by extracellular protons and shows slow kinetics of activation, no desensitization and a single channel conductance of 8 pS.

GLIC has a homo-pentameric organization, possesses a compact structure, with the absence of the amino-terminal helix, a disulphide bridge and a large cytoplasmic domain. GLIC is an allosteric protein regulating cellular, more particularly neuronal, excitability through the opening of an intrinsic transmembrane channel. Agonist binding to extracellular site(s) shifts a closed conformation into an open one, allowing ions to diffuse down their electrochemical gradient. One of the characterizing feature of GLIC is that it is activated by protons, but does not desensitize, even at proton concentrations eliciting the maximal electrophysiological response (pH 4.5).

Two agonist-free structures of GLIC have been reported. The first structure is a crystal of GLIC diffracting at 2.90 Angstrom, which has been described in Bocquet *et al.* 2009 and in the Protein Data Bank (PDB; www.pdg.org) under accession number 3EAM. Another agonist-free GLIC structure, which diffracts at 3.10 Angstrom has been described in Hilf and Dutzler 2009 and in the PDB under accession number 3EHZ.

The application describes allosteric site(s) of GLIC. This(these) allosteric site(s) is(are) contained in the transmembrane domain of GLIC. The application accordingly describes and claims a crystal comprising a GLIC structure, more particularly at least a transmembrane sub-domain of a GLIC monomer chain, onto which at least one compound is bound in said allosteric site(s).

### SUMMARY OF THE INVENTION

The application describes crystals of GLIC, and identifies allosteric site(s), which is(are) present in the transmembrane domain of the pentameric GLIC, more particularly in the transmembrane domain of a (or each) monomer of the pentameric GLIC. This(these) allosteric site(s) is(are), or contains(contain), binding site(s) for compounds which (allosterically) modulate the GLIC pore ion conductance, and which therefore (allosterically) modulate cell excitability, more particularly neurotransmission.

The application relates to the subject matter described, illustrated and claimed. The application relates more particularly to crystals comprising at least one GLIC monomer chain, and at least one compound bound thereto. Said at least one bound compound is bound to the transmembrane domain of said at least one GLIC monomer chain, more particularly to a transmembrane sub-domain comprising the sequence extending from the first N-terminal amino acid of the transmembrane M1 segment to the last C-term amino acid of the transmembrane segment M4 of said at least one GLIC monomer chain. Said at least one bound compound is in a cavity located between the M1 and M3 segments of said at least one GLIC monomer chain, more particularly between the M1, M3 and M4 segments thereof, still more particularly between the M1, M3, M4 and M2 segments thereof (even still more particularly the intra-subunit cavity shown in the Figures, for example the Figure(s) 1C and/or 6A and/or 6B).

This cavity is, or contains, allosteric binding site(s), which (allosterically) modulates(modulate) the GLIC pore ion conductance, and therefore (allosterically) modulates(modulate) the cell excitability, more particularly neurotransmission. Illustrative crystals are provided, wherein said at least one bound compound in the anesthetics desflurane or propofol, and which diffract at 3.4 or 3.5 Angstrom, respectively. Crystallographic data are shown in Table 1 below (or in the worldwide Protein Data Bank (wwPDB) under PDB accession number 3EAM).

The application relates to screening means, including screening methods and tools, such as a computer system device, which enable to identify and/or develop a compound, which is a neurotransmission modulator, and/or which (allosterically) modulates neuronal cell hyperpolarization, and/or which (allosterically) modulates neuronal cell depolarization. The application also relates to a method for producing a pharmaceutical composition, medicament or drug involving a screening method and/or tool of the invention.

### BRIEF DESCRIPTION OF THE FIGURES

Some of the figures, to which the present application refers, are in color. The application as filed contains the color print-out of the figures, which can therefore be accessed by inspection of the file of the application at the patent office.

In the Figures and in the Examples, the amino acid positions are computed with respect to the 43-359 fragment of the GLIC monomer chain, *i.e.*, with respect to SEQ ID NO: 2, whereas in the body of the description and the claims, these positions are computed with respect to the full length (359aa) GLIC monomer chain, *i.e.*, with respect to SEQ ID NO: 1. For example, position 244 in the body of the description and in the claims is position 202 (*i.e.,* 244 minus 42) in the Figures and Examples. Please see Table 2 below.

**Table 2: correspondence of the amino acid positions**

| GLIC | Position computed with respect to the full length 359aa sequence of SEQ ID NO: 1(NP_927143) | Position computed with respect to the 43-359 fragment of SEQ ID NO:2 |
|---|---|---|
| β6-β7 loop | 161 | 119 |
| | 162 | 120 |
| | 163 | 121 |
| M1 segment | 243 | 201 |
| | 244 | 202 |
| | 247 | 205 |
| | 248 | 206 |
| M3 segment | 296 | 254 |
| | 297 | 255 |
| | 300 | 258 |
| | 301 | 259 |
| M4 segment | 345 | 303 |
| | 349 | 307 |
| M2 segment | 284 | 242 |

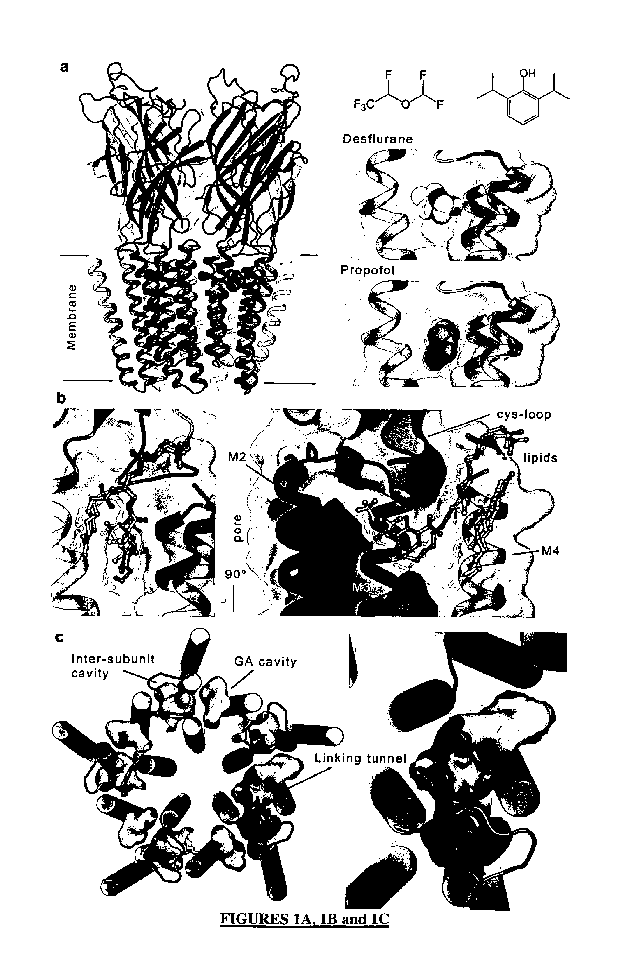

### Figures 1A, 1B, 1C: Propofol (PPF) and desflurane (DES) binding sites.

Figure **1A****.** General view of GLIC from the plane of the membrane in cartoon representation with a bound **GA** molecule in space-filling representation. The molecular surface is represented in the insets and coloured in yellow for the binding pocket.
Figure **1****B.** Cartoon and surface representation of the **GA** cavity seen from the membrane (left) and from the adjacent subunit (right, M1 removed for clarity) with propofol (green), desflurane (yellow) and lipids of the two structures (green and orange respectively) depicted as sticks. For this representation, Cα atoms were superimposed with a RMSD of 0.13 Å.
Figure **1C****.** Molecular surface of the **GA** intra-subunit cavities (yellow) and neighbouring intersubunit cavities (pink) for the whole pentamer. In one of the subunits, the communication tunnel between the two cavities is depicted in orange, and its constriction indicated by an arrow in the inset.
GA = General Anaesthetics.

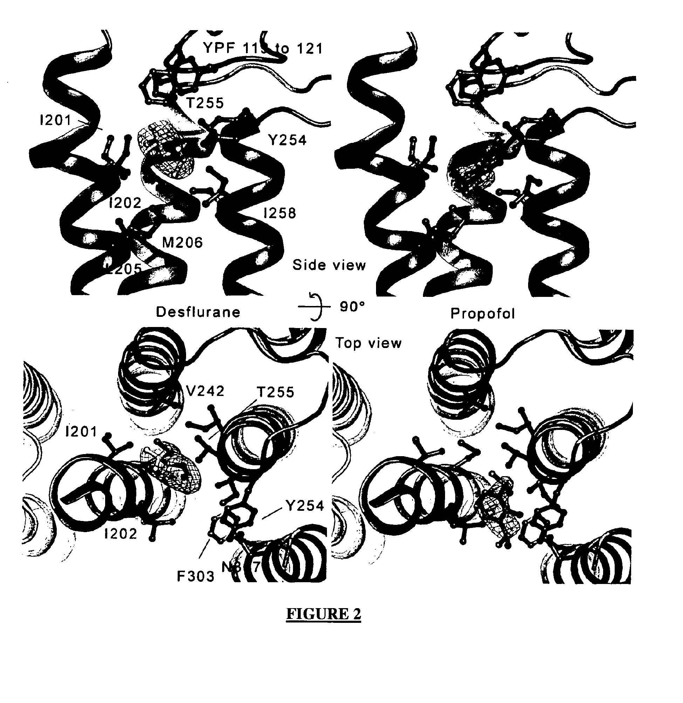

### Figure 2. Residues of the binding site.

Sites for desflurane (left panels) and propofol (right panels), viewed from the membrane (top panels with M4 helix removed), and from the extracellular domain (lower panels with extracellular removed). Residues bordering the pocket and contributing to binding are depicted as blue or red (mutated positions) sticks. Fourier difference maps Fo-Fc contoured at 3σ around the anaesthetics molecules are represented as a green mesh. Please note that, as indicated in Table 2 above, the positions indicated in Figure 2 have been computed 42aa less than those indicated in the body of the description and the claims. Please see Table 3 below for the correspondence between the positions indicated in the Figures and the positions indicated in the body of the description and the claims.

**Table 3:**

| **Figures (and examples)** | **Body of description and Claims** |
|---|---|
| Y119 | Y161 |
| P120 | P162 |
| F121 | F163 |
| I201 | I243 |
| I202 | I244 |
| L205 | L247 |
| M206 | M248 |
| V242 | V284 |
| Y254 | Y296 |
| T255 | T297 |
| I258 | I300 |
| I259 | I301 |
| F303 | F303 |
| N307 | N307 |

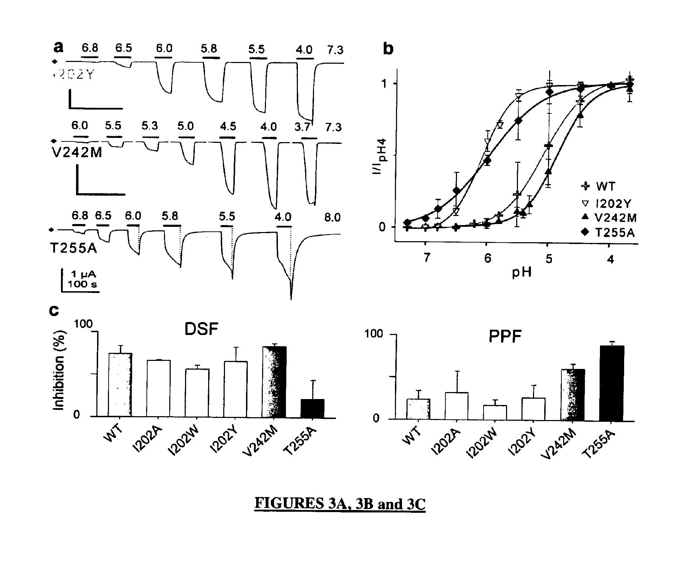

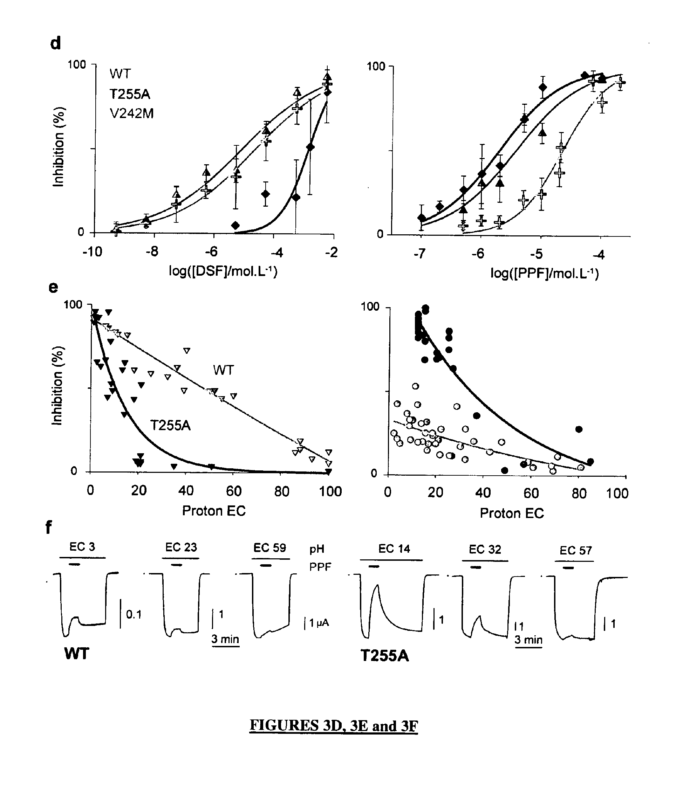

### Figures 3A, 3B, 3C, 3D, 3E and 3F. Electrophysiological characterization of binding site residues.

Figure **3A****.** Traces of currents evoked by 30 s applications of low extracellular pH separated by 30-60 s wash (from top to bottom, first trace: I202Y mutated GLIC; second line: V242M mutated GLIC; third trace: T255A mutated GLIC).
Figure **3****B.** Corresponding plots for currents normalized with respect to the value at pH 4. Mean ± s.d. of 4 to 12 cells per construct. Grey cross: wild-type GLIC (WT); yellow triangle having a vertex pointing to the bottom: I202Y mutated GLIC; blue triangle having a vertex pointing to the top: V242M mutated GLIC; red lozenge: T255A mutated GLIC.
Figure **3C**. Inhibition by 0.5 mM desflurane (DSF; left panel) or 10 µM propofol (PPF; right panel) applied for 60 s during the plateau of GLIC activation by a pH near EC₂₀ (EC₁₀₋₃₀). On the x axis, from left to right (for each panel): wild-type GLIC (WT); I202A mutated GLIC; I202W mutated GLIC; I202Y mutated GLIC; V242M mutated GLIC; T255A mutated GLIC.
Figure **3D**. Corresponding concentration-inhibition characteristics of desflurane (DSF; left panel) or propofol (PPF; right panel), for wild-type GLIC (WT; grey cross), for T255A mutated GLIC (T255A; red lozenge) and for V242M mutated GLIC (V242M; blue triangle).
Figure **3E**. Variation with the position in the proton concentration-response curve, of the effect of a single GA concentration (left panel: 0.5 mM desflurane (DSF); right panel: 10 µM propofol (PPF)) for wild-type GLIC (**WT**; grey triangle in the left panel, **grey circle in the right panel**) and for T255A mutated GLIC (**T255A**; red triangle in the left panel, **red circle in the right pane**l).
Figure **3F**. Current traces showing the effect of 10 µM propofol (PPF) on GLIC currents corresponding to proton EC₃, 23, 59 (wild-type GLIC: WT, left traces) or EC 14, 32, 57 (T255A mutated GLIC: T255A, right traces) on each cell.
   *Xenopus laevis* oocytes, holding potential -60 to -40 mV.
   For the amino acid positions indicated in Figures 3A-3F, please see Tables 2 and 3 above.

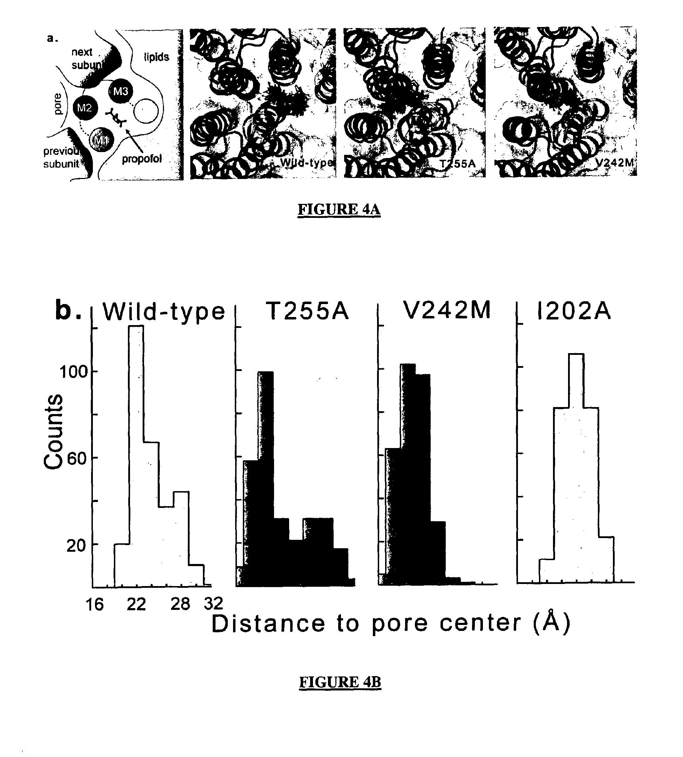

### Figures 4A, 4B. Molecular dynamics simulation of propofol (PPF) bound to GLIC.

Figure **4A**. View from the extracellular domain depicting propofol positions as green sticks, superposed at 0.5 ns intervals onto the starting (cartoon and molecular surface) and final (transparent cartoon) conformation of the protein, during the 30ns MD runs. The scheme on the left shows the positions of the various elements (clock-wise: previous subunit, pore, next sub-unit and lipids; in the centre: segments M2, M3, M4 and M1 surrounding propofol), and explains the color code (in a gradation of blue colors: segments M2, M3, M4 and M1 (clock-wise); in purple: previous and next subunits; in khaki: lipids; in bright green: propofol), and the three panels correspond to the wild-type, T255A and V242M systems (from left to right).
Figure **4****B.** Distance distribution between the propofol centre of mass and the pore centre. Same color code as in Figure 3 (grey = wild-type GLIC (WT); red = T255A mutated GLIC (T255A); blue = V242M mutated GLIC (V242M); yellow = I202A mutated GLIC (I202A)). The distribution is shifted closer to the pore centre for those showing an increased inhibition.
   For the amino acid positions indicated in Figures 4A-4B, please see Tables 2 and 3 above.

### Figures 5A, 5B, 5C, 5D. Anesthetics and lipids molecules in the X-ray structures

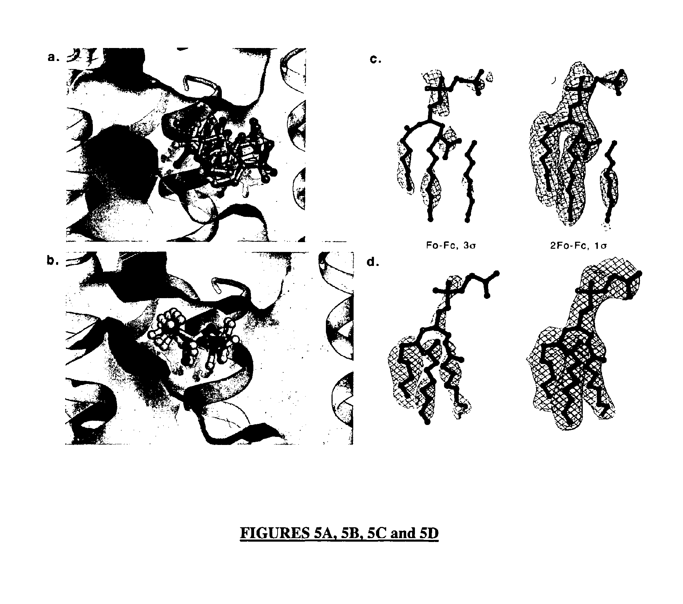

Figures **5A** and **5B**. Superimposed sticks representation of the five propofol and desflurane molecules illustrating the asymmetry of molecules modeled in the binding pocket. A single protein subunit is depicted as cartoon and molecular surface. Cαs were superimposed with a RMSD of 0.08 Å.
Figures **5C** and **5D**. Modeled lipids and Fourier difference Fo-Fc (model before adding the lipids, 3σ) or 2Fo-Fc map (final modem, 1σ) around them.

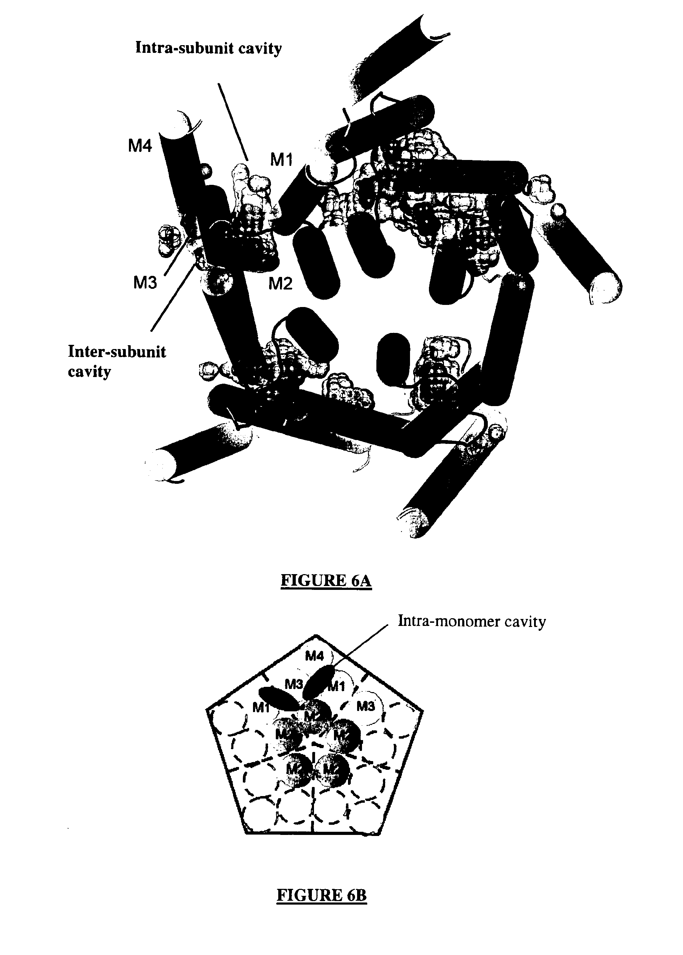

### Figures 6A, 6B. Cavities in a microsecond molecular dynamics run

Cα atoms of the core structure were used to superimpose the protein along the MD trajectory. Taking snapshots each 10 nanoseconds in our 1060 ns simulation run of GLIC at neutral pH, we filled the cavities of the transmembrane domain with pseudoatoms using the Hollow software (Ho and Gruswitz 2008). Density maps were derived from each pseudoatoms set, normalized to 1 and summed using CNS (Brunger *et al.* 1998). The resulting map, depicted as dots onto the protein snapshot closest to the mean protein structure, are contoured to reflect cavity existence in 50% (pale green) and 75% (dark green) of the snapshots. Dots corresponding to the pore lumen were removed for clarity.

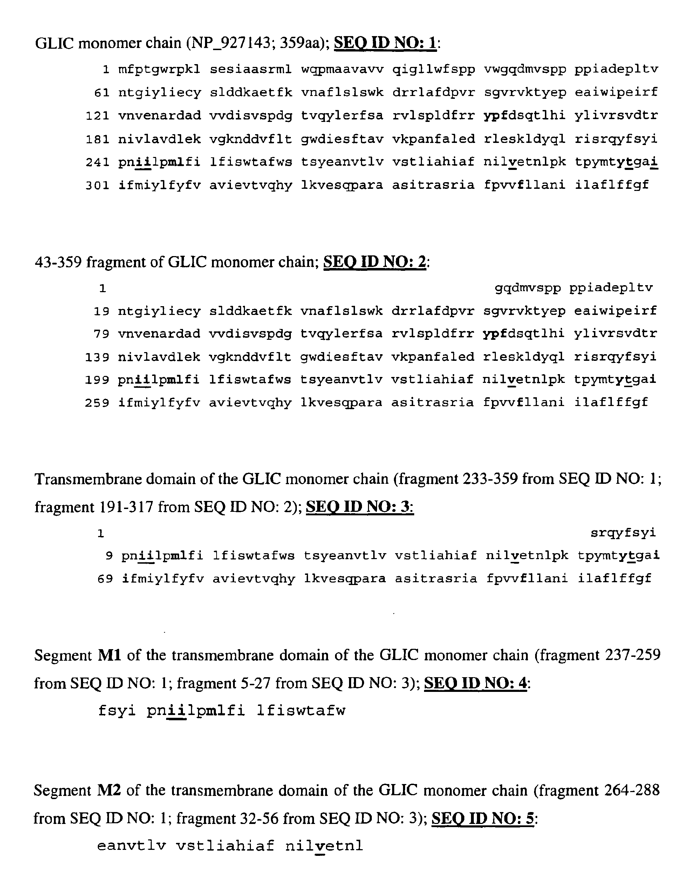

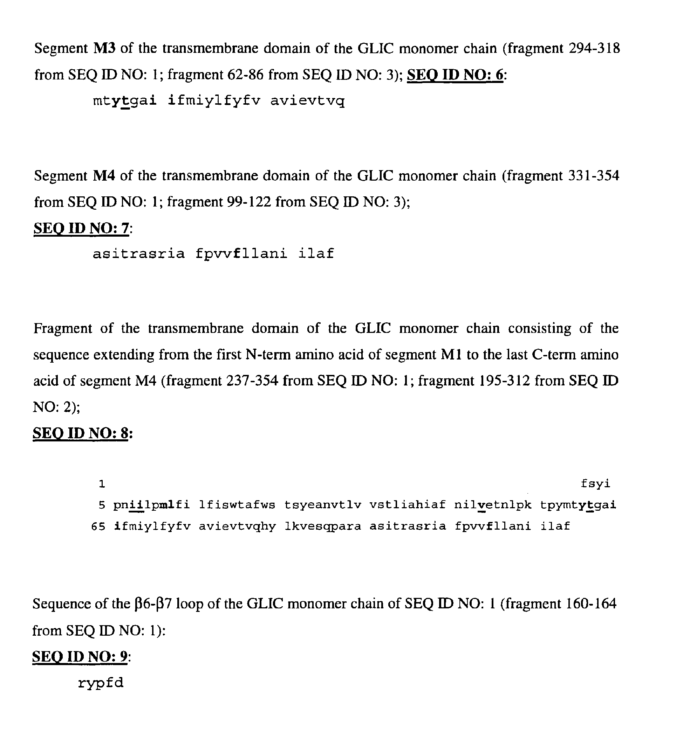

### Figure 7: illustrative GLIC amino acid sequences.

**Table 4:**

| **SEQ ID NO:** | |
|---|---|
| **1** | GLIC monomer chain (NP_927143; 359aa) |
| **2** | Fragment 43-359 from GLIC monomer chain of SEQ ID NO: 1 |
| **3** | Transmembrane domain of the GLIC monomer chain (fragment 233-359 from SEQ ID NO: 1; fragment 191-317 from SEQ ID NO: 2) |
| **4** | Segment **M1** of the transmembrane domain of the GLIC monomer chain (fragment 237-259 from SEQ ID NO: 1; fragment 5-27 from SEQ ID NO: 3) |
| **5** | Segment **M2** of the transmembrane domain of the GLIC monomer chain (fragment 264-288 from SEQ ID NO: 1; fragment 32-56 from SEQ ID NO: 3); |
| **6** | Segment **M3** of the transmembrane domain of the GLIC monomer chain (fragment 294-318 from SEQ ID NO: 1; fragment 62-86 from SEQ ID NO: 3) |
| **7** | Segment **M4** of the transmembrane domain of the GLIC monomer chain (fragment 331-354 from SEQ ID NO: 1; fragment 99-122 from SEQ ID NO: 3) |
| **8** | **Fragment of the transmembrane domain** of the GLIC monomer chain consisting of the sequence extending from the first N-term amino acid of segment M1 to the last C-term amino acid of segment M4 (fragment 237-354 from SEQ ID NO: 1; fragment 195-312 from SEQ ID NO: 2) |
| **9** | **β6**-**β7 loop** of the GLIC monomer chain of SEQ ID NO: 1 (fragment 160-164 from SEQ ID NO: 1) |

### DETAILED DESCRIPTION OF THE INVENTION

The present application relates to the subject-matter as defined in the claims as filed, as herein described and as herein illustrated.

In the application, unless specified otherwise or unless a context dictates otherwise, all the terms have their ordinary meaning in the relevant field(s).

### Complex or crystal of the invention:

The application relates to a complex, comprising:
- at least one monomer chain of the pentameric ligand-gated ion channel of *Gloebacter violaceus* (GLIC), or a fragment thereof, which has at least retained a transmembrane sub-domain of said GLIC monomer chain, and
- at least one compound bound to said at least one GLIC monomer chain or fragment thereof, more particularly to its transmembrane domain or to said transmembrane sub-domain.

The application more particularly relates to a crystal, comprising at least one of said complex.

Said transmembrane sub-domain is the fragment of the transmembrane domain of said GLIC monomer chain, which extends from the first N-terminal amino acid of the M1 segment to the last C-terminal amino acid of the segment M4 of said GLIC transmembrane domain.

A GLIC monomer chain fragment, which has at least retained said GLIC transmembrane sub-domain consists of, or comprises, said GLIC transmembrane sub-domain; more particularly, it consists of, or comprises, the full length transmembrane domain of said GLIC monomer chain.

An illustrative amino acid sequence of a GLIC transmembrane sub-domain is the sequence of SEQ ID NO: 8 (*cf.* Figure 7).

An illustrative amino acid sequence of a full length transmembrane domain of GLIC monomer chain is the sequence of SEQ ID NO: 3 (*cf.* Figure 7).

In an embodiment of the invention, the amino acid sequence of said at least one GLIC monomer chain consists of, or comprises, the sequence of SEQ ID NO: 1 (NP_927143 (359 amino acids), *cf.* Figure 7). The transmembrane sub-domain of said GLIC monomer chain of SEQ ID NO: 1 is the fragment of SEQ ID NO: 8 (fragment 237-354 from SEQ ID NO: 1; *cf.* Figure 7). Hence, according to a particular embodiment, said GLIC fragment, onto which said at least one compound is bound, consists of, or comprises at least, the sequence of SEQ ID NO: 8, more particularly the sequence of SEQ ID NO: 3 (full-length transmembrane domain of the GLIC monomer chain of SEQ ID NO: 1, *i.e.*, fragment 233-359 from SEQ ID NO: 1; *cf.* Figure 7), for example a fragment, which comprises, or consists of, the sequence of SEQ ID NO: 2 (fragment 43-359 of the GLIC monomer chain of SEQ ID NO: 1).

Said at least one bound compound is bound to said at least one GLIC monomer chain or fragment thereof in a particular location of said at least one GLIC monomer chain or fragment thereof. More particularly, said at least one bound compound is located in a particular cavity of the transmembrane domain or sub-domain of said at least one GLIC monomer chain or fragment thereof.

This particular location has been identified by the inventors as being a location containing allosteric site(s) of said GLIC. Indeed, a compound, which binds at this particularly location, can exert an allosteric effect, whereby it can modulate the ion pore conductance of said GLIC. In other words, a compound, which binds at said particularly location, can (allosterically) positively or negatively modulate the excitability of the cell expressing said GLIC, *e.g*., in the case of a neuronal cell, it can (allosterically) positively or negatively modulate neurotransmission.

Hence, said at least one bound compound is to be understood as being a compound, which is neither GLIC nor a characteristic (or GLIC-specific) fragment, portion or domain of GLIC. For example, said at least one bound compound is to be understood as being a compound, which is other than GLIC, other than GLIC monomer chain(s), other than the transmembrane domain(s) or sub-domain(s) of GLIC monomer chain(s), other than the extracellular domain(s) of GLIC monomer chain(s). Said at least one bound compound is also to be understood as being a compound other than a ligand, which binds to the extracellular domain of said GLIC.

In an embodiment of the invention, said at least one bound compound is, or comprises, at least one amphipatic or non-polar molecule, preferably at least one amphipatic molecule.

Said particular location is between the M1 and M3 segments of said transmembrane domain or sub-domain; *i.e.*, said at least one bound compound is bound to said at least one GLIC monomer chain or fragment thereof in a cavity, which is located between the M1 and M3 segments of the transmembrane domain or sub-domain of said at least one GLIC monomer chain. Hence, said M1 and M3 segments are transmembrane segments of the same GLIC monomer chain (or of the same GLIC fragment).

For example, said cavity (and hence said at least one bound compound) is located between:
- on a first side, the amino acids of said M1 segment, which are at **positions 243**, **244, 247 and 248** in the full length (359aa) amino acid sequence of said at least one GLIC monomer chain (*e.g*., the amino acids, which are at positions 243, 244, 247 and 248 in the sequence of SEQ ID NO: 1), and
- on a second side, the amino acids of said M3 segment, which are at **positions 296, 297, 300 and 301** in the full length (359aa) amino acid sequence of said at least one GLIC monomer chain (*e.g*., the amino acids, which are at positions 296, 297, 300 and 301 in the sequence of SEQ ID NO: 1),
and/or
said cavity (and hence said at least one bound compound) is located between:
- on a first side, the atoms of said M1 segment, which have the **atomic coordinates** that are given under the ATOM field for residue sequence numbers 201, 202, 205 and 206 in **Table 1**, or coordinates which differ from these coordinates by a root mean square deviation (rmsd) of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
   and
- on a second side, the atoms of said M3 segment, which have the atomic coordinates that are given under the ATOM field for residue sequence numbers 254, 255, 258 and 259 in Table 1, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;
and/or
said cavity (and hence said at least one bound compound) is located between:
- on a first side, the atoms of said M1 segment, which have the **atomic coordinates** that are given under the ATOM field for residue sequence numbers 201, 202, 205 and 206 in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and
- on a second side, the atoms of said M3 segment, which have the atomic coordinates that are given under the ATOM field for residue sequence numbers 254, 255, 258 and 259 in chain A, B, C, D or E of the PDB file accessible under identity number 3EAM as last modified 9 June 2009, respectively, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;
and/or
said cavity (and hence said at least one bound compound) is located between:
- on a first side, the atoms of said M1 segment, which have the **atomic coordinates** that are given under the ATOM field for residue sequence numbers 200, 201, 204 and 205 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and
- on a second side, the atoms of said M3 segment, which have the atomic coordinates that are given under the ATOM field for residue sequence numbers 253, 254, 257 and 259 in chain A, B, C, D or E of the PDB file accessible under identity number 3EHZ as last modified 25 August 2009, respectively, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

Said at least one bound compound can be bound to amino acid(s) of said M1 segment and/or amino acid(s) of said M3 segment. For example, it can be bound to:
- the amino acid of said M1 segment, which is at **position 244** in the full length (359aa) amino acid sequence of said at least one GLIC monomer chain (*e.g*., the amino acid, which is at position 244 in the sequence of SEQ ID NO: 1), and/or
- the atom of said M1 segment, which has the atomic coordinates that are given under the ATOM field for residue sequence number 202 in Table 1, or coordinates which differ from these coordinates by a root mean square deviation (rmsd) of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atom of said M1 segment, which has the atomic coordinates that are given under the ATOM field for residue sequence number 202 in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atom of said M1 segment, which has the atomic coordinates that are given under the ATOM field for residue sequence number 201 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;
and/or
- the amino acid of said M3 segment, which is at **position 297** in the full length (359aa) amino acid sequence of said at least one GLIC monomer chain (*e.g*., the amino acid, which is at position 297 in the sequence of SEQ ID NO: 1), and/or
- the atom of said M3 segment, which has the atomic coordinates that are given under the ATOM field for residue sequence number 255 in Table 1, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atom of said M3 segment, which has the atomic coordinates that are given under the ATOM field for residue sequence numbers 255 in chain A, B, C, D or E of the PDB file accessible under identity number 3EAM as last modified 9 June 2009, respectively, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atom of said M3 segment, which has the atomic coordinates that are given under the ATOM field for residue sequence number 254 in chain A, B, C, D or E of the PDB file accessible under identity number 3EHZ as last modified 25 August 2009, respectively, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;
and/or
- the amino acid of said M3 segment, which is at **position 300** in the full length (359aa) amino acid sequence of said at least one GLIC monomer chain (*e.g*., the amino acid, which is at position 300 in the sequence of SEQ ID NO: 1), and/or
- the atom of said M3 segment, which has the atomic coordinates that are given under the ATOM field for residue sequence number 258 in Table 1, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atom of said M3 segment, which has the atomic coordinates that are given under the ATOM field for residue sequence numbers 258 in chain A, B, C, D or E of the PDB file accessible under identity number 3EAM as last modified 9 June 2009, respectively, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atom of said M3 segment, which has the atomic coordinates that are given under the ATOM field for residue sequence number 257 in chain A, B, C, D or E of the PDB file accessible under identity number 3EHZ as last modified 25 August 2009, respectively, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

Alternatively or complementarily, said at least one bound compound can for example be bound to:
- the amino acid of said M1 segment, which is at **position 243** in the full length (359aa) amino acid sequence of said at least one GLIC monomer chain (*e.g*., the amino acid, which is at position 243 in the sequence of SEQ ID NO: 1), and/or
- the atom of said M1 segment, which has the atomic coordinates that are given under the ATOM field for residue sequence number 201 in Table 1, or coordinates which differ from these coordinates by a root mean square deviation (rmsd) of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atom of said M1 segment, which has the atomic coordinates that are given under the ATOM field for residue sequence number 201 in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atom of said M1 segment, which has the atomic coordinates that are given under the ATOM field for residue sequence number 200 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

In an embodiment of the invention, said particular location is between the M1, M3 and M4 segments of said transmembrane domain or sub-domain; *i.e.*, said at least one bound compound is bound to said at least one GLIC monomer chain or fragment thereof in a cavity, which is located between the M1, M3 and M4 segments of the transmembrane domain or sub-domain of said at least one GLIC monomer chain, *i.e.,* it is located between said M1 segment (or said M1 amino acids) on a first side, said M3 segment (or said M3 amino acids) on a second side, and, on a third side, the M4 segment of said at least one GLIC monomer chain. Hence, said M1, M3 and M4 segments are transmembrane segments of the same GLIC monomer chain (or of the same GLIC fragment).

For example, said cavity (and hence said at least one bound compound) is located between:
- said M1 segment (or said M1 amino acids) on a first side,
- said M3 segment (or said M3 amino acids) on a second side, and,
on a third side:
- the amino acids of said M4 segment, which are at **positions 345 and 349** in the full length (359aa) amino acid sequence of said at least one GLIC monomer chain (*e.g*., the amino acids, which are at positions 345 and 349 in the sequence of SEQ ID NO: 1),
and/or
- the atoms of said M4 segment, which have the **atomic coordinates** that are given under the ATOM field for residue sequence numbers 303 and 307 in **Table 1** or in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- the atoms of said M4 segment, which have the **atomic coordinates** that are given under the ATOM field for residue sequence numbers 302 and 306 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

Said at least one bound compound can be bound to amino acid(s) of said M1 segment and/or amino acid(s) of said M3 segment (please see above for illustrative M1 and/or M3 amino acid(s)).

In an embodiment of the invention, said particular location is between the M1, M3, M4 and M2 segments of said transmembrane domain or sub-domain; *i.e.*, said at least one bound compound is bound to said at least one GLIC monomer chain or fragment thereof in a cavity, which is located between the M1, M3, M4 and M2 segments of the transmembrane domain or sub-domain of said at least one GLIC monomer chain, *i.e.,* it is located between said M1 segment (or said M1 amino acids) on a first side, said M3 segment (or said M3 amino acids) on a second side, said M4 segment (or said M4 amino acids) on a third side, and, on a fourth side, the M2 segment of said at least one GLIC monomer chain.

Hence, said M1, M3, M4 and M2 segments are transmembrane segments of the same GLIC monomer chain (or of the same GLIC fragment).

For example, said cavity (and hence said at least one bound compound) is located between:
- said M1 segment (or said M1 amino acids) on a first side,
- said M3 segment (or said M3 amino acids) on a second side,
- said M4 segment (or said M4 amino acids) on a third side, and,
- on a fourth side:
   - the amino acid of said M2 segment, which is at **position 284** in the full length (359aa) amino acid sequence of said at least one GLIC monomer chain (*e.g*., the amino acid, which is at position 284 in the sequence of SEQ ID NO: 1),
   and/or
   - the atoms of said M2 segment, which have the **atomic coordinates** that are given under the ATOM field for residue sequence number 242 in **Table 1** or in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
   and/or
   - the atoms of said M2 segment, which have the **atomic coordinates** that are given under the ATOM field for residue sequence number 241 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

Said at least one bound compound can be bound to amino acid(s) of said M1 segment and/or amino acid(s) of said M3 segment (please see above for illustrative M1 and/or M3 amino acid(s)). Said at least one bound compound can be bound to amino acid(s) of said M2 segment. For example, it can be bound to:
- the amino acid of said M2 segment, which is at **position 284** in the full length (359aa) amino acid sequence of said at least one GLIC monomer chain (*e.g*., the amino acid, which is at position 284 in the sequence of SEQ ID NO: 1), and/or
- the atom of said M2 segment, which has the atomic coordinates that are given under the ATOM field for residue sequence number 242 in Table 1, or coordinates which differ from these coordinates by a root mean square deviation (rmsd) of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atom of said M2 segment, which has the atomic coordinates that are given under the ATOM field for residue sequence number 242 in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atom of said M2 segment, which has the atomic coordinates that are given under the ATOM field for residue sequence number 241 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

In an embodiment of the invention, said particular location is between the M1, M3, M4 segments and the β₆-β₇ loop of said at least one GLIC monomer chain; *i.e.*, said at least one bound compound is bound to said at least one GLIC monomer chain or fragment thereof in a cavity, which is located between the M1segment, the M3 segment, the M4 segment and the β₆-β₇ loop of said at least one GLIC monomer chain, *i.e.*, it is located between said M1 segment (or said M1 amino acids) on a first side, said M3 segment (or said M3 amino acids) on a second side, said M4 segment (or said M4 amino acids) on a third side, and, on a fourth side, the β₆-β₇ loop of said at least one GLIC monomer chain. Hence, said M1 segment, said M3 segment, said M4 segment and said β₆-β₇ loop are part of the same GLIC monomer chain (or fragment thereof).

For example, said cavity (and hence said at least one bound compound) is located between:
- said M 1 segment (or said M 1 amino acids) on a first side,
- said M3 segment (or said M3 amino acids) on a second side,
- said M4 segment (or said M4 amino acids) on a third side, and on a fourth side:
   - the amino acids of said β₆-β₇ loop, which are at **positions 161, 162 and 163** in the full length (359aa) amino acid sequence of said at least one GLIC monomer chain (*e.g*., the amino acids, which are at positions 161, 162 and 163 in the sequence of SEQ ID NO: 1),
   and/or
   - the atoms of said β₆-β₇ loop, which have the **atomic coordinates** that are given under the ATOM field for residue sequence numbers 119, 120 and 121 in **Table 1** or in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
   and/or
   - the atoms of said β₆-β₇ loop, which have the **atomic coordinates** that are given under the ATOM field for residue sequence numbers 118, 119 and 120 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

Said at least one bound compound can be bound to amino acid(s) of said M1 segment and/or amino acid(s) of said M3 segment (please see above for illustrative M1 and/or M3 amino acid(s)).

In an embodiment of the invention, said particular location is between the M1, M3, M4, M2 segments and the β₆-β₇ loop of said at least one GLIC monomer chain; *i.e.*, said at least one bound compound is bound to said at least one GLIC monomer chain or fragment thereof in a cavity, which is located between the M1, M3, M4, M2 segments and the β₆-β₇ loop of said at least one GLIC monomer chain, *i.e.*, it is located between said M1 segment (or said M1amino acids) on a first side, said M3 segment (or said M3 amino acids) on a second side, said M4 segment (or said M4 amino acids) on a third side, said M2 segment (or M2 amino acids) on a fourth side, and, on a fifth side, the β₆-β₇ loop of said at least one GLIC monomer chain.

Said at least one bound compound can be bound to amino acid(s) of said M1 segment and/or amino acid(s) of said M3 segment (please see above for illustrative M1 and/or M3 amino acid(s)), and/or amino acid(s) of said M2 segment (please see above for an illustrative M2 amino acid).

Figure 7 shows illustrative amino acid sequences of GLIC monomer chain (SEQ ID NO: 1), of fragments of GLIC monomer chain (SEQ ID NO: 8; SEQ ID NO: 3; SEQ ID NO: 2) of M1, M2, M3 and M4 segments (SEQ ID NO: 4-7) and of a GLIC β₆-β₇ loop (SEQ ID NO: 9); *cf.* also Table 2 above.

The amino acid sequence of said M1 segment may for example comprise or consist of the sequence of SEQ ID NO: 4.

The amino acid sequence of said M3 segment may for example comprise or consist of the sequence of SEQ ID NO: 6.

The amino acid sequence of said M4 segment may for example comprise or consist of the sequence of SEQ ID NO: 7.

The amino acid sequence of said M2 segment may for example comprise or consist of the sequence of SEQ ID NO: 5.

The amino acid sequence of said β₆-β₇ loop may for example comprise or consist of the sequence of SEQ ID NO: 9.

Table 1 below (and/or the data in the worldwide Protein Data Bank (wwPDB) under PDB accession number 3EAM) shows illustrative crystallographic data, more particularly illustrative atomic coordinates.

For example, in addition to or alternatively to said feature of amino acid sequence, a M1 segment of a complex or crystal of the invention may consist of, or comprise, atoms, which have:
- the **atomic coordinates** that are given under the ATOM field for residue sequence numbers 195 to 217 in **Table 1** or in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- the **atomic coordinates** that are given under the ATOM field for residue sequence numbers 194 to 216 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

For example, in addition to or alternatively to said feature of amino acid sequence, a M2 segment of a complex or crystal of the invention may consist of, or comprise, atoms, which have:
- the **atomic coordinates** that are given under the ATOM field for residue sequence numbers 222 to 246 in **Table 1** or in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- the **atomic coordinates** that are given under the ATOM field for residue sequence numbers 221 to 245 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

For example, in addition to or alternatively to said feature of amino acid sequence, a M3 segment of a complex or crystal of the invention may consist of, or comprise, atoms, which have:
- the **atomic coordinates** that are given under the ATOM field for residue sequence numbers 252 to 276 in **Table 1** or in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- the **atomic coordinates** that are given under the ATOM field for residue sequence numbers 251 to 275 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

For example, in addition to or alternatively to said feature of amino acid sequence, a M4 segment of a complex or crystal of the invention may consist of, or comprise, atoms, which have:
- the **atomic coordinates** that are given under the ATOM field for residue sequence numbers 289 to 312 in **Table 1** or in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- the **atomic coordinates** that are given under the ATOM field for residue sequence numbers 288 to 311 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

Figures 1 to 6, more particularly figures 1A, 1B, 1C, 2, 4A, 5A, 5B, 5C, 5D, 6A and 6B, illustrate the location of a cavity of the invention (and hence, of said at least one bound compound). Preferably, said cavity is an intra-monomer cavity (*cf.* Figures 1C and/or 6A and/or 6B).

It can be seen that said cavity (and hence said at least one bound compound) is located in the upper half of the transmembrane domain of said at least GLIC monomer chain or fragment thereof. The upper half of the transmembrane domain is the half portion of said transmembrane domain cut according to a plane perpendicular to the longitudinal axis of the ion conductance pore, which is the closest to the extracellular domain.

It can be observed that said at least one bound compound is in a cavity, which extends in a direction contained in a plane perpendicular to the longitudinal axis of the GLIC pore (the GLIC pore being the ion conductance pore that would be formed by the assembly of a pentameric GLIC formed by five of said at least one GLIC monomer chain, *e.g*., a GLIC pore located as illustrated by Figure 4A, and apparent in Figures 1C and/or 6A and/or 6B), preferably on a length of 9 Ångstrom or less, more preferably of 8.5 to 7.5 Ångstrom.

It can be observed that said at least one bound compound is in a cavity, which opens to the outside of said at least one GLIC monomer chain or fragment thereof, the outside of said at least one GLIC monomer chain or fragment thereof being the space that would be outside the pentameric GLIC formed by five of said at least one GLIC monomer chain (*e.g*., as illustrated by Figure(s) 1C and/or 6A and/or 6B).

In an embodiment of the invention, said at least one bound compound is in a cavity bordered by at least six, more particularly at least seven, still more particularly at least eight, even still more particularly nine hydrophobic amino acids or more of said at least one GLIC monomer chain or fragment thereof.

In a particular embodiment of the invention, said nine hydrophobic amino acids have particular locations, *i.e.*, said nine hydrophobic amino acids:
- are at positions 120, 121, 201, 202, 206, 242, 258, 259 and 303 in the full length amino acid sequence of said at least one GLIC monomer chain (*e.g*., in the GLIC monomer chain of SEQ ID NO: 1),
and/or
- have the atomic coordinates that are given under the ATOM field for residue sequence numbers 78, 79, 159, 160, 164, 200, 216, 217 and 261 in Table 1 or in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- have the atomic coordinates that are given under the ATOM field for residue sequence numbers 77, 78, 158, 159, 163, 199, 215, 216 and 260 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

Alternatively or complementarily to said location feature, said nine hydrophic amino acids can be P (Proline), F (Phenylalanine), I (Isoleucine), L (Leucine), V (Valine), C (Cystein), W (Tryptophan), M (Methionine), A (Alanine) or Y (Tyrosine), more particularly P (Proline), F (Phenylalanine), I (Isoleucine), L (Leucine), V (Valine), C (Cystein), W (Tryptophan) or M (Methionine), still more particularly P (Proline), F (Phenylalanine), I (Isoleucine), L (Leucine) or V (Valine).

In a particular embodiment of the invention, said nine hydrophobic amino acids:
- are P at position 120, F at position 121, I at position 201, I at position 202, L at position 206, V at position 242, I at position 258, I at position 259, F at position 303 (said positions being computed with respect to the full length amino acid sequence of said at least one GLIC monomer chain (*e.g*., the GLIC monomer chain of SEQ ID NO: 1)),
and/or
- are P, F, I, I, L, V, I, I and F, and respectively have the atomic coordinates that are given under the ATOM field for residue sequence numbers 78, 79, 159, 160, 164, 200, 216, 217 and 261 in Table 1 or in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- are P, F, I, I, L, V, I, I and F, and respectively have the atomic coordinates that are given under the ATOM field for residue sequence numbers 77, 78, 158, 159, 163, 199, 215, 216 and 260 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

Said at least six, more particularly at least seven, still more particularly at least eight hydrophobic amino acids may be selected among said nine particular hydrophobic amino acids.

In an embodiment of the invention, the atoms of said at least one GLIC monomer chain have the atomic coordinates that are given:
- in the ATOM field of Table 1 under atom numbers 1 to 2,525, or the coordinates, which are obtained by transforming said 1 to 2,525 atom coordinates with any one of the four transformation matrices of Table 1; or
- in the ATOM field of the PDB file accessible under identity number 3EAM as last modified 9 June 2009 under atom numbers 1 to 2,526, or 2,527 to 5,052, or 5,053 to 7,578, or 7,579 to 10,104, or 10,105 to 12,630; or
- in the ATOM field of the PDB file accessible under identity number 3EHZ as last modified 25 August 2009 under atom numbers 1 to 2,522, or 2,523 to 5,044, or 5,045 to 7,566, or 7,567 to 10,088, or 10,089 to 12,657;
or atomic coordinates which differ from these coordinates by a root mean square deviation (rmsd) of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

Since said particular location or cavity has been identified by the inventors to be a location or cavity, which contains allosteric site(s) of said GLIC, said at least one bound compound advantageously is a neurotransmission modulator, and/or a compound, which (allosterically) modulates neuronal cell depolarization, and/or a compound, which (allosterically) modulates neuronal cell hyperpolarization, and/or a compound, which modulates the ion conductance of GLIC.

In the application, the term "modulate" is herein intended as encompassing the meaning of the terms "inhibit", "stop", "block", "activate", "stimulate", "potentiate", more particularly the meaning of the terms "inhibit" or "stimulate".

Therefore, the term "negatively modulate" is herein intended as encompassing the meaning of the terms "inhibit", "stop", "block", more particularly the meaning of the term "inhibit"; and the term "positively modulate" is herein intended as encompassing the meaning of the terms "activate", "stimulate", "potentiate", more particularly the meaning of the term "stimulate".

The term "modulate" also encompasses the meaning of "allosterically modulate", more particularly the meaning of "allosterically stimulate" or "allosterically inhibit".

Indeed, said at least one compound, which is bound to said at least one GLIC monomer chain in the crystal of the invention, is bound to a site, which has been identified by the inventors as being an allosteric site of said at least one GLIC monomer.

It is therefore expected that compounds, which can bind to said at least one GLIC monomer chain in the same cavity as said at least one compound is bound in the crystal of the invention, are allosteric GLIC modulators.

The term "neuronal cell" is herein intended in accordance with its ordinary meaning in the field. It notably encompasses vertebrate neurons, more particularly mammal neurons, still more particularly human neurons. It encompasses neurons of the CNS and neurons of the PNS, more particularly neurons of the CNS, still more particularly brain neurons.

The term "neuronal cell" encompasses neurons at any cell cycle stage, *i.e.,* from neuronal pluripotent stem cells to differentiated neuronal cells, more particularly differentiated neuronal cells.

The modulation effect, which is obtained with said at least one compound of the invention, may *e.g*., be observed at the dendritic site of said neuronal cell.

According to a particular embodiment of the invention, said at least one compound is a compound, which (allosterically) modulates the ion conductance of a vertebrate anionic or cationic pentameric Ligand-Gated Ion Channel (pLGIC), more particularly of a gamma-aminobutyric acid receptor (GABA_{A}R) and/or a glycine receptor (GlyR) and/or a nicotinic acetylcholine receptor (AChR) and/or a serotonine receptor (5HT₃-R).

Said vertebrate pLGIC preferably is a mammal pLGIC, more particularly a human pLGIC.

Most usually, a compound, which negatively modulates the ion conductance of a vertebrate anionic pLGIC, such as a vertebrate anionic GABA_{A}R and/or GlyR, positively modulates the ion conductance of said at least one GLIC monomer chain, and, conversely, a compound, which positively modulates the ion conductance of a vertebrate anionic pLGIC, such as a vertebrate anionic GABA_{A}R and/or GlyR, negatively modulates the ion conductance of said at least one GLIC monomer chain.

Most usually, a compound, which negatively modulates the ion conductance of a vertebrate cationic pLGIC, such as a vertebrate anionic AChR and/or 5HT₃-R, negatively modulates the ion conductance of said at least one GLIC monomer chain, and conversely, a compound, which positively modulates the ion conductance of a vertebrate cationic pLGIC, such as a vertebrate anionic AChR and/or 5HT₃-R, positively modulates the ion conductance of said at least one GLIC monomer chain.

A compound, which positively modulates neurotransmission, and/or positively modulates neuronal cell depolarization, and/or negatively modulates neuronal cell hyperpolarization, and/or allosterically stimulates neuronal cell depolarisation, and/or allosterically inhibits neuronal cell hyperpolarization, is useful in the treatment and/or palliation and/or prevention of a disease involving a neurotransmission deficit or insufficiency.

A compound, which negatively modulates neurotransmission, and/or negatively modulates neuronal cell depolarization, and/or positively modulates neuronal cell hyperpolarization, and/or allosterically inhibits neuronal cell depolarisation, and/or allosterically stimulates neuronal cell hyperpolarization, is useful in the treatment and/or palliation and/or prevention of a disease involving excessive or undesired neurotransmission.

For example, said at least one bound compound can be or comprise:
- a steroid (*e.g*., a hormone), or a benzodiazepine, or an azapirone, or a piperazine and/or diphenylmethane (such as hydroxyzine or (±)-2-(2-{4-[(4-chlorophenyl)-phenylmethyl]piperazin-1-yl}ethoxy)ethanol), or an halogenated ether (*e.g*., a tetrafluoroethane, such as desflurane or 2-(difluoromethoxy)-1,1,1,2-tetrafluoro-ethane), or a propylphenol (such as propofol or 2,6-diisopropylphenol), or a carboxylated imidazole derivative (such as etomidate or ethyl 3-[(1*R*)-1-phenylethyl]imidazole-4-carboxylate), or 3-hydroxybutyric acid, or
- donepezil ((*RS*)-2-[(1-benzyl-4-piperidyl)methyl]-5,6-dimethoxy-2,3-dihydroinden-1-one), or galantamine ((4a*S*,6*R*,8a*S*)-5,6,9,10,11,12-hexahydro-3-methoxy-11-methyl-4a*H-*[1]benzofuro[3a,3,2-*ef*] [2] benzazepin-6-ol), or rivastigmine ((*S*)-*N*-Ethyl-*N-*methyl-3-[1-(dimethylamino)ethyl]-phenylcarbamate), or memantine (3,5-dimethyladamantan-1-amine), or
- an alcohol (ethanol, propanol, butanol, pentanol, hexanol, heptanol, octanol, nonanol, decanol), or
- a barbiturate (more particularly, pentobarbital).

Said at least one bound compound advantageously is a compound, which is useful for compensating or counter-acting a neurotransmission alteration, *e.g*., for compensating or counter-acting a disequilibrium in the balance of positive (*i.e.*, stimulatory) *versus* negative (*i.e.*, inhibitory) neurotransmission signals in an individual.

Alteration of the neurotransmission signal equilibrium can notably be observed in:
- diseases, such as neurodegenerative diseases (*e.g*., Alzheimer's disease; Parkinson's disease), metabolic encephalopathy(ies) (*e.g*., mercury or lead intoxication), epilepsy, amnesia, depression, schizophrenia, Attention Deficit Hyperactivity Disorder (ADHD), as well as in
- addictive disorders, such as nicotine or cocaine addiction.

Hence, in an embodiment of the invention, said at least one bound compound is, or comprises the active principle (or one the active principles) of, a medicament intended for the treatment and/or prevention and/or palliation of a disease or disorder of the nervous system, more particularly the CNS and/or the PNS, still more particularly the CNS, even still more particularly the brain.

Depending on the disequilibrium observed in the individual to be treated, said at least one bound compound can be a compound that (allosterically) stimulate neurotransmission or a compound that (allosterically) inhibits neurotransmission.

For example, for the treatment and/or prevention and/or palliation of diseases such as neurodegenerative diseases (*e.g*., Alzheimer's disease; Parkinson's disease) and metabolic encephalopathy(ies) (*e.g*., mercury or lead intoxication), allosteric stimulation of neurotransmission is desired, whereas allosteric inhibition of neurostransmission is desired for the treatment and/or prevention and/or palliation of diseases such as epilepsy, amnesia, depression, schizophrenia, Attention Deficit Hyperactivity Disorder (ADHD), for the treatment and/or prevention and/or palliation of undesired side-effect(s) such as emetic effects induced by chemotherapy, and for the treatment and/or prevention and/or palliation of addictive disorders, such as nicotine or cocaine addiction.

Alternatively, said at least one bound compound advantageously is a compound, which is useful for inducing a disequilibrium in the balance of positive *versus* negative transmission signals in an individual, more particularly for inducing a (medically-desired) disequilibrium in an individual, for example a temporary disequilibrium such as the one to induced for anaesthesia (*e.g*., as a preparatory surgical act).

Hence, in an embodiment of the invention, said at least one bound compound is, or comprises the active principle (or one the active principles) of, a drug intended for inducing a (medically-desired) disorder, more particularly a temporary disorder, of the nervous system.

In an embodiment of the invention, said at least one bound compound is a neurotransmission inhibitor, and/or a compound, which (allosterically) positively modulates, more particularly stimulates, neuronal cell hyperpolarization, and/or a compound, which (allosterically) negatively modulates, more particularly inhibits, neuronal cell depolarization, and/or a compound, which negatively modulates the ion conductance of GLIC.

For example, said at least one bound compound can be or comprise the active principle (or one of the active principles) of:
- an anesthetic, and/or
- a muscle relaxant, and/or
- a sedative, and/or
- a hypnotic, and/or
- an anxiolytic, and/or
- an antipsychotic, and/or
- an antidepressant, and/or
- an anticonvulsant, and/or
- an anti-epileptic, and/or
- an anti-emetic (*e.g*., an anti-emetic intended for the treatment and/or prevention and/or palliation of motion sickness and/or of the side-effects of medications such as opioids and chemotherapy), and/or
- an amnesic, and/or
- a medicament intended for the treatment and/or prevention and/or palliation of a chemical addiction (a chemical addiction that alters neurotransmission, *e.g*., nicotine addiction and/or cocaine addiction).

Illustrative compounds of this kind comprise steroids (*e.g*., a hormone), benzodiazepine, azapirone, piperazine and diphenylmethane (such as hydroxyzine), halogenated ethers (*e.g*., a tetrafluoroethane, such as desflurane), propylphenol (such as propofol), carboxylated imidazole derivatives (such as etomidate) and 3-hydroxybutyric acid.

For example, said anesthetic can be an anesthetic, which is intended for administration to a patient in need thereof by inhalation (such as an anesthetics, which is or comprises desflurane) or by injection, *e.g*., by intravenous injection (such as an anesthetics, which is or comprises propofol). Other illustrative anesthetics comprise an anesthetic, which is, or comprises, etomidate or 3-hydroxybutyric acid.

In an embodiment of the invention, said at least one bound compound is a neurotransmission stimulator, and/or a compound, which (allosterically) negatively modulates, more particularly inhibits, neuronal cell hyperpolarization, and/or a compound, which (allosterically) positively modulates, more particularly stimulates, neuronal cell depolarization, and/or a compound, which positively modulates the ion conductance of GLIC.

For example, said at least one bound compound is, or comprises the active principle (or one of the active principles) of:
- a nootropic drug, and/or
- an euphorisant, and/or
- a medicament intended for the treatment and/or prevention and/or palliation of toxic and/or metabolic encephalopathy(ies) (*e.g*., mercury or lead intoxication), and/or
- a medicament intended for the treatment and/or prevention and/or palliation of neurodegenerative disease(s) (*e.g*., Alzheimer's disease and/or Parkinson's disease), and/or
- a medicament intended for the treatment and/or prevention and/or palliation of diseases involving a cognitive deficit and/or the inability to focus attention (*e.g*., schizophrenia, more particularly the first stages of schizophrenia, and/or ADHD).

Illustrative compounds of this kind comprise donepezil ((*RS*)-2-[(1-benzyl-4-piperidyl)methyl]-5,6-dimethoxy-2,3-dihydroinden-1-one), galantamine ((4a*S*,6*R*,8a*S*)-5,6,9,10,11,12-hexahydro-3-methoxy-11-methyl-4a*H*- [1]benzofuro[3a,3,2-*ef*] [2] benzazepin-6-ol), rivastigmine ((*S*)-*N*-Ethyl-*N*-methyl-3-[1-(dimethylamino)ethyl]-phenylcarbamate), memantine (3,5-dimethyladamantan-1-amine).

In an embodiment of the invention, said at least one bound compound **is bound to:**
- the amino acid of said at least one GLIC monomer chain or fragment thereof, which is at **position 244 in the full length amino acid sequence** of said GLIC monomer chain (*e.g*., position 244 when computed with respect to SEQ ID NO: 1),
   and/or
- the atoms, which have the atomic coordinates that are given under the ATOM field for residue sequence number 202 in Table 1 or in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
   and/or
- the atoms, which have the atomic coordinates that are given under the ATOM field for residue sequence number 201 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;

In an embodiment of the invention, said **position 244** amino acid is, and/or said atoms are the atoms of, a hydrophobic amino acid (*e.g*., A, I, L, M, F, W, Y, V) or a polar amino acid (*e.g*., S, T, N, Q). In an embodiment of the invention, said hydrophobic or polar amino acid is I, T, A, Y, W, M or V, more particularly I, A, W, Y, still more particularly I or Y, even still more particularly I.

In an embodiment of the invention, said at least one bound compound **is bound to:**
- the amino acid of said at least one GLIC monomer chain or fragment thereof, which is at **position 297 in the full length amino acid sequence** of said at least one GLIC monomer chain (*e.g*., amino acid 297 when computed with respect to SEQ ID NO: 1),
   and/or
- the atoms, which have the atomic coordinates that are given under the ATOM field for residue sequence number 255 in Table 1 or in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
   and/or
- the atoms, which have the atomic coordinates that are given under the ATOM field for residue sequence number 254 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;

In an embodiment of the invention, said position 297 amino acid is, and/or said atoms are the atoms of, a hydrophobic amino acid (*e.g*., A, I, L, M, F, W, Y, V) or a polar amino acid (*e.g*., S, T, N, Q). In an embodiment of the invention, said hydrophobic or polar amino acid is I, T, A, Y, W, M or V, more particularly T or A, still more particularly T.

In an embodiment of the invention, said **is bound to:**
- the amino acid of said at least one GLIC monomer chain or fragment thereof, which is at **position 300 in the full length amino acid sequence** of said at least one GLIC monomer chain (*e.g*., amino acid 300 when computed with respect to SEQ ID NO: 1),
   and/or
- the atoms, which have the atomic coordinates that are given under the ATOM field for residue sequence number 258 in Table 1 or in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
   and/or
- the atoms, which have the atomic coordinates that are given under the ATOM field for residue sequence number 257 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

In an embodiment of the invention, said **position 300** amino acid is, and/or said atoms are the atoms of, a hydrophobic amino acid (*e.g*., A, I, L, M, F, W, Y, V) or a polar amino acid (*e.g*., S, T, N, Q). In an embodiment of the invention, said hydrophobic or polar amino acid is I, T, A, Y, W, M or V, more particularly I, A or M, still more particularly I.

In a particular embodiment of the invention, said at least one bound compound is bound to at least two, preferably the three, of the above-mentioned amino acids or corresponding atoms.

In an embodiment of the invention, said at least one bound compound **is further bound to:**
- the amino acid of said at least one GLIC monomer chain or fragment thereof, which is at **position 243 in the full length amino acid sequence** of said at least one GLIC monomer chain (*e.g*., amino acid 243 when computed with respect to SEQ ID NO: 1),
   and/or
- the atoms, which have the atomic coordinates that are given under the ATOM field for residue sequence number 201 in Table 1 or in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
   and/or
- the atoms, which have the atomic coordinates that are given under the ATOM field for residue sequence number 200 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

In an embodiment of the invention, said position 243 amino acid is, and/or said atoms are the atoms of, a hydrophobic amino acid (*e.g*., A, I, L, M, F, W, Y, V) or a polar amino acid (*e.g*., S, T, N, Q). In an embodiment of the invention, said hydrophobic or polar amino acid is I.

In an embodiment of the invention, said at least one bound compound **is further bound to:**
- the amino acid of said at least one GLIC monomer chain or fragment thereof, which is at **position 284 in the full length amino acid sequence** of said at least one GLIC monomer chain (*e.g*., amino acid 284 when computed with respect to SEQ ID NO: 1),
   and/or
- the atoms, which have the atomic coordinates that are given under the ATOM field for residue sequence number 242 in Table 1 or in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
   and/or
- the atoms, which have the atomic coordinates that are given under the ATOM field for residue sequence number 241 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1:5 Ångstrom or less.

In an embodiment of the invention, said position 284 amino acid is, and/or said atoms are the atoms of, a hydrophobic amino acid (*e.g*., A, I, L, M, F, W, Y, V) or a polar amino acid (*e.g*., S, T, N, Q). In an embodiment of the invention, said hydrophobic or polar amino acid is I, T, A, Y, W, M or V, more particularly V, A or M, still more particularly V.

In a particular embodiment of the invention, said at least one bound compound is further bound to the two of the above-mentioned amino acids or corresponding atoms.

In an embodiment of the invention, said at least one bound compound comprises, or consists of, atoms, which have the atomic coordinates that are given in the HETATM field of Table 1 for the residue named DES in said Table 1 or for the residue named PPL in said Table 1, or atomic coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

In an embodiment of the invention, said complex or crystal comprises at least five elements selected from the GLIC monomer chains and the fragments thereof, which have retained said GLIC transmembrane domain or sub-domain. Said at least five elements of GLIC monomer chain(s) and/or fragment(s) thereof are preferably assembled in a multimer.

In a particular embodiment of the invention, said at least five elements of GLIC monomer chain(s) and/or fragment(s) thereof comprises atoms, which have the atomic coordinates that are given:
- in the ATOM field of Table 1 under atom numbers 1 to 2,525 for one of said five elements, and, the coordinates, which are obtained by transforming said 1 to 2,525 atom coordinates with each one of the four transformation matrices of Table 1, for each one of said four other elements, respectively, or
- in the ATOM field of the PDB file accessible under identity number 3EAM as last modified 9 June 2009 under atom numbers 1 to 12,630, or
- in the ATOM field of the PDB file accessible under identity number 3EHZ as last modified 25 August 2009 under atom numbers 1 to 12,657;
or atomic coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

In a particular embodiment of the invention, each one of said at least five elements of GLIC monomer chain(s) and/or fragment(s) thereof contains at least one compound bound thereto in said location or cavity, said at least one bound compound having preferably the same chemical compound in each one of said at least five elements.

Alternatively or complementarily, said cavity in which said at least one bound compound is located, is (and hence, said at least one bound compound is located) as illustrated on Figure(s) 1A and/or 1B and/or 1C and/or 2 and/or 4A and/or 5A and/or 5B and/or 5C and/or 5D and/or 6A and/or 6B.

For example, in the embodiment(s) illustrated by Figure(s) 1A and/or 1B and/or 1C and/or 2 and/or 4A and/or 5A and/or 5B and/or 5C and/or 5D and/or 6A and/or 6B, the cavity, in which said at least one bound compound is located, is a cavity, which is contained in the transmembrane domain of the GLIC monomer chain.

This "allosteric" cavity can be seen as a cavity that is internal to said GLIC monomer chain (or fragment thereof), *i.e*., an intra-monomer cavity.

Said intra-monomer cavity has been identified by the inventors as being or containing a site(sites), where allosteric modulation of the GLIC ion conductance can be exerted by making a compound enter said cavity and bind to some site(s) of the wall(s) of the cavity. In a multimer composed of at least two of said GLIC monomer chain (or fragment thereof), there is one of said "allosteric" intra-monomer cavity per GLIC monomer chain (or fragment thereof).

Between these at least two GLIC monomer chain(s) and/or fragment(s) thereof, another cavity can be observed. This other cavity can be seen as an inter-monomer cavity. Figures 1C, 6A and 6B illustrate the location (and orientation) of an intra-monomer cavity and of an inter-monomer cavity.

For example, in the embodiment(s) illustrated by Figure(s) 1A and/or 1B and/or 1C and/or 2 and/or 4A and/or 5A and/or 5B and/or 5C and/or 5D and/or 6A and/or 6B, it can be observed that said inter-monomer cavity is located between M1 and M3 segments (more particularly between M1 and M3 segments, and a M2 segment and/or a β6-β7 loop), but that it is not located between M1, M3 and M4 segments. More particularly, said inter-monomer cavity is located between the M1 segment of a GLIC monomer chain and the M3 segment of another GLIC monomer chain. It can be observed that said intra-monomer cavity communicates with said inter-monomer cavity, preferably through a tunnel of 3 Ångstrom or less in length.

Said inter-monomer cavity can be or contain a site(sites), where allosteric modulation of the GLIC ion conductance can be exerted by making a compound enter said cavity and bind to some site(s) of the wall(s) of the cavity. This inter-monomer allosteric modulation can be exerted complementarily (or synergistically) to the allosteric modulation exerted through the intra-monomer cavity. This inter-monomer allosteric modulation can be exerted independently from the allosteric modulation exerted through the intra-monomer cavity. A compound as herein defined (*e.g*., an allosteric modulator) can be located in said inter-monomer cavity(ies), more particularly can be bound to said GLIC monomer chain or fragment thereof and be located in said inter-monomer cavity(ies). Said inter-monomer compound can be different or the same as the intra-monomer compound.

Hence, the application also relates to a complex or crystal, comprising at least two GLIC monomer chains or fragments thereof as herein defined and at least one compound bound to at least one of said at least two GLIC monomer chains or fragments thereof, wherein said at least one bound compound is in a cavity located between:
- the M1 segment of one of said at least two GLIC monomer chains or fragments thereof and
- the M3 segment of the other of said at least two GLIC monomer chains or fragments thereof,
more particularly between:
- the M1 segment of one of said at least two GLIC monomer chains or fragments thereof,
- the M3 segment of the other of said at least two GLIC monomer chains or fragments thereof, and
- the M2 segment of said other GLIC monomer chain or fragment thereof (*i.e*., the M3 and M2 segments are part of the same GLIC monomer chain or fragment thereof).

Such a complex or crystal may for example comprise two, three, four or five of said GLIC monomer chains or fragments thereof. It may comprise a bound compound in each of its inter-monomer cavities, or in only one or some of them. Such a complex may further comprise at least one compound bound in one or several of the intra-monomer cavity(ies) as herein defined and illustrated, said intra-monomer compound(s) being either the same as, or different from, preferably different from the compound(s) bound in the inter-monomer cavity(ies).

Figure 7 shows illustrative amino acid sequences of GLIC monomer chain, GLIC fragments and segments (segments M1 to M4).

Table 1 (or the data available in the wwPDB under PDB accession number 3EAM) shows illustrative atomic coordinates of a GLIC monomer chain or GLIC fragment that can be contained in a complex or crystal of the invention, more particularly atomic coordinates of illustrative M1, M3 and M2 segments.

In an embodiment of the invention, said crystal is obtainable by a method comprising:
i. forming a crystallization volume comprising a precipitant solution and said at least one GLIC monomer chain or fragment thereof, and
ii. forming crystal(s) of said at least one GLIC monomer chain or fragment thereof in said crystallization volume,
wherein said at least one compound is contacted with said at least one GLIC monomer chain before and/or during and/or after step ii., whereby crystal(s) of said at least one GLIC monomer chain or fragment thereof, which contains said at least one compound bound thereto as defined in the present application, are formed;
said method further comprising:
- collecting said crystal(s) of at least one GLIC monomer chain or fragment thereof containing said least one compound bound thereto; and
- optionally, freezing the collected crystal(s), preferably in a cryoprotectant solution (*e.g*., a solution containing glycerol), more preferably in a cryoprotectant solution containing said at least one compound.

This method of production of crystal is encompassed by the application.

The methods, conditions and means for implementation of the method should be selected and/or adjusted so that crystal(s) of said at least one GLIC monomer chain or fragment thereof is(are) formed at X-ray diffraction quality (*i.e*., crystal(s), which diffracts(diffract) at Ångstrom-level resolution, and preferably, which contains(contain) as few impurities as possible), while allowing said compound to bind and to remain bound to said at least one GLIC monomer chain or fragment thereof.

In an embodiment of the invention, said at least one compound can be contacted with said at least one GLIC monomer chain or fragment thereof before crystal formation, *e.g*., it can be added in the crystallization volume of i. (for example at a central part thereof) before crystal(s) of GLIC monomer chain(s) of ii. is(are) formed.

Such a contacting can notably be used when said at least one compound is a liquid compound, such as propofol, which does not easily evaporates, at least which does not evaporate at a temperature of 17-23°C, for example at a temperature of 20°C, under atmospheric pressure.

In an embodiment of the invention, said at least one compound can be contacted with said at least one GLIC monomer chain after crystal formation has begun, *e.g*., it can be made to diffuse (more particularly by vapour diffusion from the mother liquor) into crystal(s) of GLIC monomer chain(s) formed at any stage of step ii. Said contacting may be made as soon as crystal nucleation has begun, and/or after nucleation during crystal growth, and/or once crystals are fully formed.

Such a contacting can notably be used when said at least one compound is a compound, such as desflurane, which easily evaporates, at least which evaporates at a temperature of 17-23°C, for example at a temperature of 20°C, under atmospheric pressure.

Said precipitant solution comprises one or more precipitants, and optionally a buffer (such as a TRIS, MES, CHES, cacodylate, acetate, citrate buffer, or a combination thereof). The term "precipitant" is intended in accordance with its ordinary meaning in the field of crystallography. Illustrative precipitants notably comprise:
- salts, such as:
   - ammonium salts, such as ammonium thiocyanate,
   - calcium salts,
   - lithium salts,
   - magnesium salts,
   - potassium salts,
   - sodium salts;
- organic solvents, such as:
   - ethanol,
   - methanol, isopropanol,
   - acetone,
   - DMSO, dioxane, 2-propanol, acetonitrile, ethylene glycol, n-propanol, tertiary butanol, ethyl acetate, hexane-1,6-diol,
   - 1,3-propanediol, 1,4-butanediol, 1-propanol;
- long-chain polymers, such as polyethylene glycol (PEG), *e.g*., PEG 4000.

Any precipitant, or combination of precipitants, which are found appropriate by the person of ordinary skill in the art, can be used.

For example, the precipitant solution may contain PEG (*e.g*., PEG 4000), ammonium thiocyanate and sodium acetate.

When said at least one compound is an amphipatic or non-polar compound, or comprises an amphipatic or non-polar molecule, said crystallization solution preferably comprises one or more organic solvent(s), such as DMSO (*e.g*., up to 3% DMSO, for example 2% DMSO), to increase the solubility of said at least one compound.

For example, the precipitant solution may contain PEG (*e.g*., PEG 4000), ammonium thiocyanate, sodium acetate and DMSO (*e.g*., up to 3% DMSO, for example 2% DMSO).

The pH of said precipitant solution preferably is an acidic pH, notably a pH of 3 to 5, for example of 4.3 to 4.9, more particularly a pH of 4.5 to 4.7, for example of 4.6.

Methods, conditions and means for producing protein crystals are known to the person of ordinary skill in the art. A description of various methods, conditions and means can for example be found in the International Tables for Crystallography, 2006, volumes A to G, more particularly volume F, edited Sydney Hall and Brain McMahon (ISBN: 978-1-4020-4969-9; which are available in print or online from http://it.iucr.org/).

Such conditions and means notably comprise those of the crystallization by batch, dialysis (*e.g*., microdialysis) or vapour diffusion (*e.g*., sitting drop or hanging drop), more particularly vapour diffusion, still more particularly the hanging drop diffusion method. Suitable conditions may *e.g*., comprise temperature conditions that are suitable for crystallization, such as temperatures of 17-23°C, for example a temperature of 20°C.

Preferably, said at least one GLIC monomer chain or fragment thereof is contained in said crystallization volume at a high concentration, *e.g*., a concentration of at least 4 mg/mL.

In addition to said at least one GLIC monomer chain (or fragment thereof) and said at least one bound compound, said crystal may further comprise:
- molecules originating from the solution in which said crystal has been made, such as molecules of precipitant(s), of buffer, of water, as well as impurities; and/or
- molecules originating from the cell from which said at least GLIC monomer chain (or fragment thereof) has been isolated, such as lipid(s), *e.g*., lipid(s), which comprises atoms having the atomic coordinates shown under the HETATM field for residue name Q in Table 1.

In an embodiment of the invention, said crystal diffracts at 3.5 Ångstrom or less, for example at 3.5 or 3.4 Ångstrom.

In an embodiment of the invention, said crystal has a lattice in the C 1 2 1 space group and/or a unit cell having the following dimensions (at ± 2%):
a = 181.20; b = 132.37; c = 159.69; alpha = 90°; beta = 102.13° and gamma = 90°, or
a = 181.13; b = 132.78; c = 159.97; alpha = 90°; beta = 102.32° and gamma = 90°.

In an embodiment of the invention, the amino acid sequence of said at least one GLIC monomer chain is:
- the sequence of SEQ ID NO: 1 (illustrative full length GLIC monomer amino acid sequence) or
- a fragment thereof, which has retained at least the sequence of SEQ ID NO: 8 (transmembrane sub-domain consisting of fragment M1-M4 from SEQ ID NO: 1; *cf.* Figure 7), more particularly at least the sequence of SEQ ID NO: 3 (full length amino acid sequence of the transmembrane sequence of the GLIC monomer of SEQ ID NO: 1, *i.e.,* fragment 233-359 from NP_927143; *cf.* Figure 7), for example at least the sequence of SEQ ID NO: 2 (fragment 43-359 of the GLIC monomer of SEQ ID NO: 1; *cf.* Figure 7).

In an embodiment of the invention, said at least one GLIC monomer chain comprises its extracellular domain. In a particular embodiment, a ligand is bound to said extracellular domain. Said extracellular ligand may be different from the compound, which is bound to the transmembrane domain or sub-domain of said at least one GLIC monomer chain. Said extracellular ligand may be an agonist of the GLIC pentamer that contains five of said GLIC monomer chain, *i.e*., a compound, which can induce a shift in the conformation of the GLIC pore, more particularly the opening of said pore.

The application also encompasses a method for the determination of the structure of a complex comprising at least one GLIC monomer chain and at least one compound bound thereto, said method comprising:
- producing a crystal of the invention as above-defined and herein illustrated;
- generating and recording X-ray diffraction data;
- optionally, digitising the data;
- computationally reconstructing the data obtained by X-ray diffraction;
- determining the three-dimensional structure of the crystal components, e.g., by determining the crystal coordinates;
- optionally, storing the crystal coordinates on a data carrier.

### Applications of the complex or crystal of the invention:

The application relates to any use of a complex or crystal of the invention, and to any use of the 3D conformation or atomic coordinates of a complex or crystal of the invention, as well as to any method involving such a use.

A complex or crystal of the invention notably has biotechnological and/or medical uses, notably in screening methods or as screening tools, including computer device systems.

In a complex or crystal of the invention, the at least one bound compound marks the location, where allosteric modulation of GLIC can be obtained by binding to said GLIC at said location. Hence, screening for ligands, which bind similarly to said at least one bound compound, enables to identify compounds, which can have allosteric modulation effect(s) similar to the one(s) shown by said at least one compound.

Therefore, the application notably relates to a method for identifying and/or developing a compound, which is a neurotransmission modulator, and/or which (allosterically) modulates neuronal cell hyperpolarization, and/or which (allosterically) modulates neuronal cell depolarization, wherein said method involves the use of a complex or crystal of the invention, notably to identify and/or develop ligand compound(s), which binds(bind) to the GLIC transmembrane domain or sub-domain at a location similar to the one where said at least one bound compound is bound in a complex or crystal of the invention.

In an embodiment of the invention, said method comprises:
- identifying and/or developing ligand(s) of the transmembrane domain or sub-domain of a GLIC monomer chain, which, when bound to said GLIC transmembrane domain sub-domain:
- locates(locate) in the cavity as defined and/or illustrated in the application, and/or
- locates(locate) at the same location as the at least one bound compound as defined and/or illustrated in the application, and/or
- binds(bind) to said GLIC transmembrane domain at the same binding site(s) as the at least one bound compound as defined and/or illustrated in the application,
wherein said GLIC transmembrane sub-domain is a fragment of said GLIC transmembrane domain, which consists of, or comprises, the sequence extending from the first N-terminal amino acid of the segment M1 to the last C-terminal amino acid of the segment M4 of said GLIC transmembrane domain,
said identification and/or development optionally comprising, for each one of said ligands, complexing or co-crystallizing said ligand and at least one GLIC monomer chain (or a fragment thereof, which has at least retained said GLIC transmembrane domain or sub-domain), and determining the location of said ligand in said at least one GLIC monomer chain (or fragment thereof), and comparing the ligand location thus determined with the location of the at least one bound compound in a complex or crystal of the invention,
- optionally selecting those ligands, which allosterically modulates the ion conductance of a vertebrate anionic or cationic pentameric Ligand-Gated Ion Channel (pLGIC), more particularly of a gamma-aminobutyric acid receptor (GABA_{A}R) and/or a glycine receptor (GlyR) and/or a nicotinic acetylcholine receptor (AChR) and/or a serotonine receptor (5HT₃-R), and/or
- optionally selecting those ligands, which compete with propofol and/or desflurane for binding to said GLIC transmembrane domain or sub-domain, said optional selection optionally comprising, for each one of said ligands, complexing or co-crystallizing said ligand and at least one GLIC monomer chain (or a fragment thereof, which has retained said GLIC transmembrane domain or sub-domain), determining the atomic coordinates of said ligand in the complex or crystal thus formed, and comparing it with the atomic coordinates that are given in the HETATM field of Table 1 for the residue named DES and/or for the residue named PPL.

The term "ligand" means a compound, which binds to the transmembrane domain of a GLIC monomer chain; it is used to avoid confusion with at least one compound, which is bound at a specific location of a GLIC monomer chain in the crystal of the invention (*i.e*., in the cavity identified by the inventors).

In other words, the method of the invention comprises identifying or developing ligand(s) of said GLIC transmembrane domain or sub-domain, which has(have) a binding site on the part(s) of said GLIC transmembrane domain or sub-domain, which is(are) the wall(s) of the GLIC transmembrane cavity identified by the inventors.

Hence, when it is bound to said GLIC transmembrane domain, said ligand locates as the at least one bound compound in the crystal of the invention, *i.e*., in the cavity identified by the inventors.

Within said cavity, said ligand may bind at the same particular binding site(s) of the GLIC transmembrane cavity wall(s) as the at least one bound compound (*e.g*., desflurane or propofol), or may bind at different binding site(s) but still in said cavity.

Said ligand(s) can notably have the same compound identity or chemical nature as the one(s) given above for said at least one bound compound contained in a complex or crystal of the invention.

Said ligand(s) can alternatively or complementarily have the same modulation effect(s) as the one(s) given above for said at least one bound compound contained in a complex or crystal of the invention.

For example, according to a particular embodiment of the invention, said ligand compound is a compound, which (allosterically) modulates the ion conductance of a vertebrate anionic or cationic pentameric Ligand-Gated Ion Channel (pLGIC), more particularly of a gamma-aminobutyric acid receptor (GABA_{A}R) and/or a glycine receptor (GlyR) and/or a nicotinic acetylcholine receptor (AChR) and/or a serotonine receptor (5HT₃-R).

For example, said ligand compound(s) can be or comprise:
- a steroid (*e.g*., a hormone), or a benzodiazepine, or an azapirone, or a piperazine and/or diphenylmethane (such as hydroxyzine), or an halogenated ether (*e.g*., a tetrafluoroethane, such as desflurane), or a propylphenol (such as propofol), or a carboxylated imidazole derivative (such as etomidate), or 3-hydroxybutyric acid, or
- donepezil ((*RS*)-2-[(1-benzyl-4-piperidyl)methyl]-5,6-dimethoxy-2,3-dihydroinden-1-one), or galantamine ((4a*S*,6*R*,8a*S*)-5,6,9,10,11,12-hexahydro-3-methoxy-11-methyl-4a*H-*[1]benzofuro[3a,3,2-*ef*] [2] benzazepin-6-ol), or rivastigmine ((*S*)-*N*-Ethyl-*N*-methyl-3-[1-(dimethylamino)ethyl]-phenylcarbamate), or memantine (3,5-dimethyladamantan-1-amine).

The application also relates to the ligand compounds thereby identified and/or developed.

The application also relates to a computer system device for identifying and/or developing a compound, which is a neurotransmission modulator, and/or a compound, which (allosterically) modulates neuronal cell hyperpolarization, and/or a compound, which (allosterically) modulates neuronal cell depolarization.

Said computer system device comprises:
- storage means storing:
   - the atomic coordinates, which said at least one GLIC monomer chain or a fragment thereof has in the complex or crystal of the invention, and storing
   - the location of the GLIC transmembrane cavity as defined and/or illustrated in the invention and/or the location of the at least one bound compound as defined and/or illustrated in the invention, more particularly the atomic coordinates thereof, and
   - software for identifying or developing, based on said coordinates and location(s), ligand(s) of a GLIC transmembrane domain or to a sub-domain thereof, which, when bound to said GLIC transmembrane domain or sub-domain, locates(locate) in said cavity and/or locates at the same location as said at least one bound compound,
   wherein said GLIC transmembrane sub-domain is a fragment of said GLIC transmembrane domain, which consists of, or comprises, the sequence extending from the first N-terminal amino acid of the segment M1 to the last C-terminal amino acid of the segment M4 of said GLIC transmembrane domain.

Said storage means may for example store the atomic coordinates that are given in the HETATM field of Table 1 for the residue named DES and/or for the residue named PPL. A computer system device of the invention may further comprise:
- means displaying representations of the candidate ligand(s) or a portion thereof, and displaying representations of said GLIC monomer chain or fragment thereof, or a portion thereof; and/or
- processing means for calculating binding affinities between the candidate ligand(s), and those portion(s) of said GLIC transmembrane domain or sub-domain thereof, where said ligand(s) is(are) located in said crystal.

The application also relates to a method for producing a pharmaceutical composition or medicament or drug, more particularly:
- a pharmaceutical composition or medicament for use in the treatment and/or prevention and/or palliation of a disease or disorder of the nervous system, more particularly the CNS and/or the PNS, still more particularly the CNS, and/or
- a pharmaceutical composition or drug for use in inducing a medically-desired temporary disorder of the nervous system.

Said method comprises the use of a complex or crystal of the invention, or of the 3D conformation or atomic coordinates thereof.

In an embodiment of the invention, said method comprises:
- identifying or developing at least one compound by the above-mentioned method of the invention for identifying or developing a compound, which is a neurotransmission modulator, and/or which (allosterically) modulates neuronal cell hyperpolarization and/or which (allosterically) modulates neuronal cell depolarization);
- optionally, modifying said at least one identified and/or developed compound to increase its specificity with respect to neuronal cells and/or its lipophilicity (preferably, provided said modified compound has retained the property of being a neurotransmission modulator, and/or of modulating neuronal cell hyperpolarization, and/or of modulating neuronal cell depolarization, and/or of allosterically modulating neuronal cell hyperpolarization and/or of allosterically modulating neuronal cell depolarization);

- optionally, testing the neurotransmission modulation effect(s) induced by said at least one identified and/or developed compound in one or several *in vitro* and/or *in vivo* assays in the presence of neuronal cells;
- formulating said at least one identified and/or developed, optionally modified, compound with a pharmaceutically acceptable carrier or diluent.

In an embodiment of the invention, said pharmaceutical composition, medicament or drug is:
- an anesthetic, and/or
- a muscle relaxant, and/or
- a sedative, and/or
- a hypnotic, and/or
- an anxiolytic, and/or
- an antipsychotic, and/or
- an antidepressant, and/or
- an anticonvulsant, and/or
- an anti-epileptic, and/or
- an anti-emetic (*e.g*., an anti-emetic intended for the treatment and/or prevention and/or palliation of motion sickness and/or of the side-effects of medications such as opioids and chemotherapy), and/or
- an amnesic, and/or
- a medicament intended for the treatment and/or prevention and/or palliation of a chemical addiction (a chemical addiction that alters neurotransmission, *e.g*., nicotine addiction and/or cocaine addiction).

In an embodiment of the invention, said pharmaceutical composition, medicament or drug is:
- a nootropic drug, and/or
- an euphorisant, and/or
- a medicament intended for the treatment and/or prevention and/or palliation of toxic and/or metabolic encephalopathy(ies) (*e.g*., mercury or lead intoxication), and/or
- a medicament intended for the treatment and/or prevention and/or palliation of neurodegenerative disease(s) (*e.g*., Alzheimer's disease and/or Parkinson's disease), and/or
- a medicament intended for the treatment and/or prevention and/or palliation of diseases involving a cognitive deficit and/or the inability to focus attention (*e.g*., schizophrenia, more particularly the first stages of schizophrenia, and/or ADHD).

The application also relates to the pharmaceutical compositions, medicaments or drugs thereby produced.

The term "comprising", which is synonymous with "including" or "containing", is openended, and does not exclude additional, unrecited element(s), ingredient(s) or method step(s), whereas the term "consisting of" is a closed term, which excludes any additional element, step, or ingredient which is not explicitly recited.

The term "essentially consisting of" is a partially open term, which does not exclude additional, unrecited element(s), step(s), or ingredient(s), as long as these additional element(s), step(s) or ingredient(s) do not materially affect the basic and novel properties of the invention.

The term "comprising" (or "comprise(s)") hence includes the term "consisting of" ("consist(s) of"), as well as the term "essentially consisting of" ("essentially consist(s) of"). Accordingly, the term "comprising" (or "comprise(s)") is, in the present application, meant as more particularly encompassing the term "consisting of" ("consist(s) of"), and the term "essentially consisting of" ("essentially consist(s) of").

In an attempt to help the reader of the present application, the description has been separated in various paragraphs or sections. These separations should not be considered as disconnecting the substance of a paragraph or section from the substance of another paragraph or section. To the contrary, the present description encompasses all the combinations of the various sections, paragraphs and sentences that can be contemplated. Each of the relevant disclosures of all references cited herein is specifically incorporated by reference. The following examples are offered by way of illustration, and not by way of limitation.

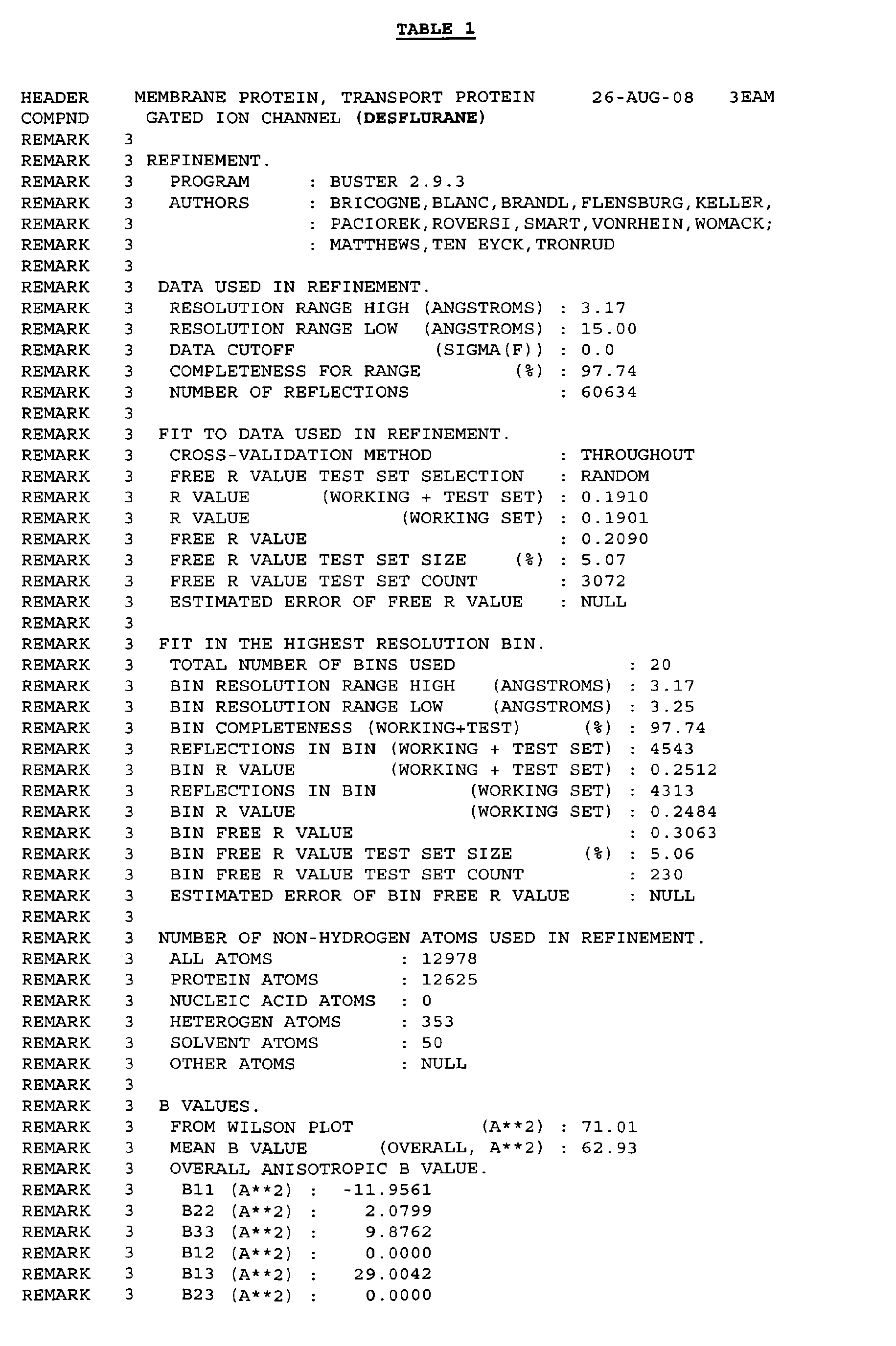

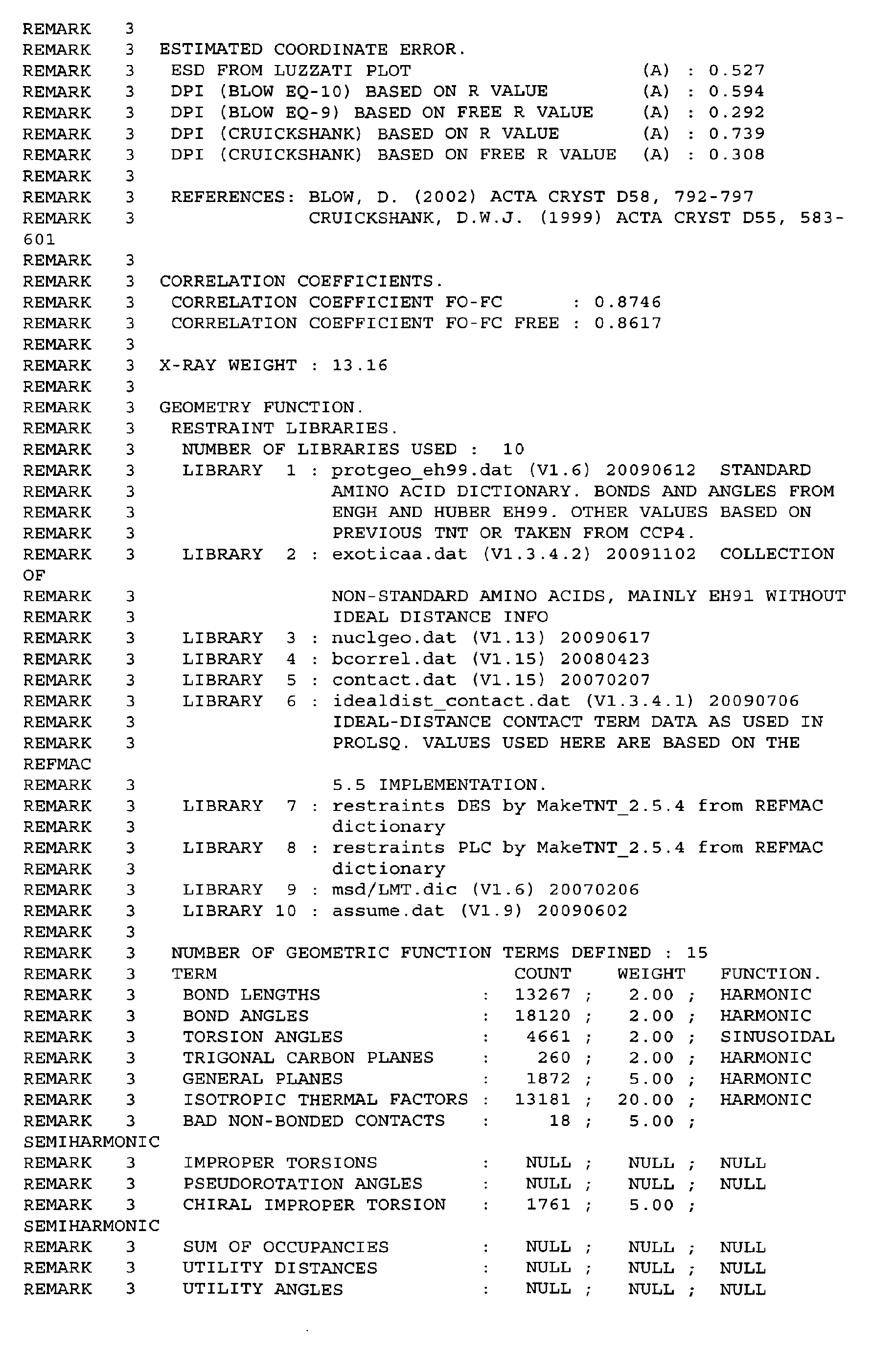

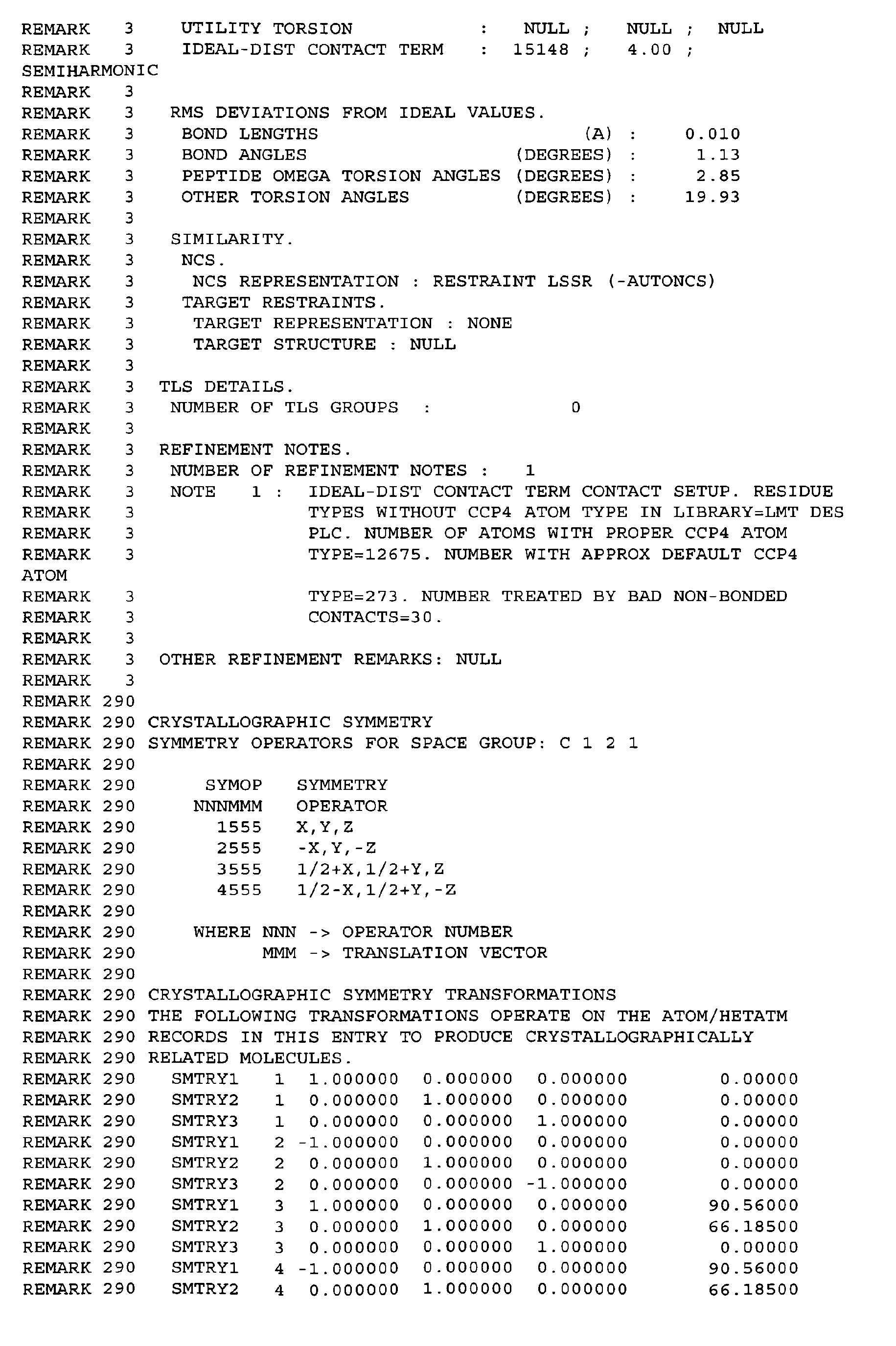

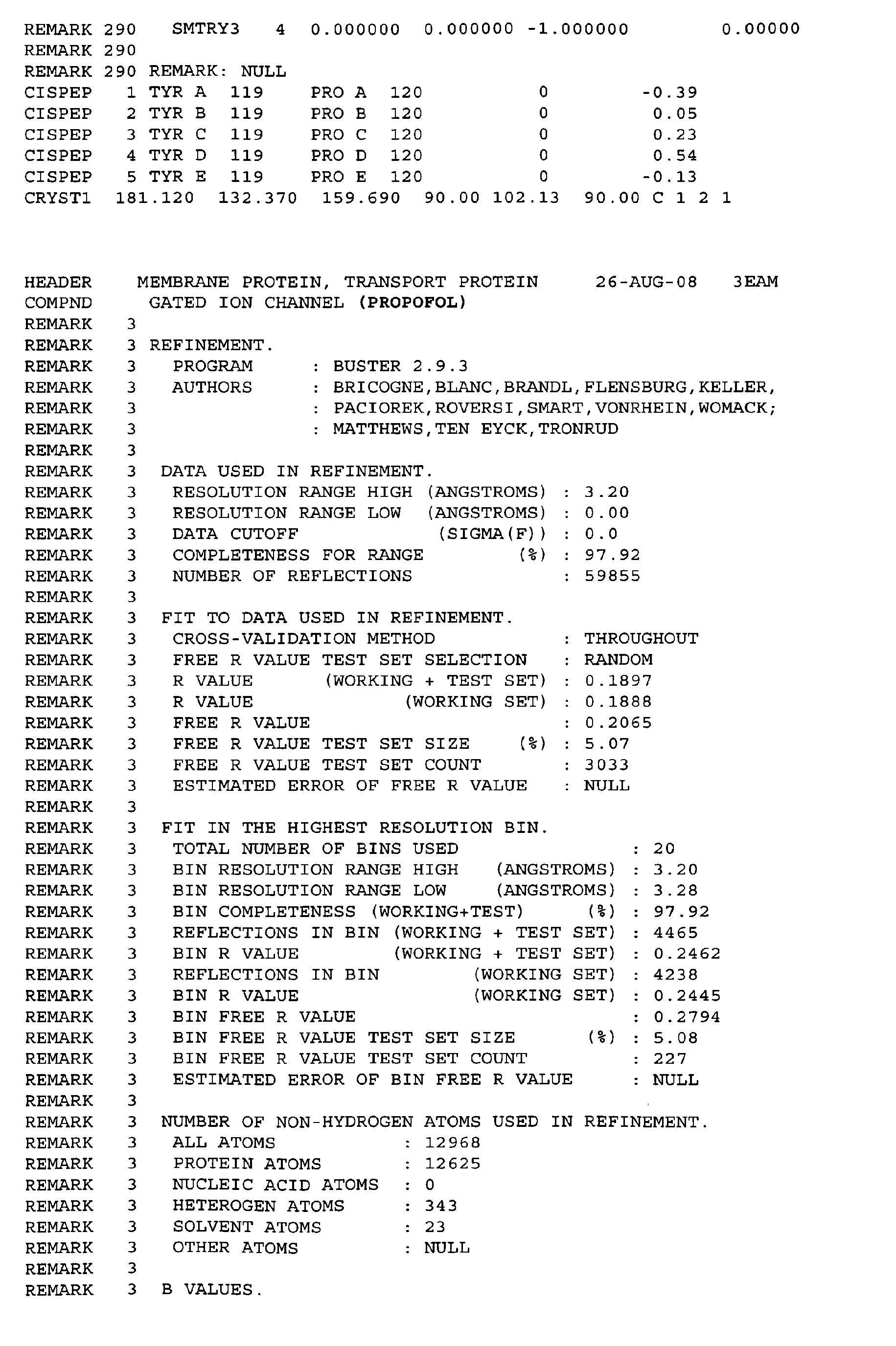

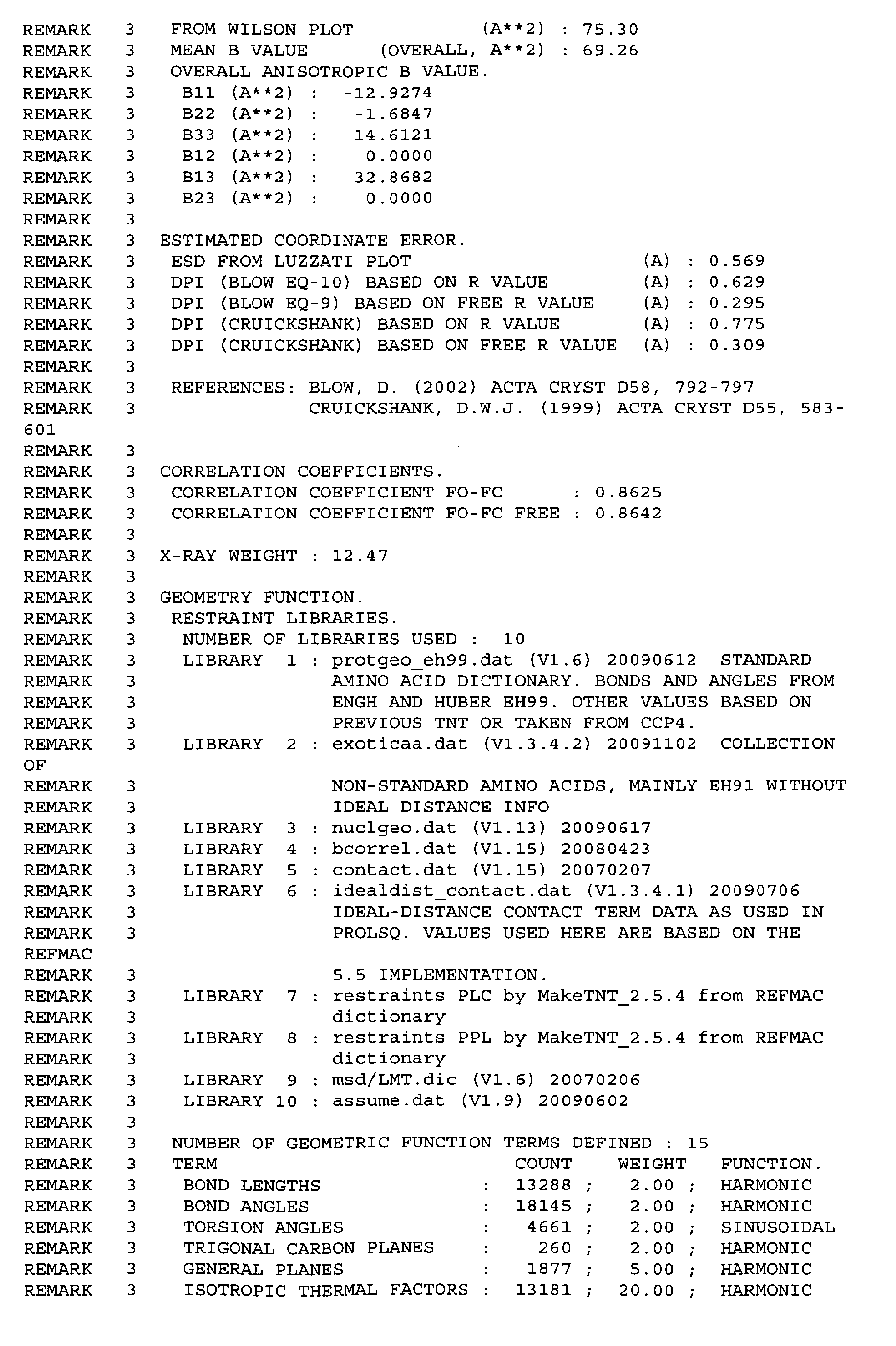

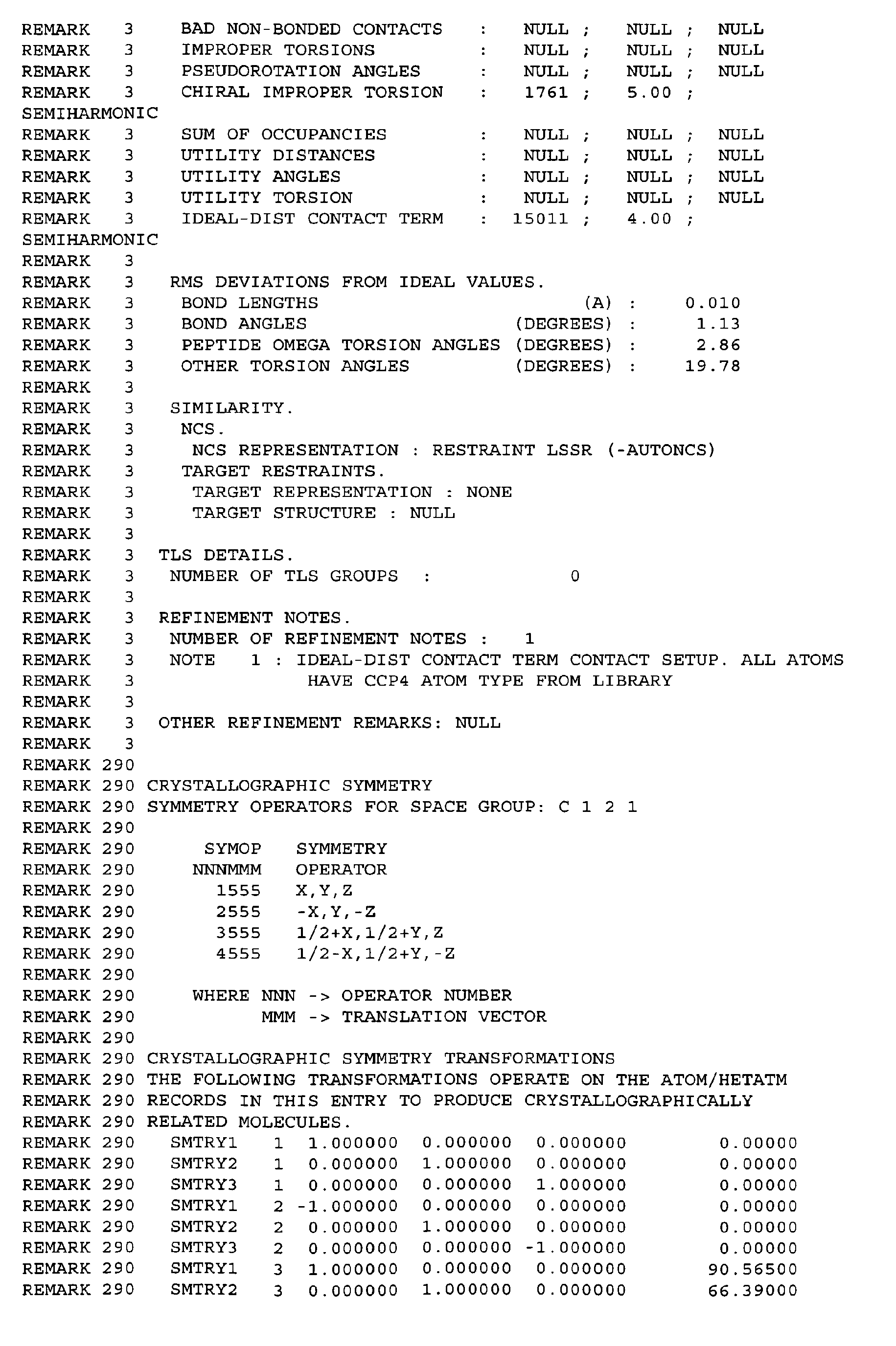

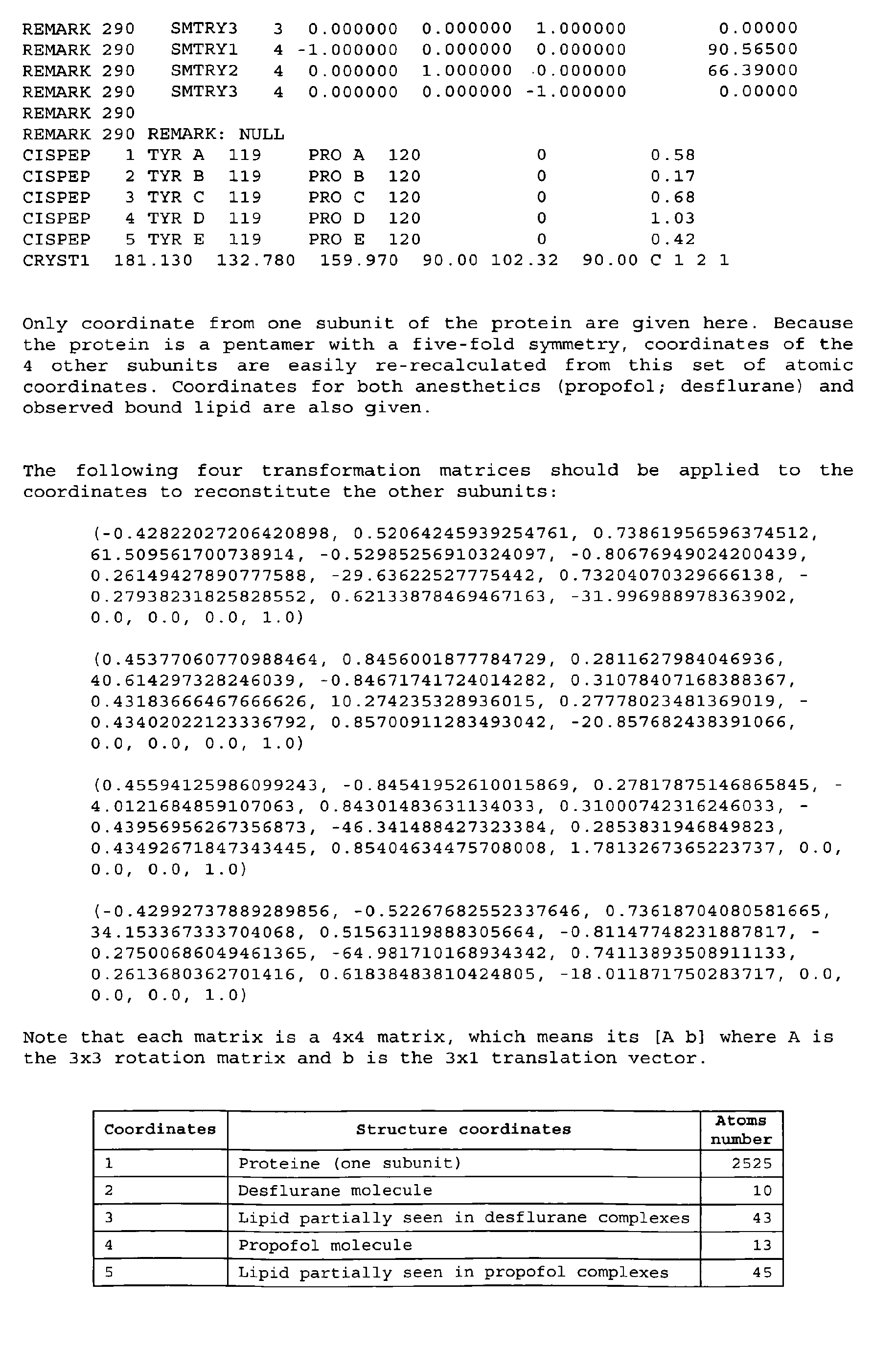

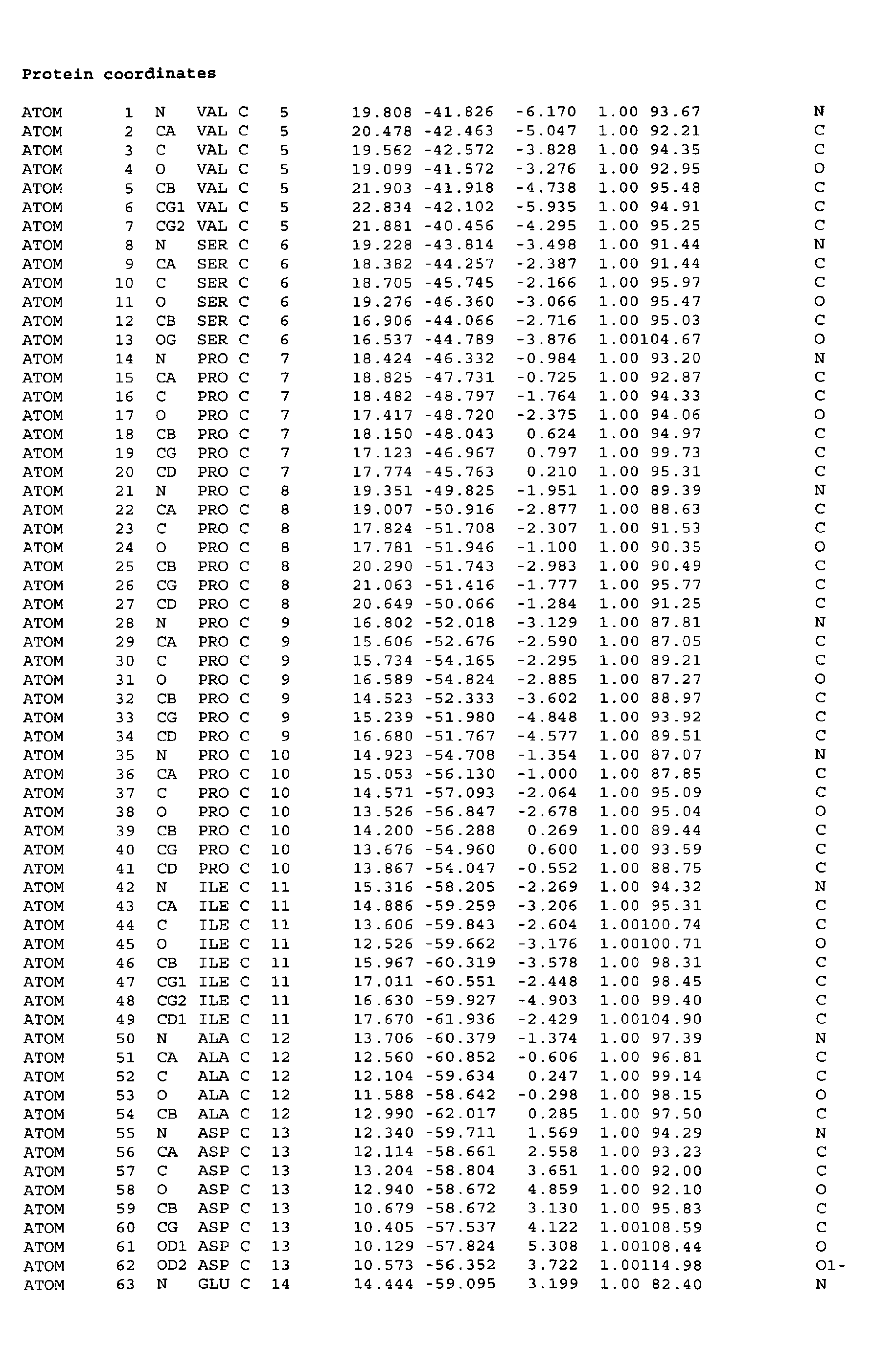

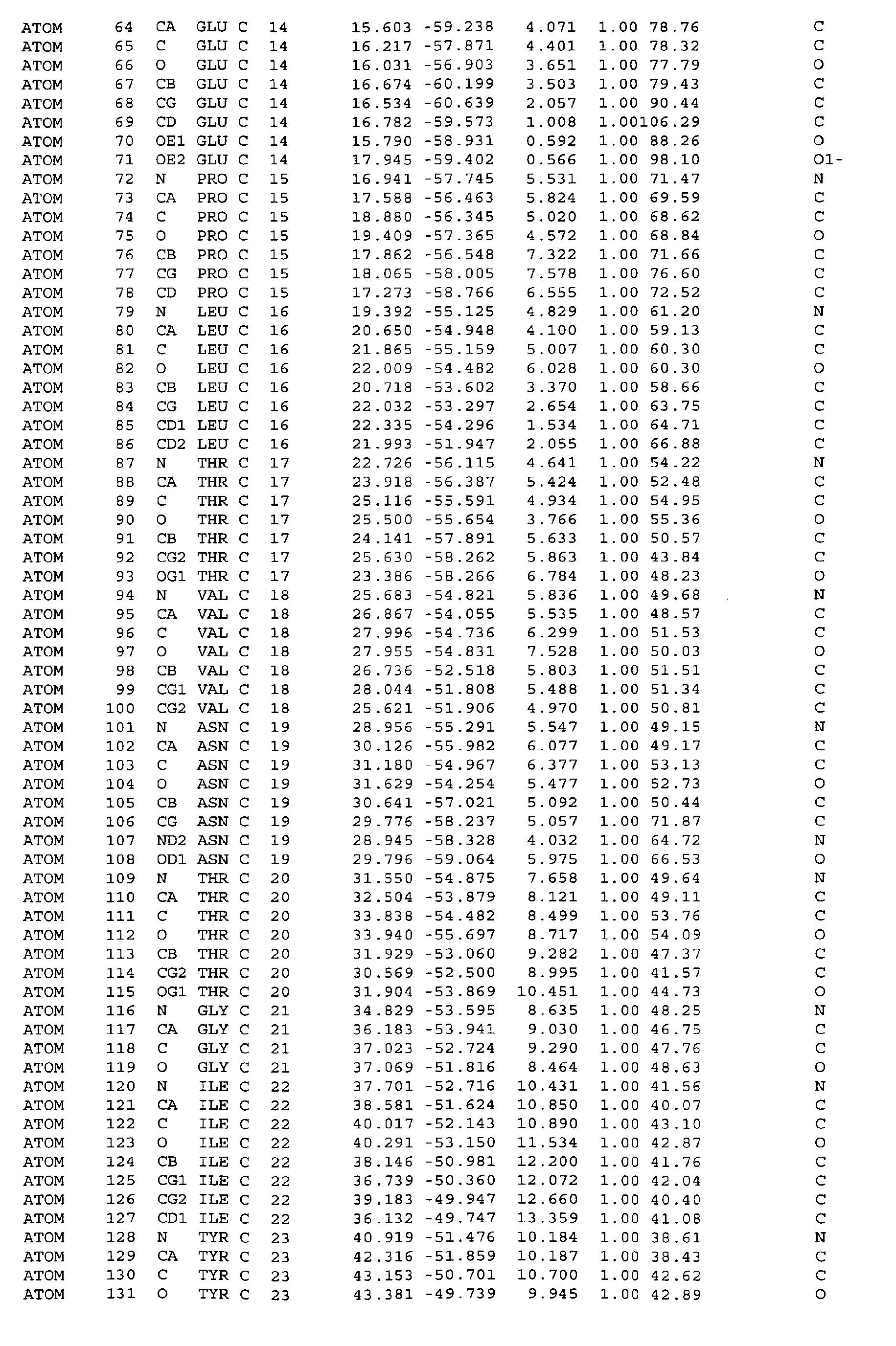

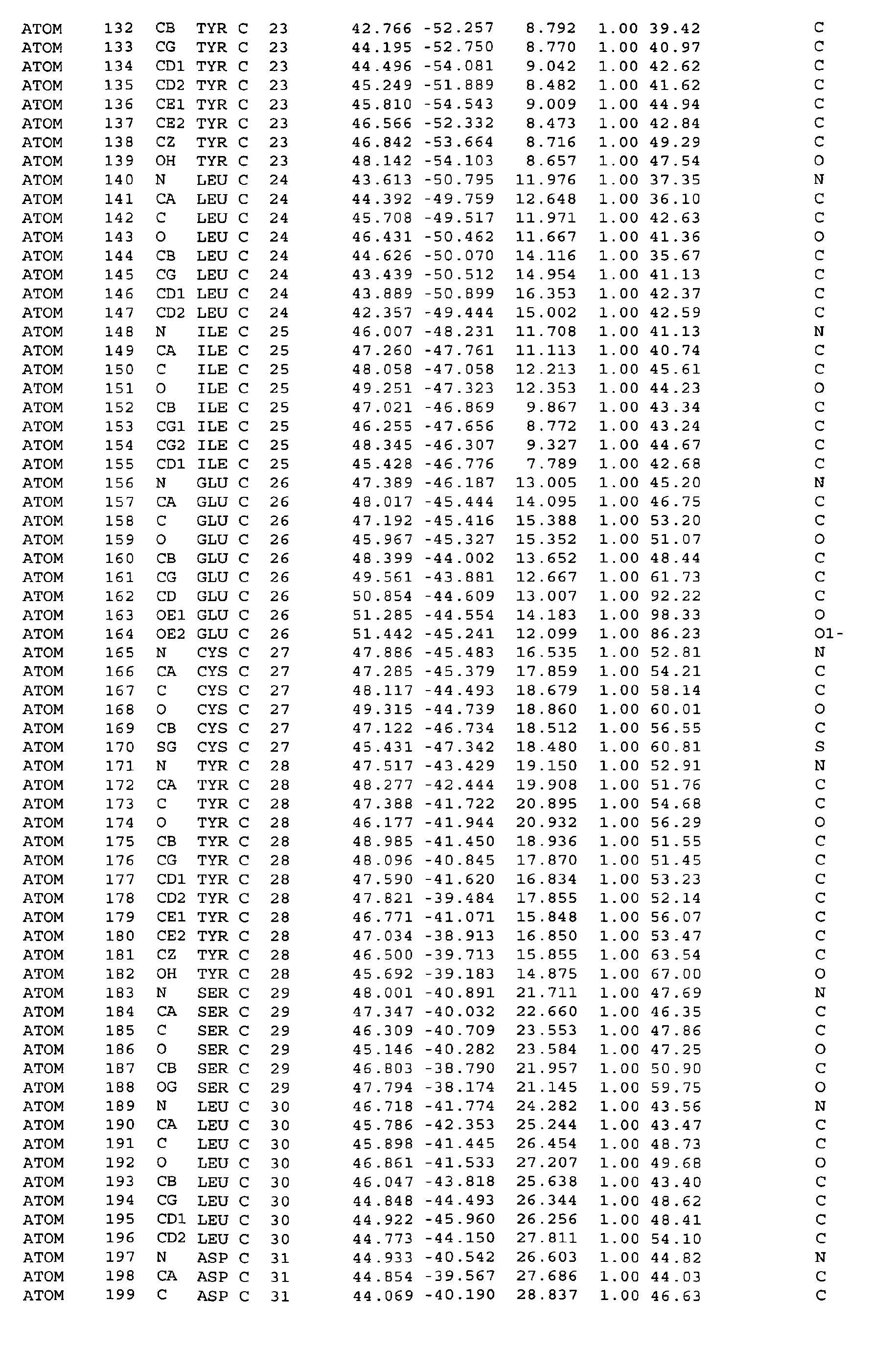

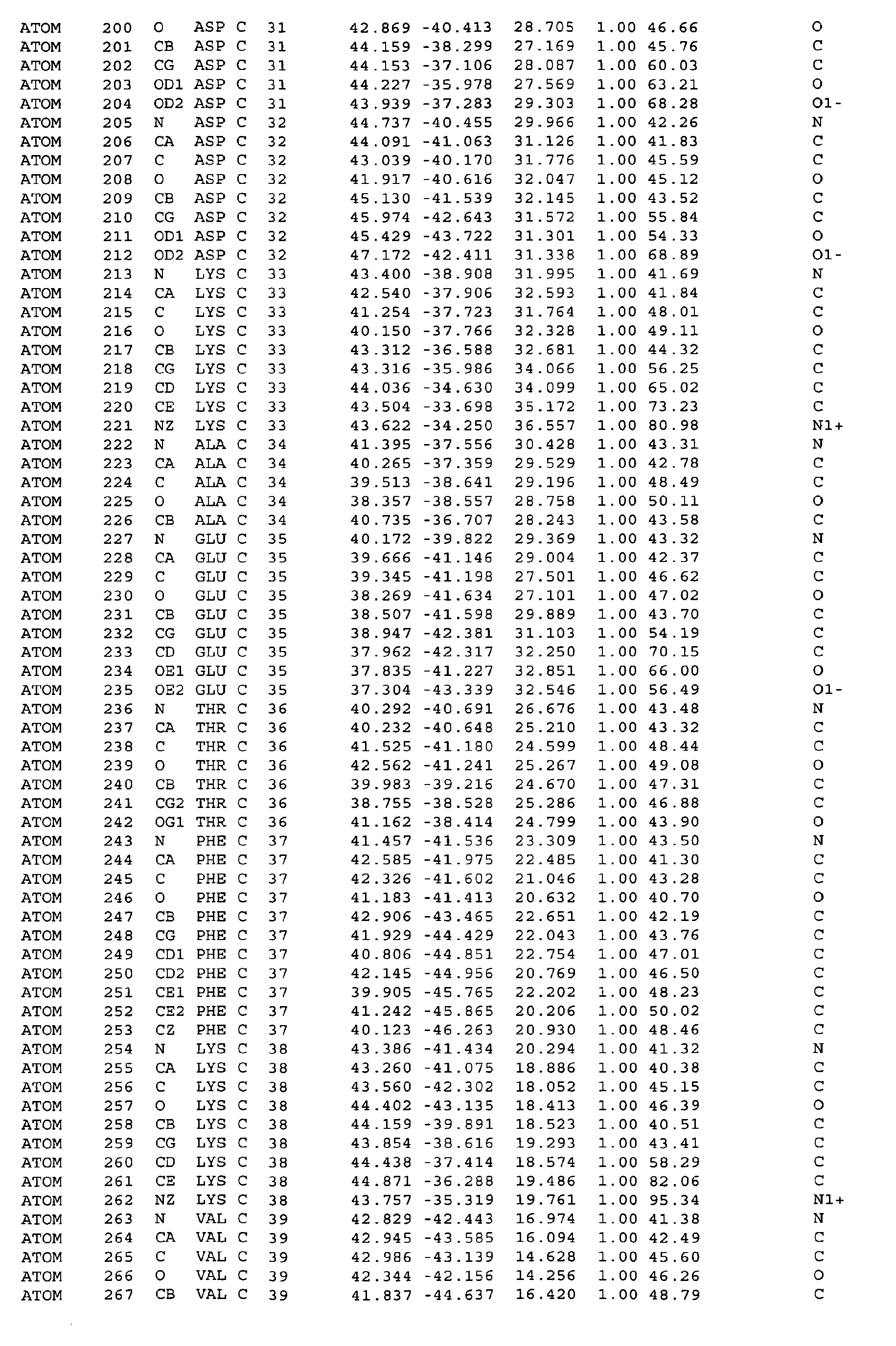

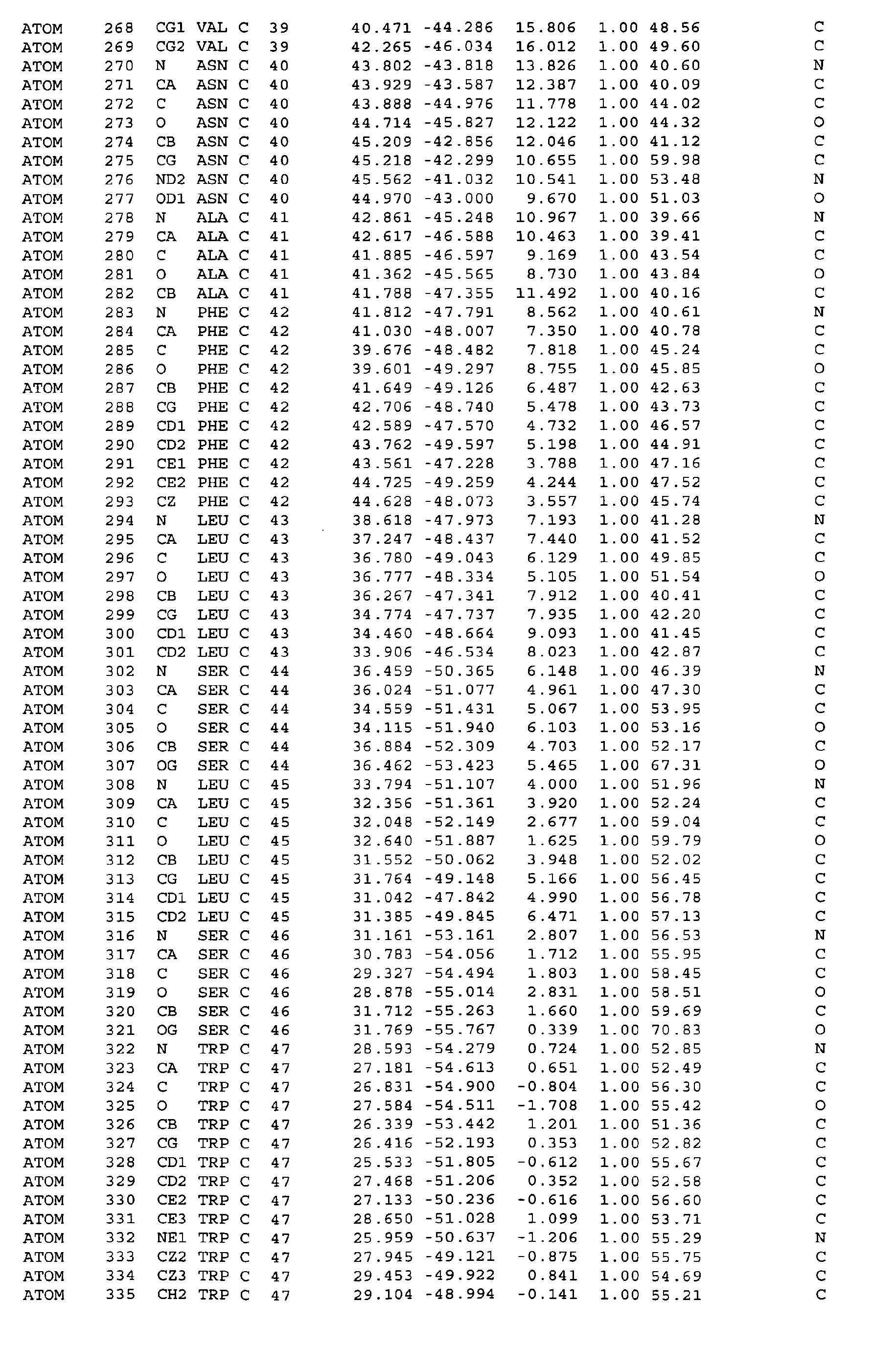

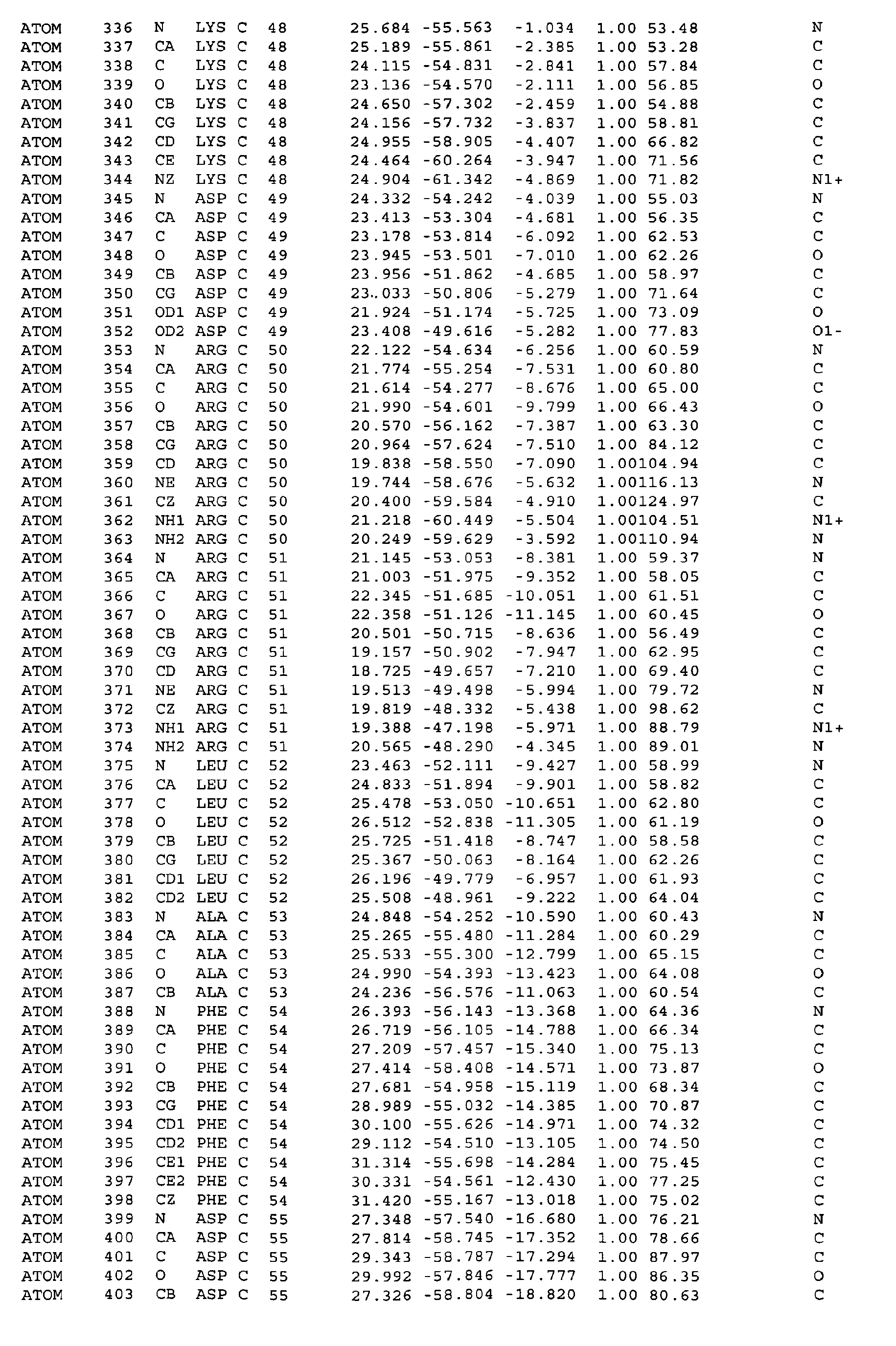

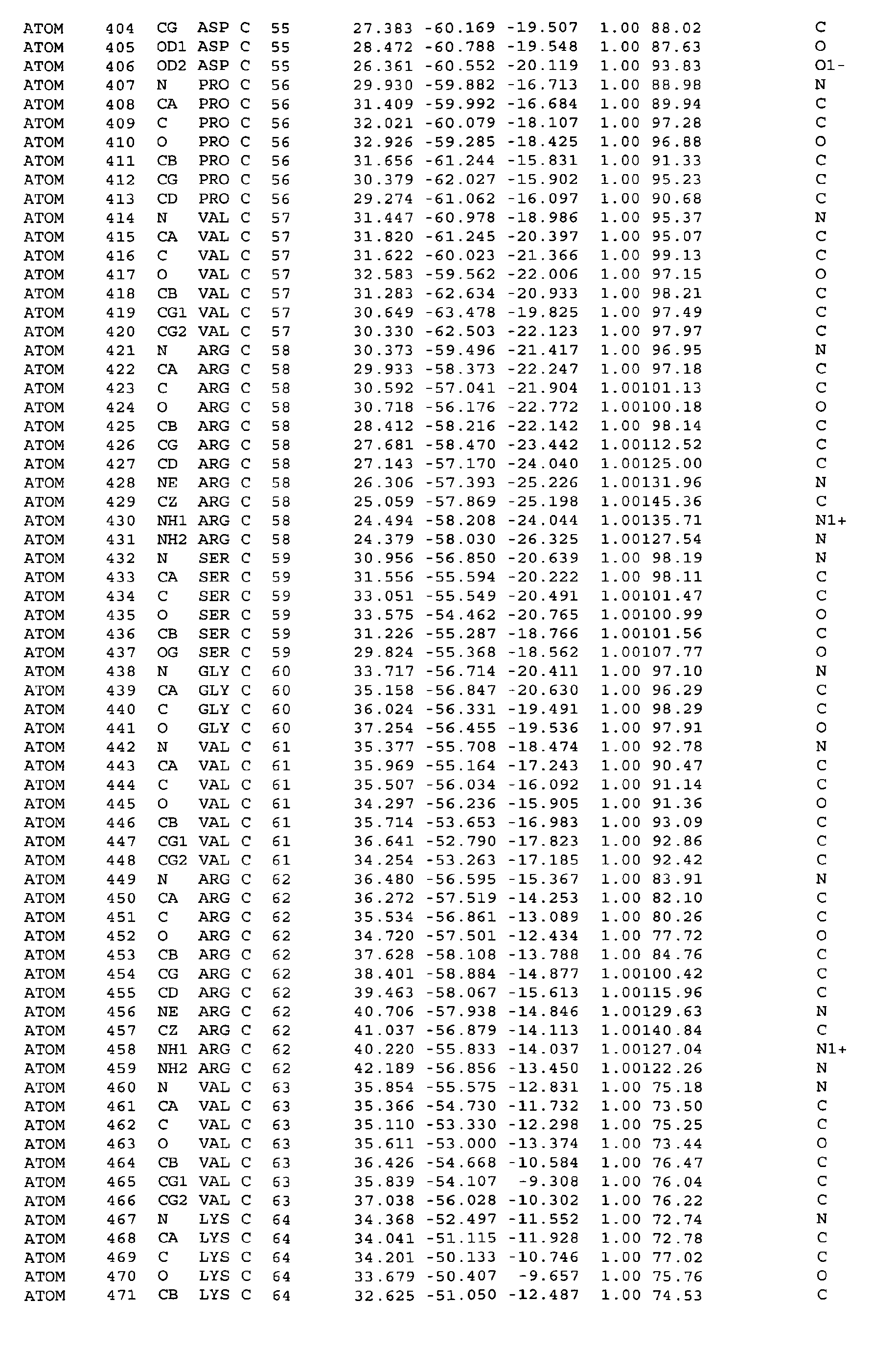

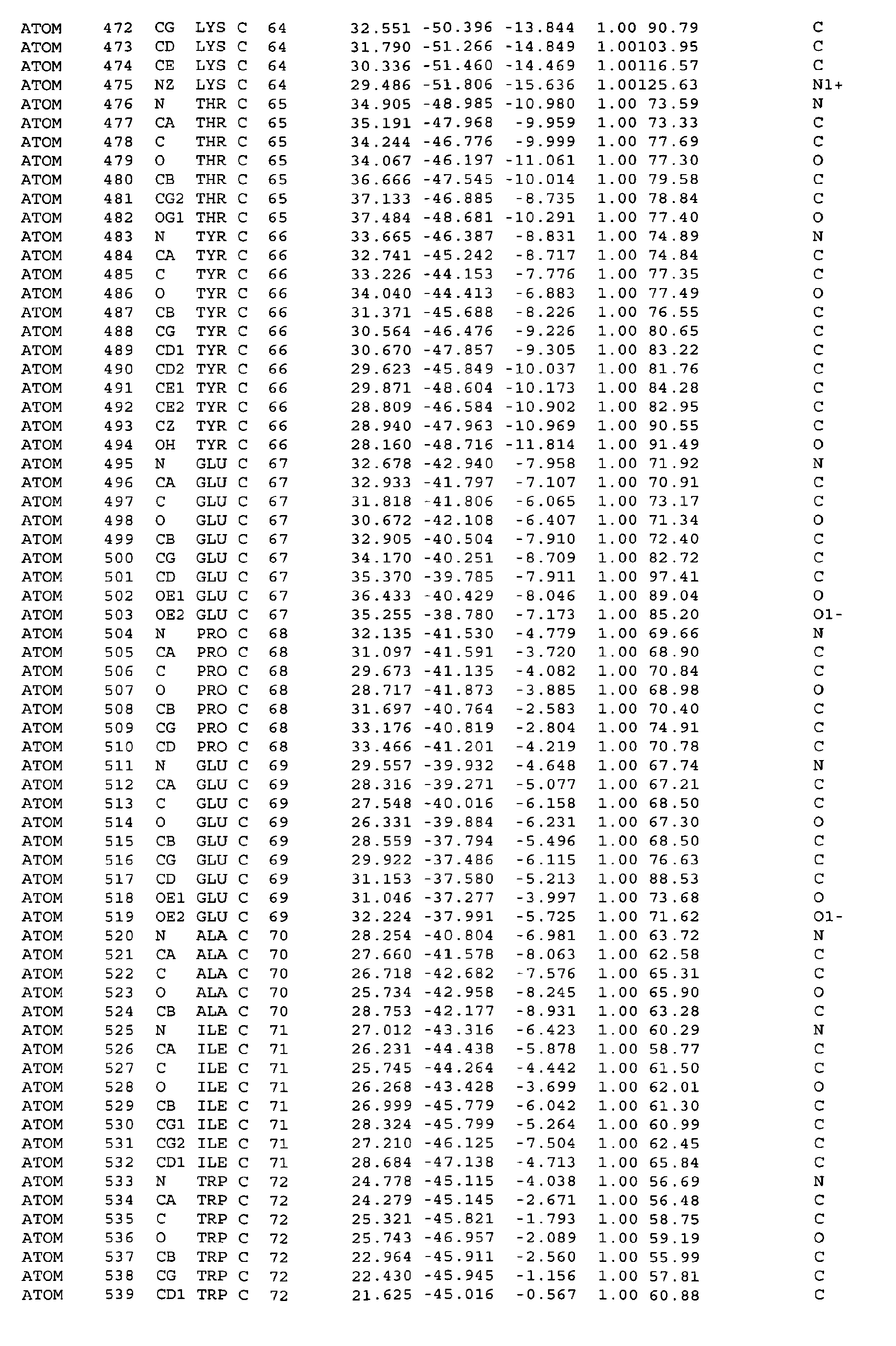

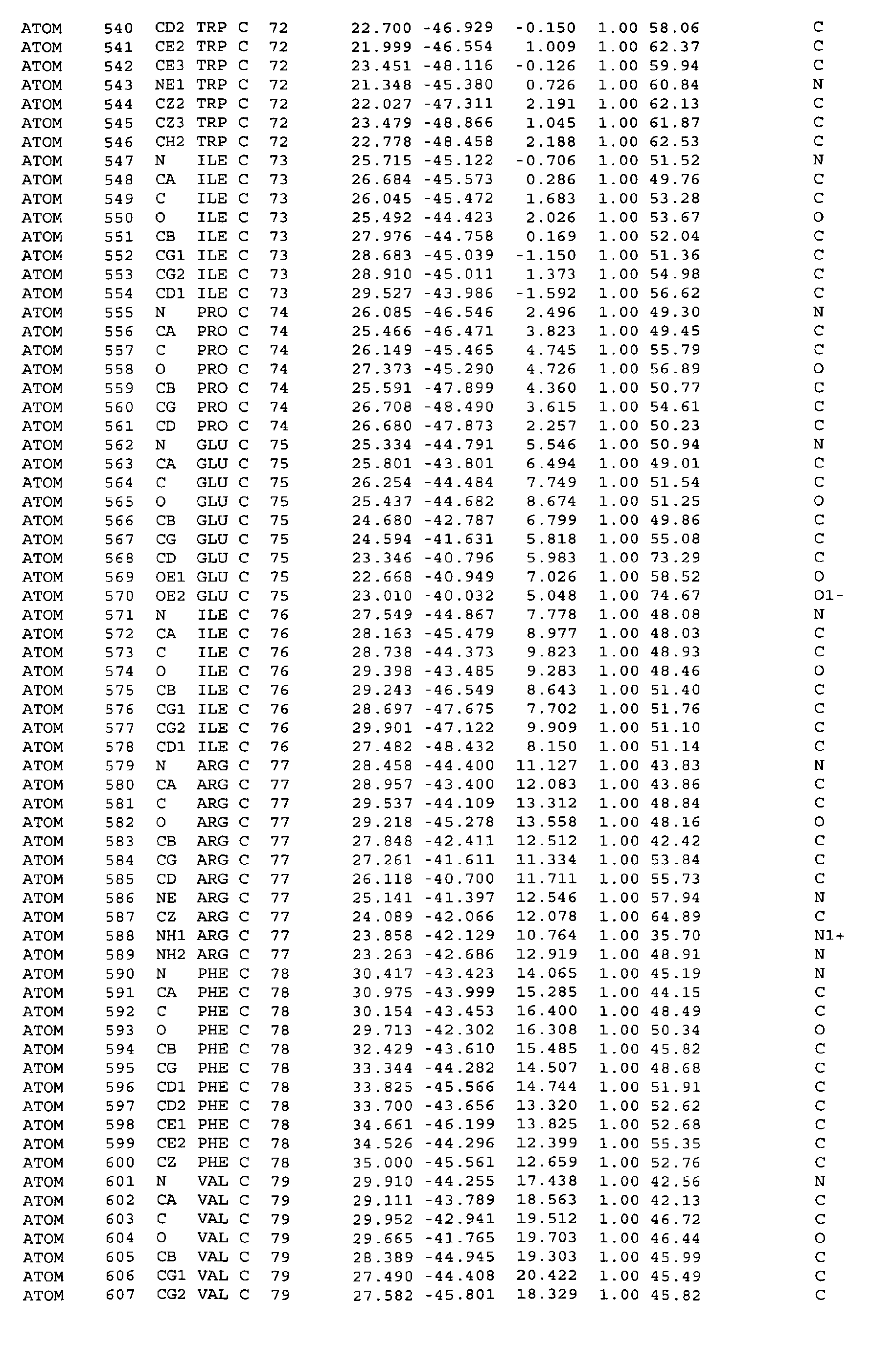

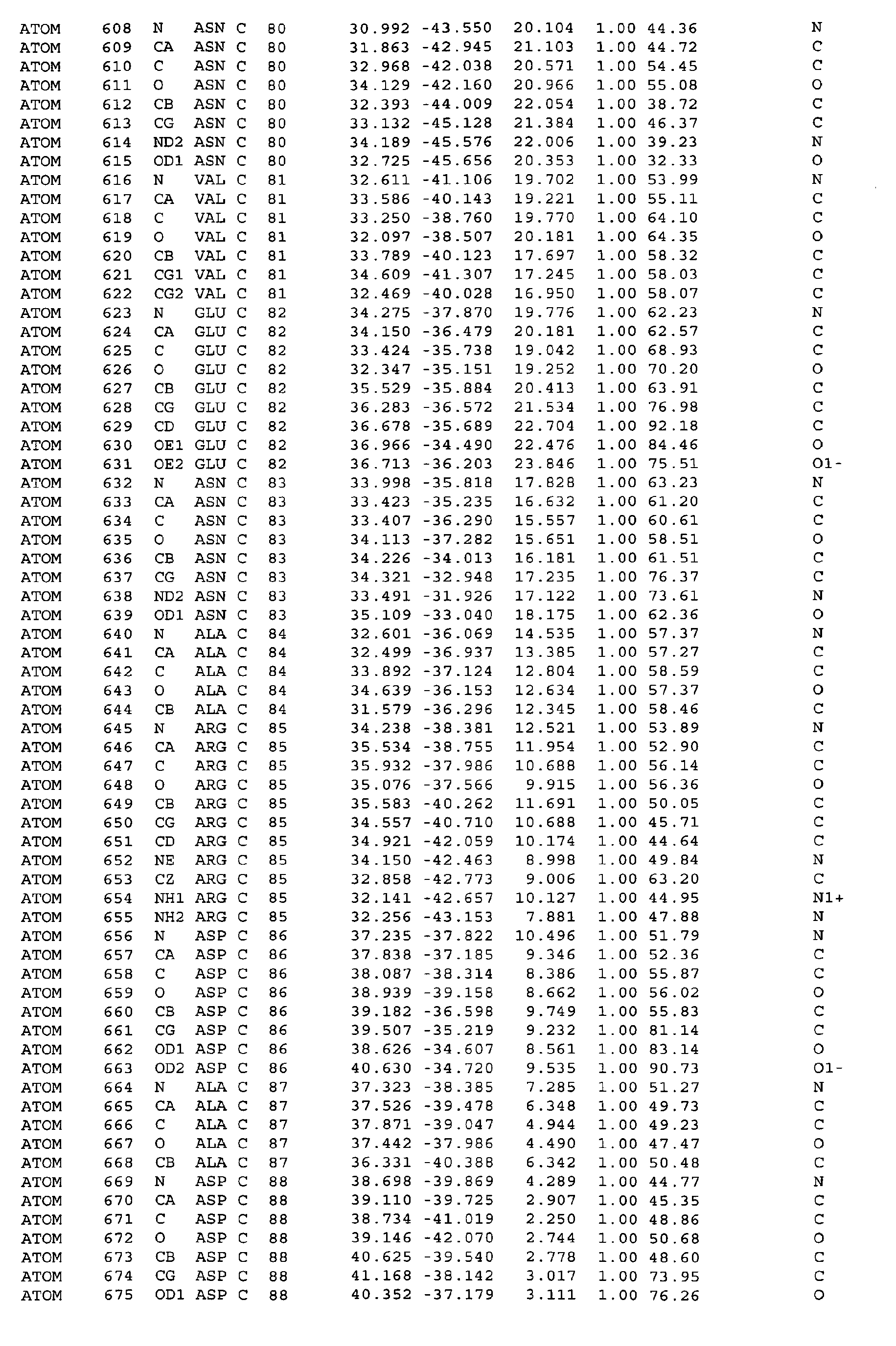

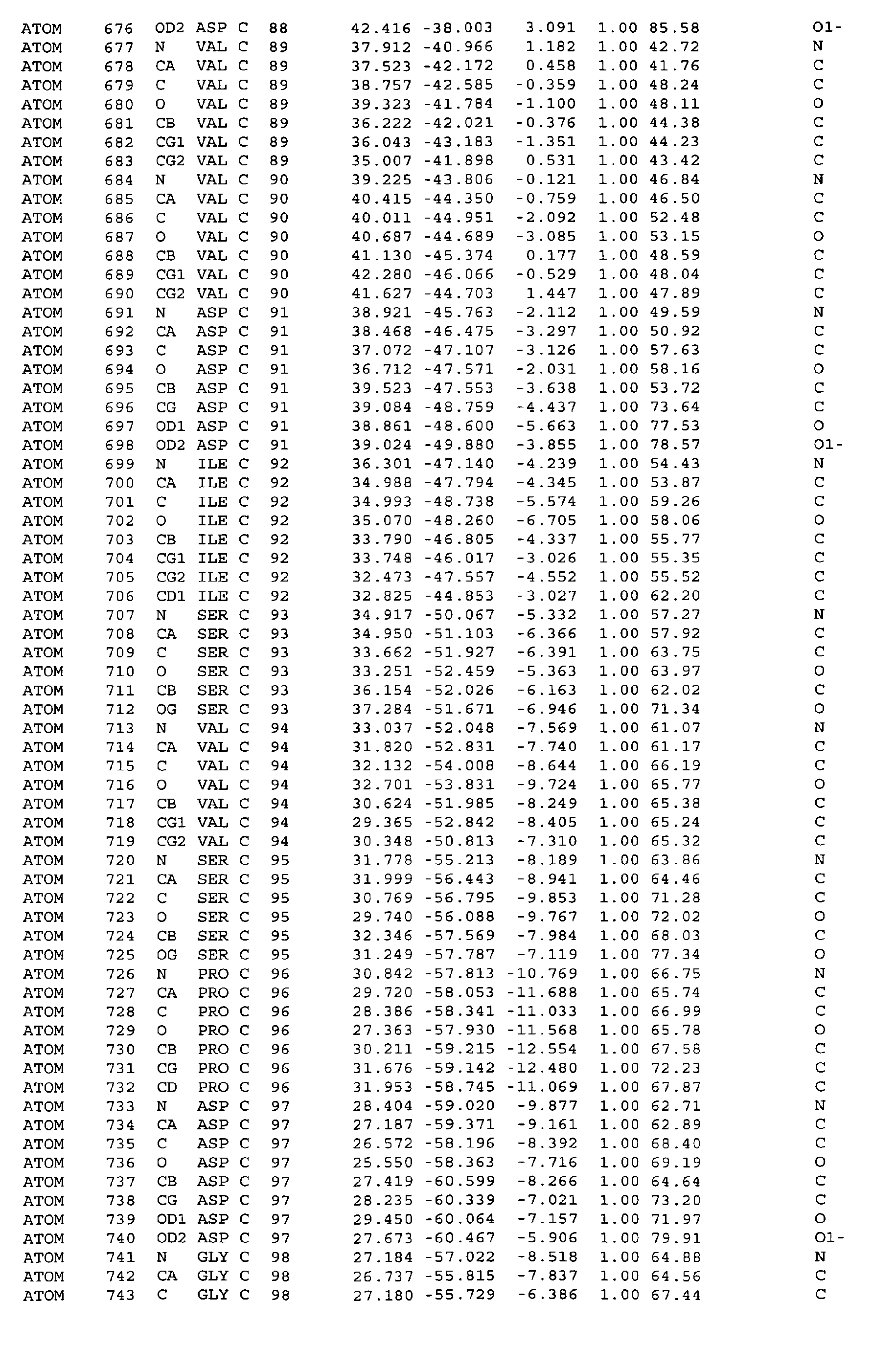

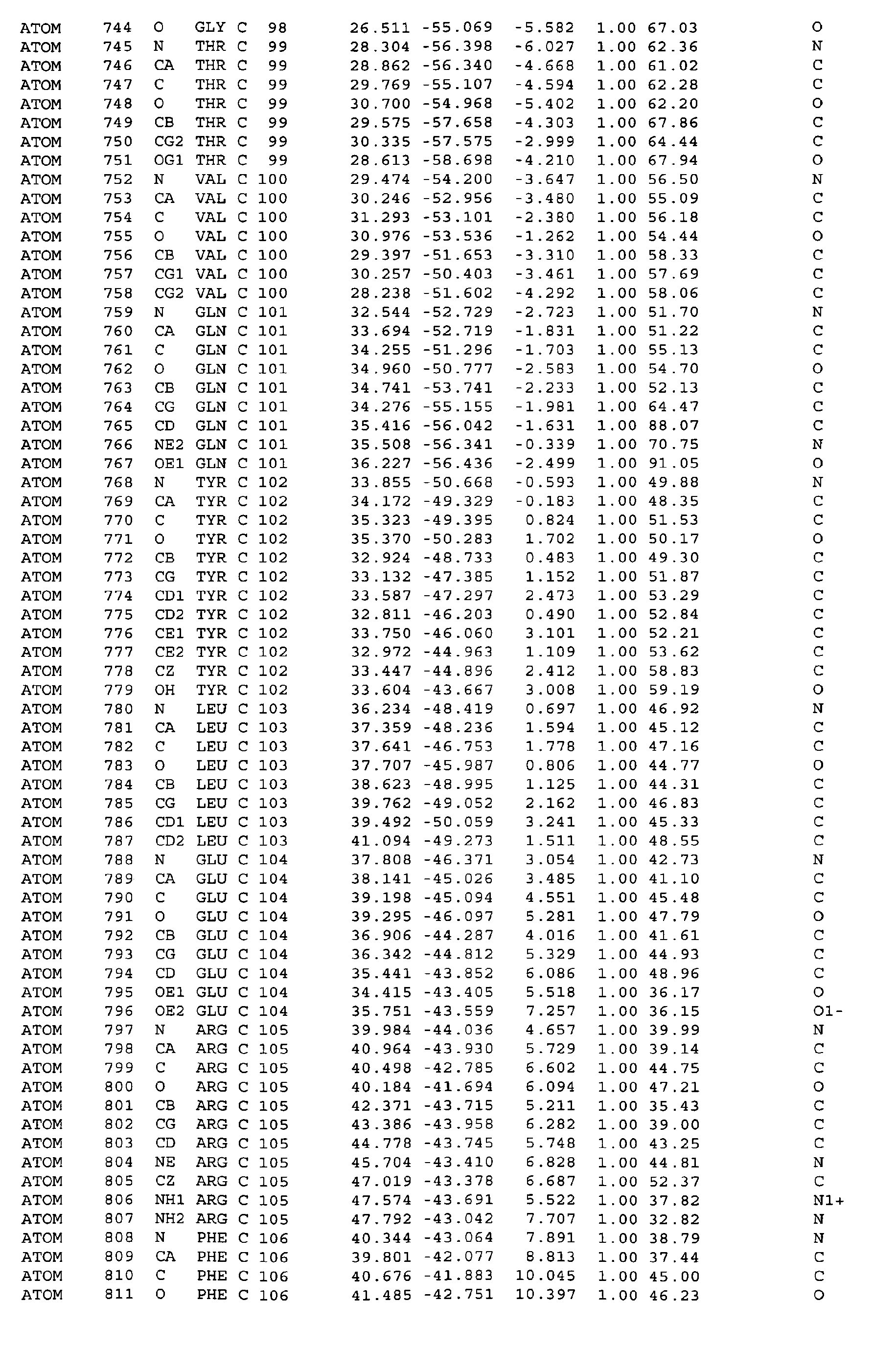

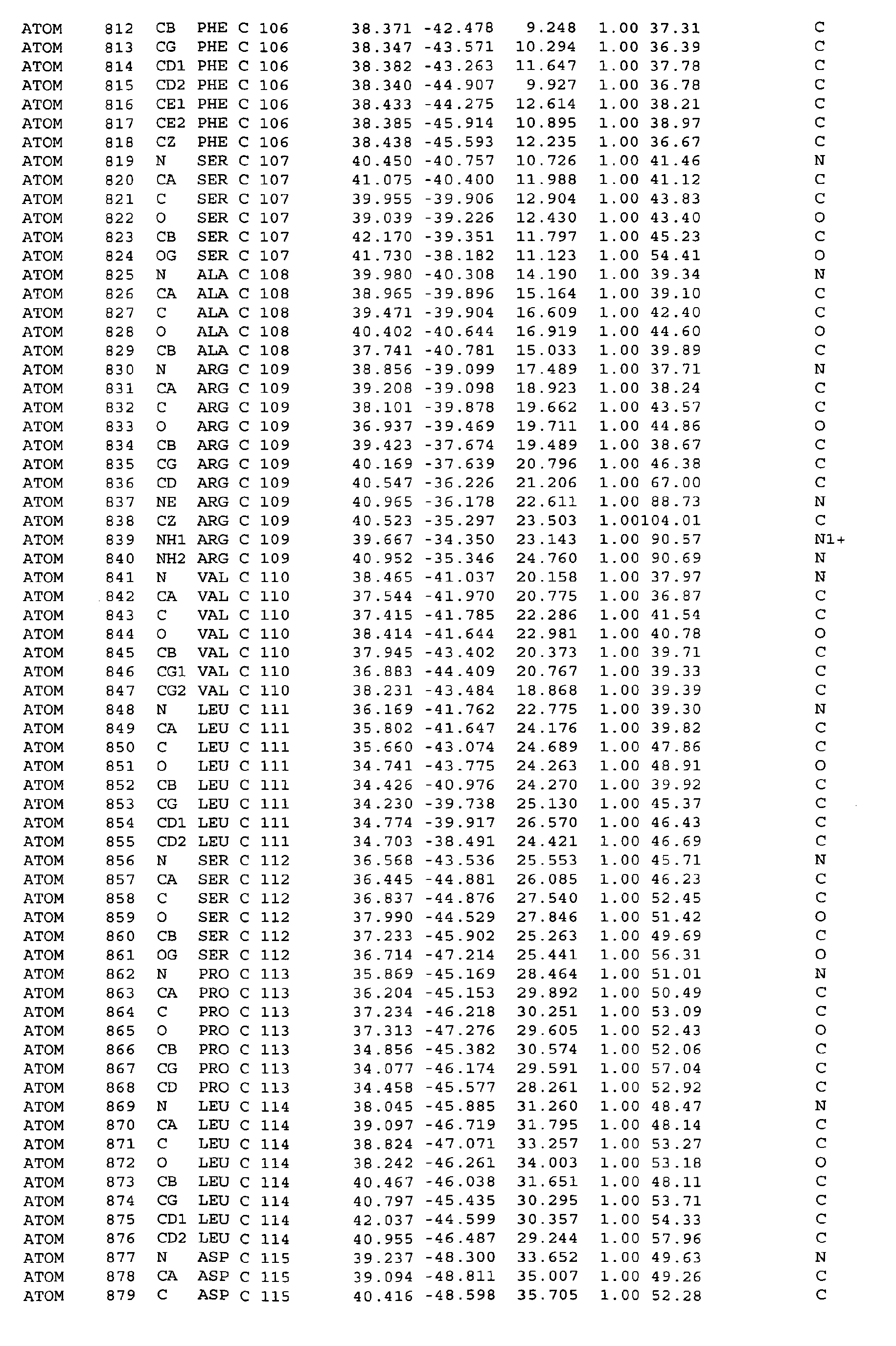

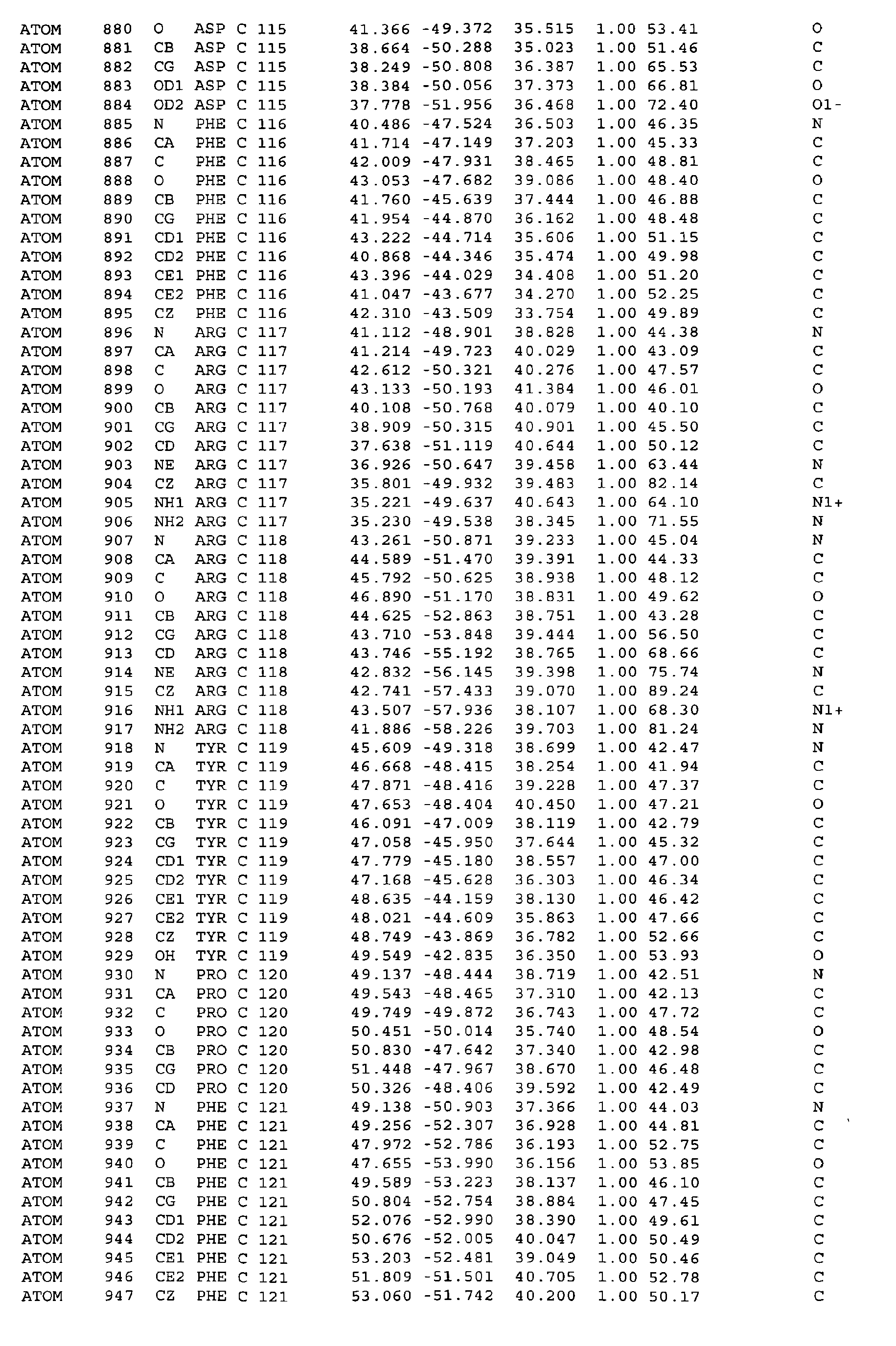

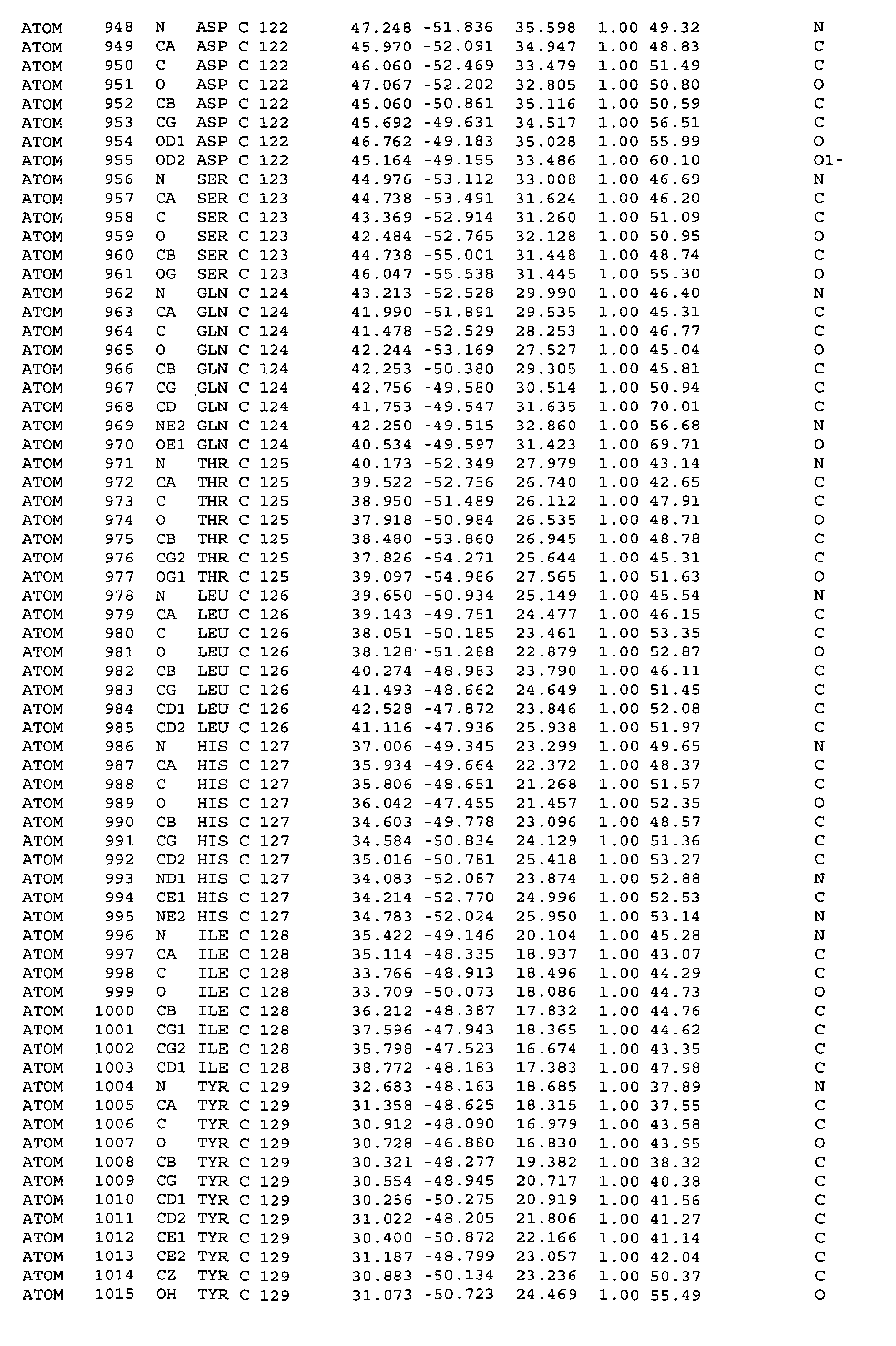

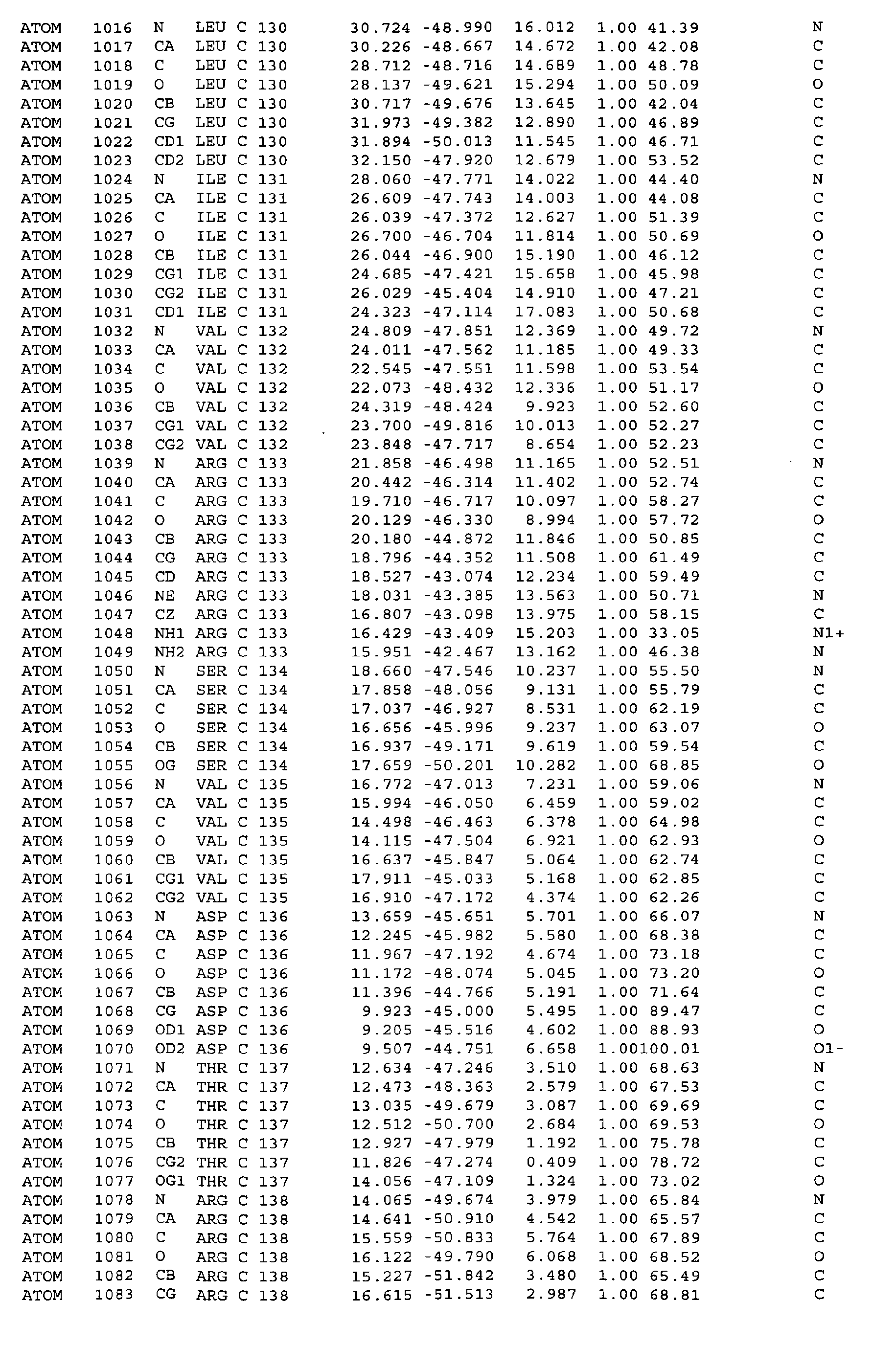

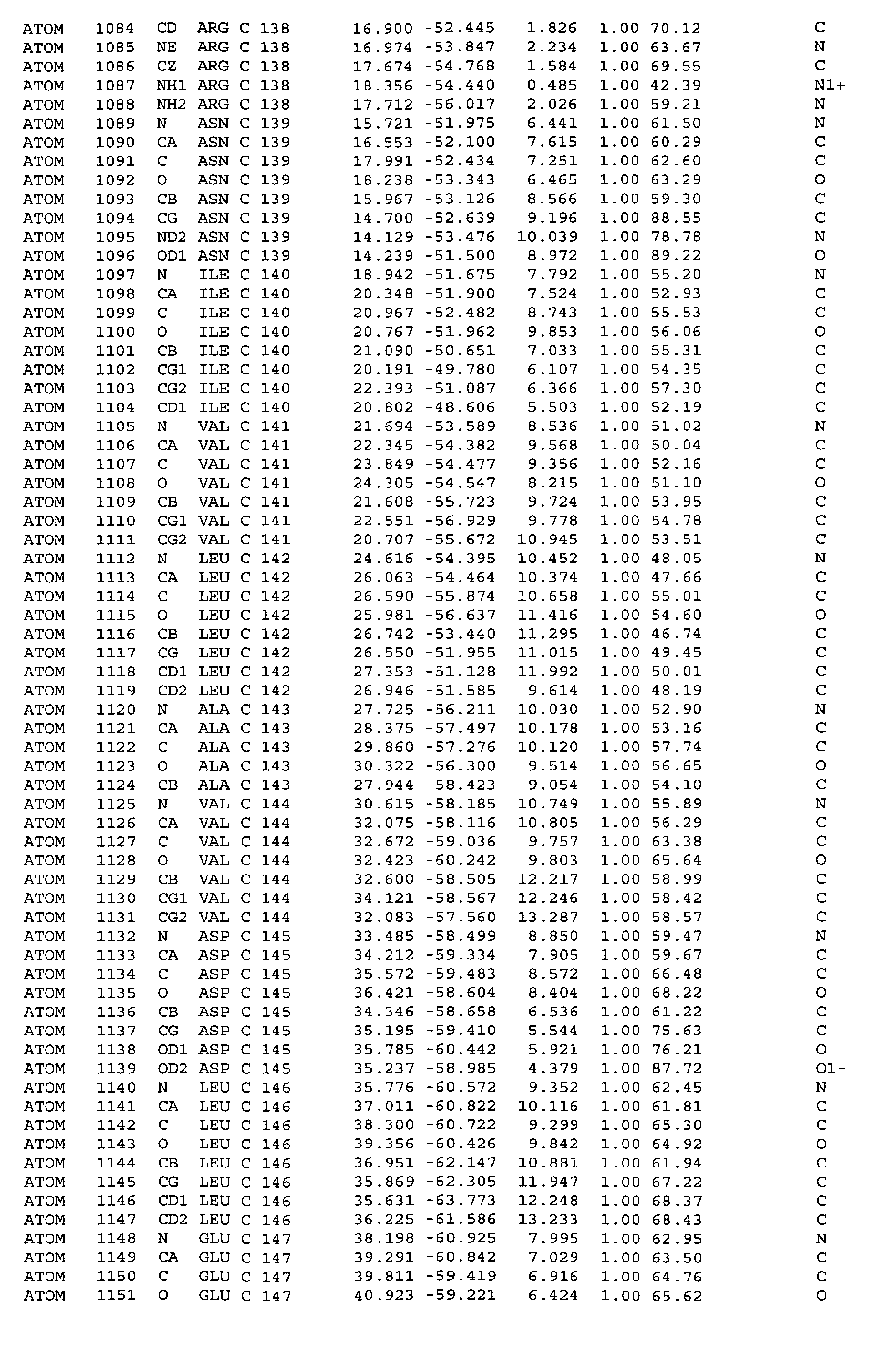

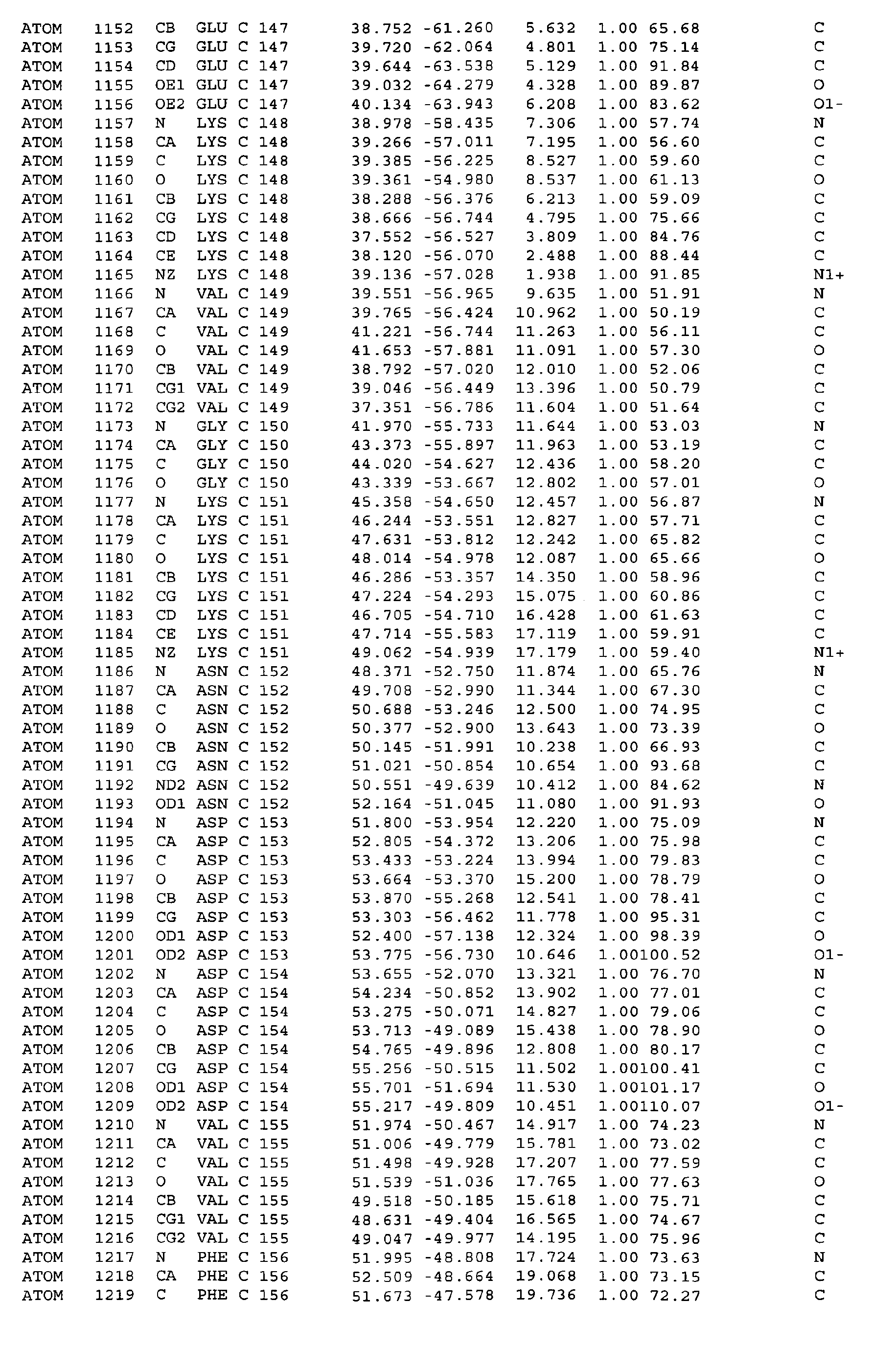

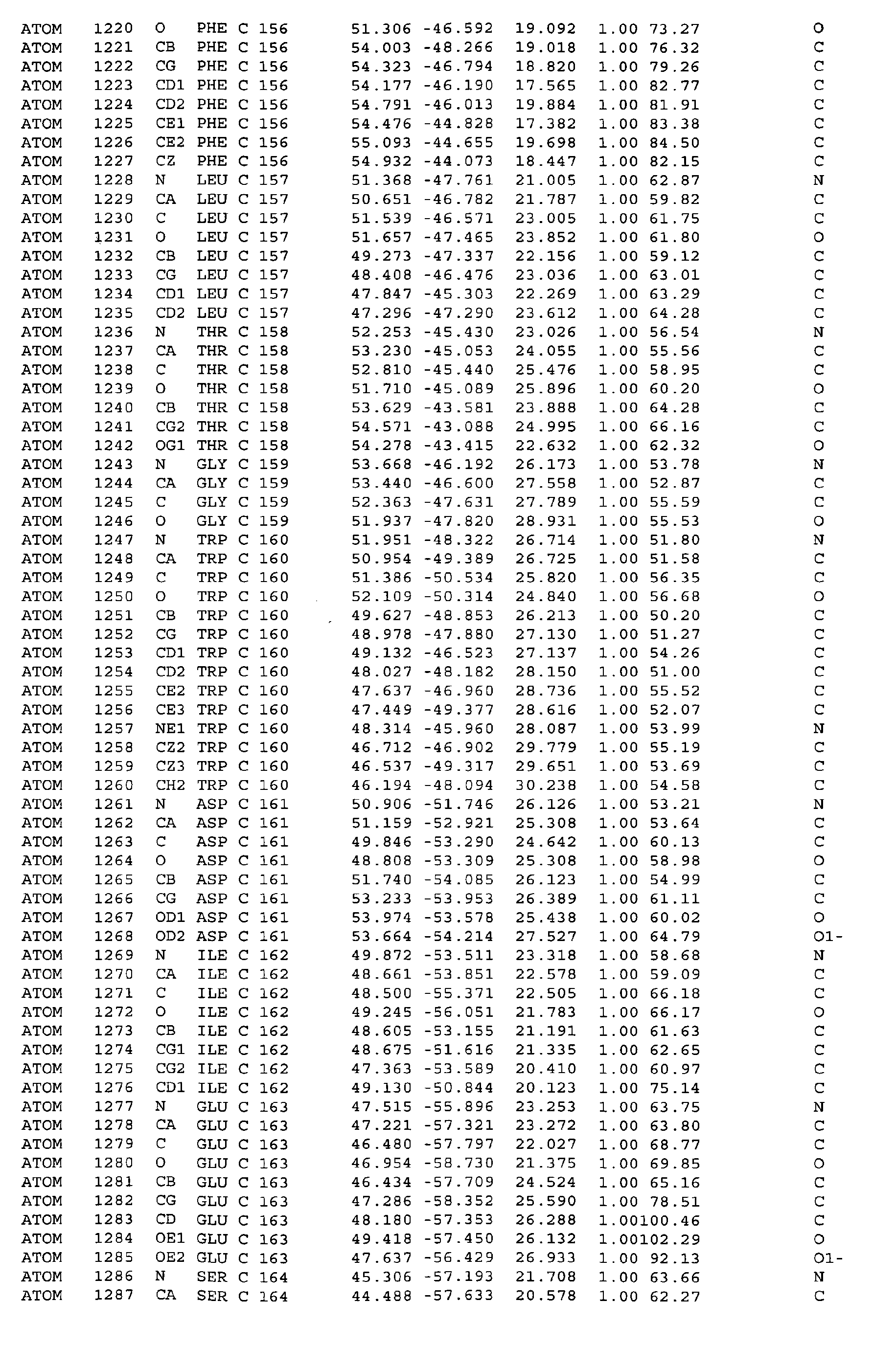

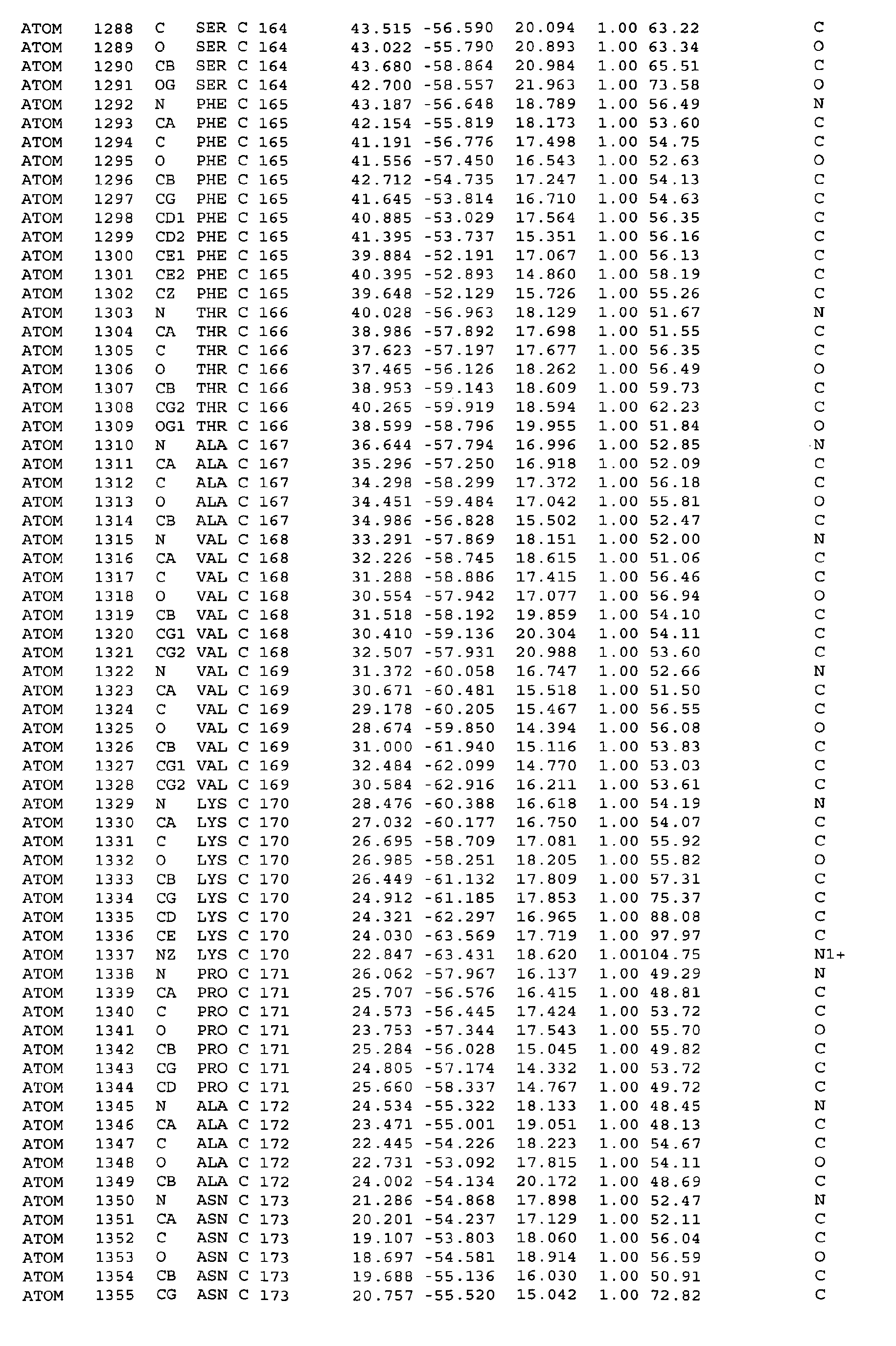

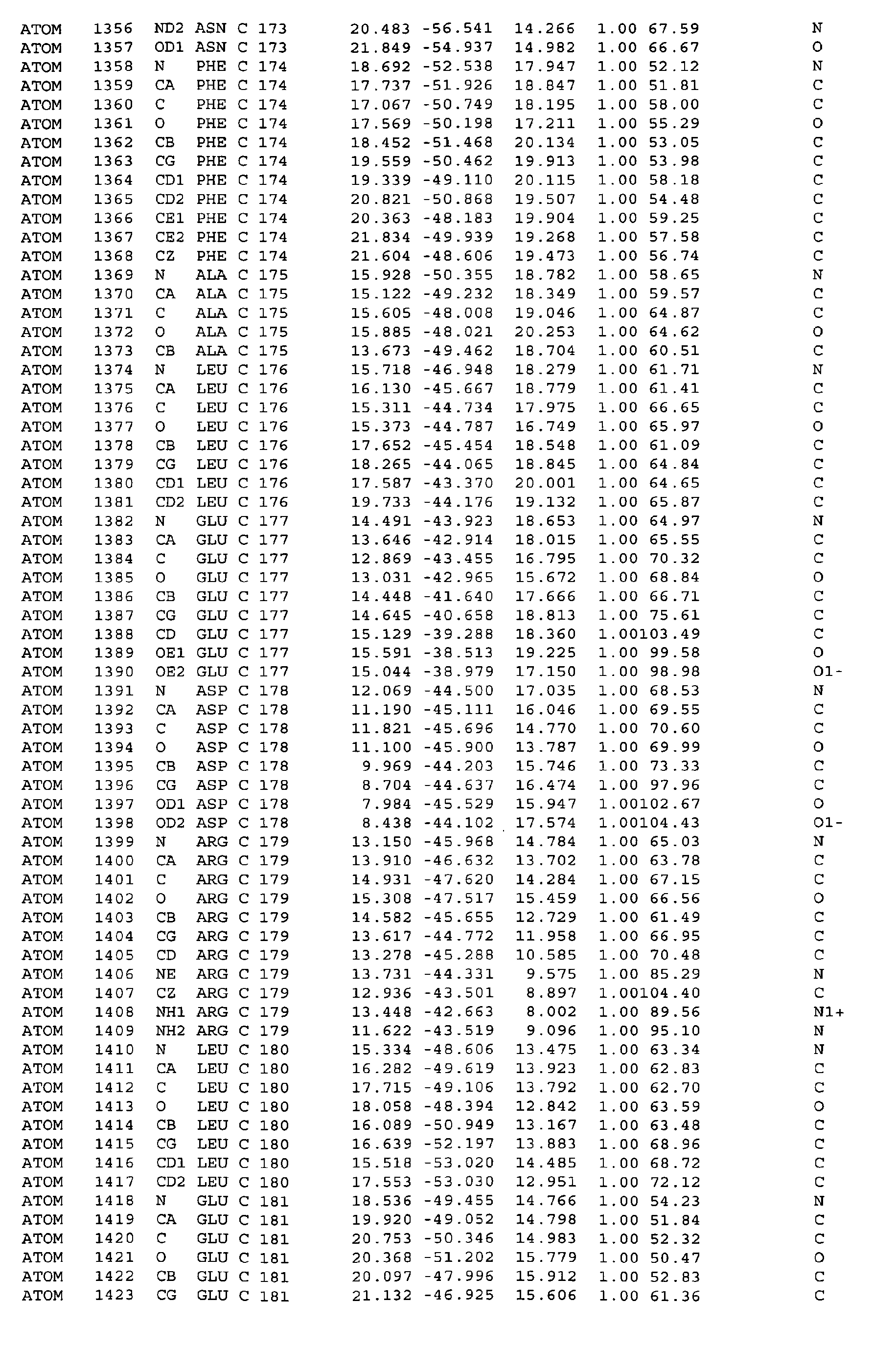

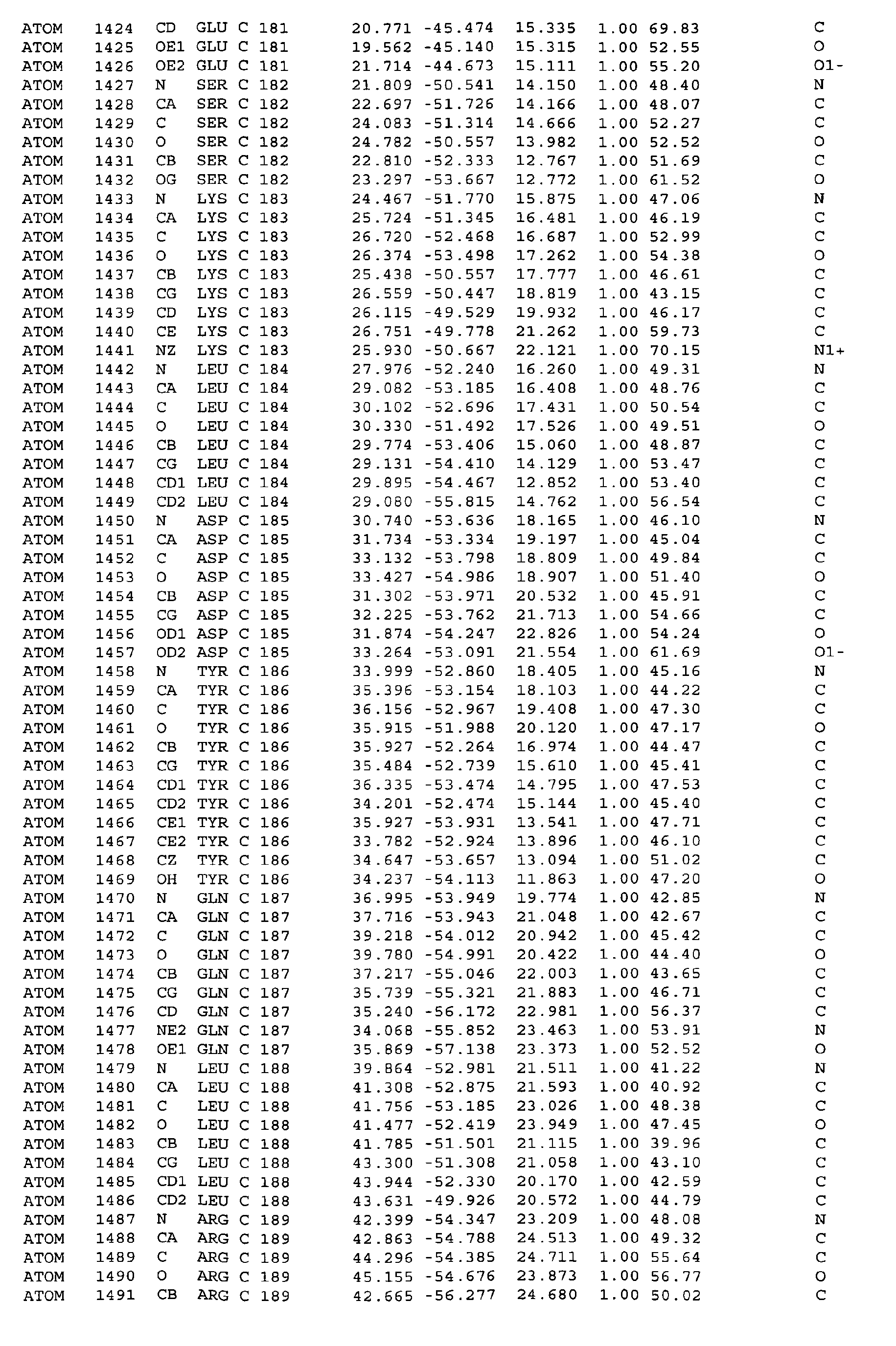

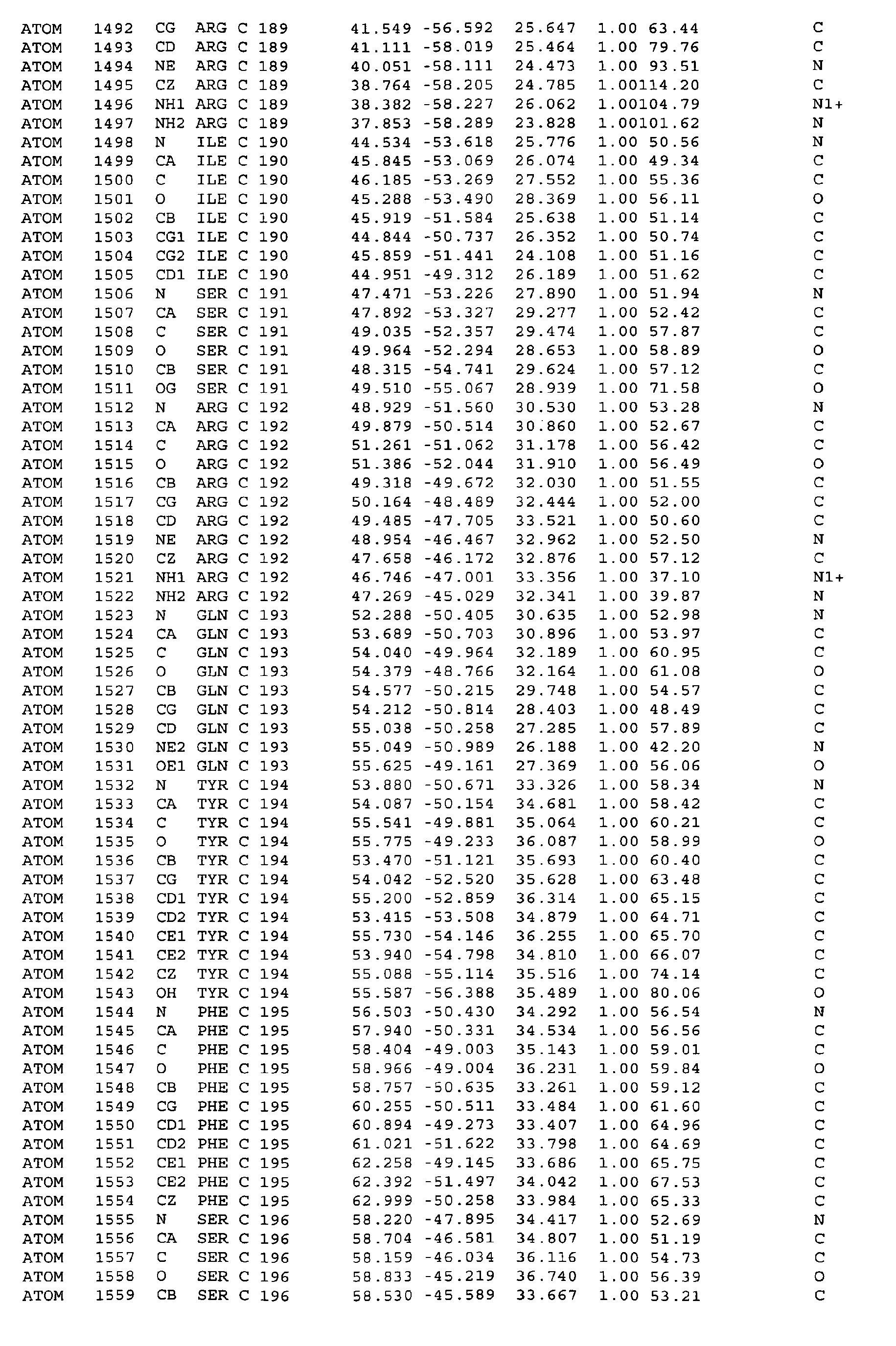

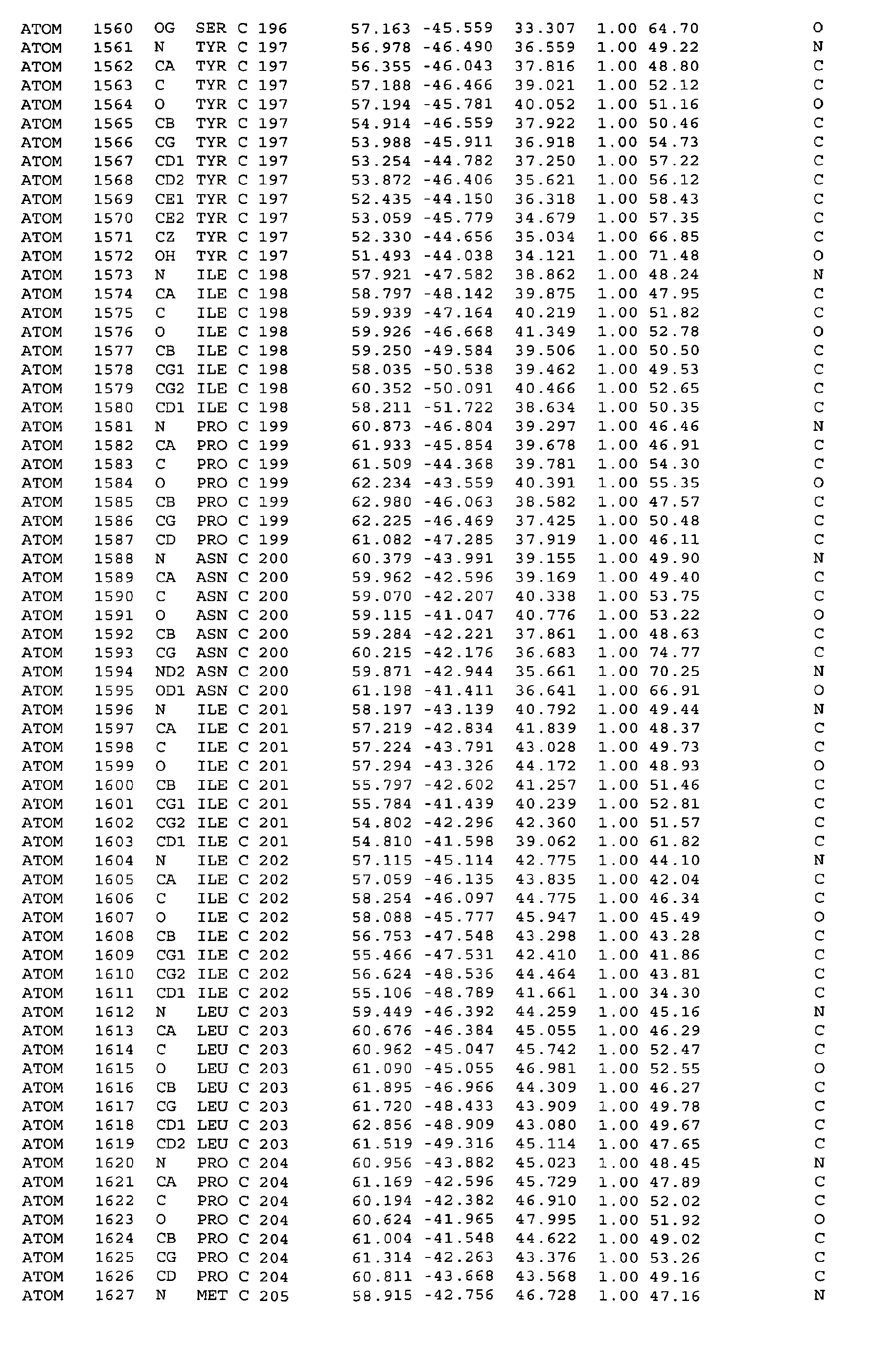

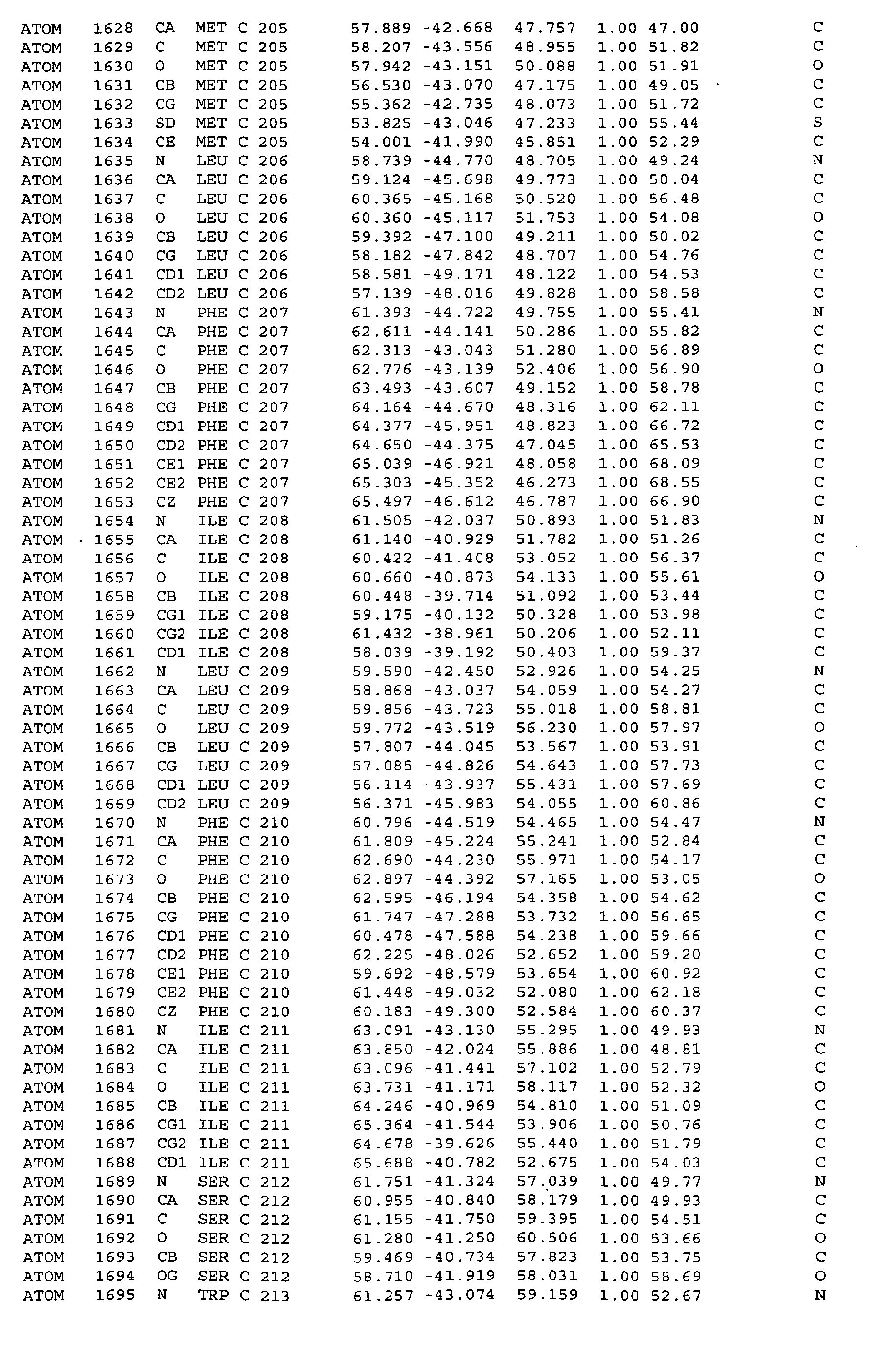

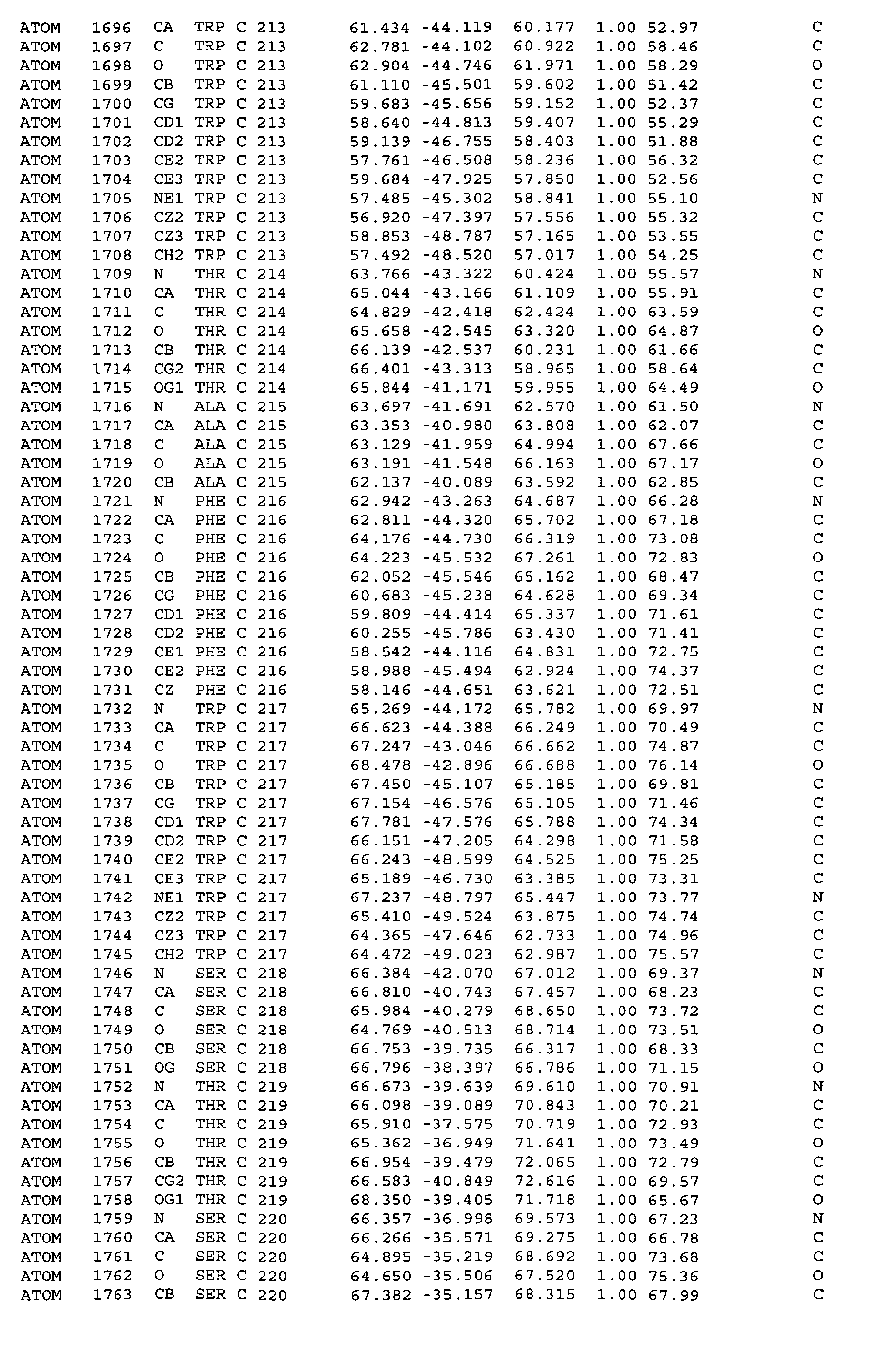

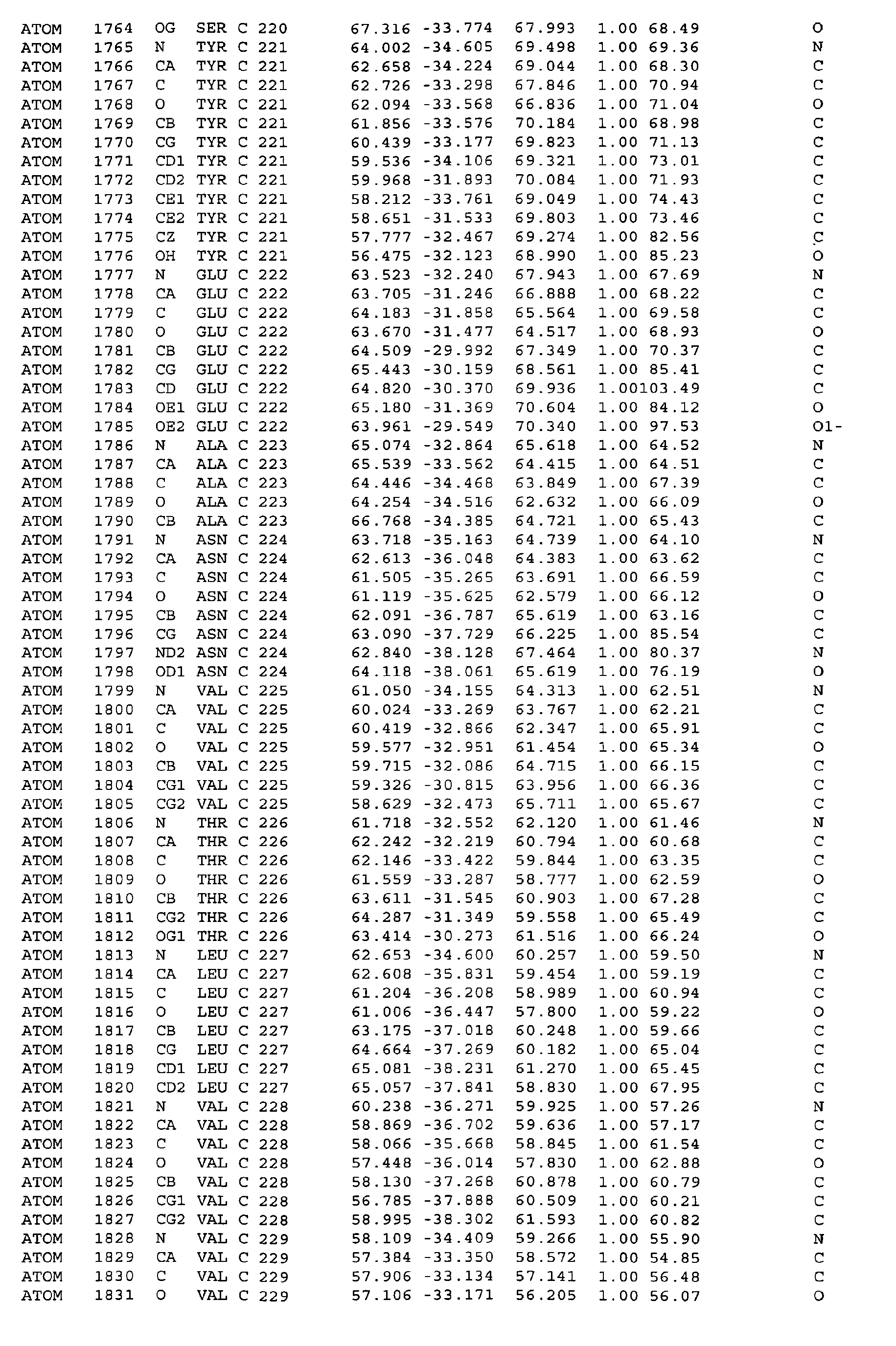

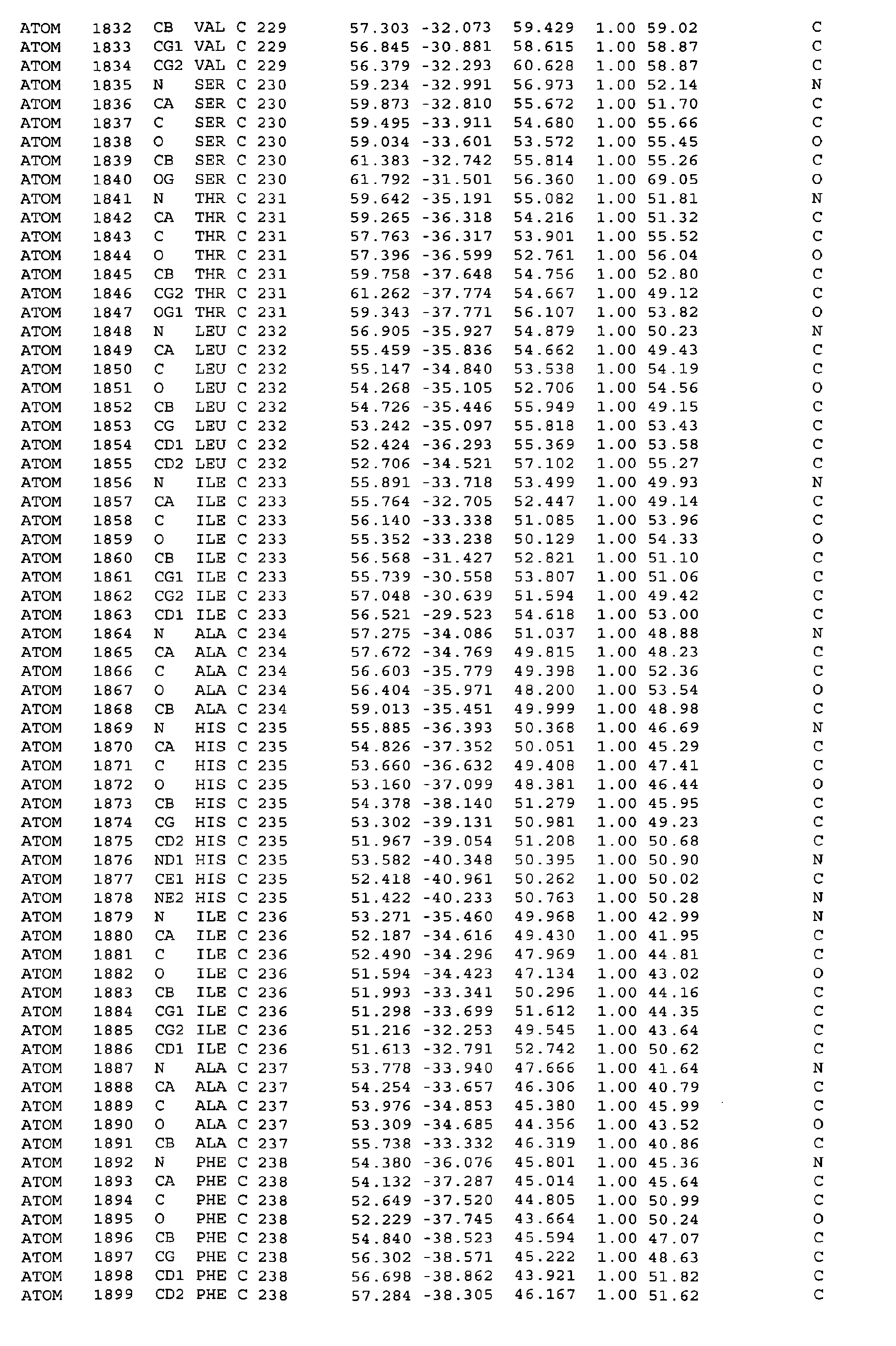

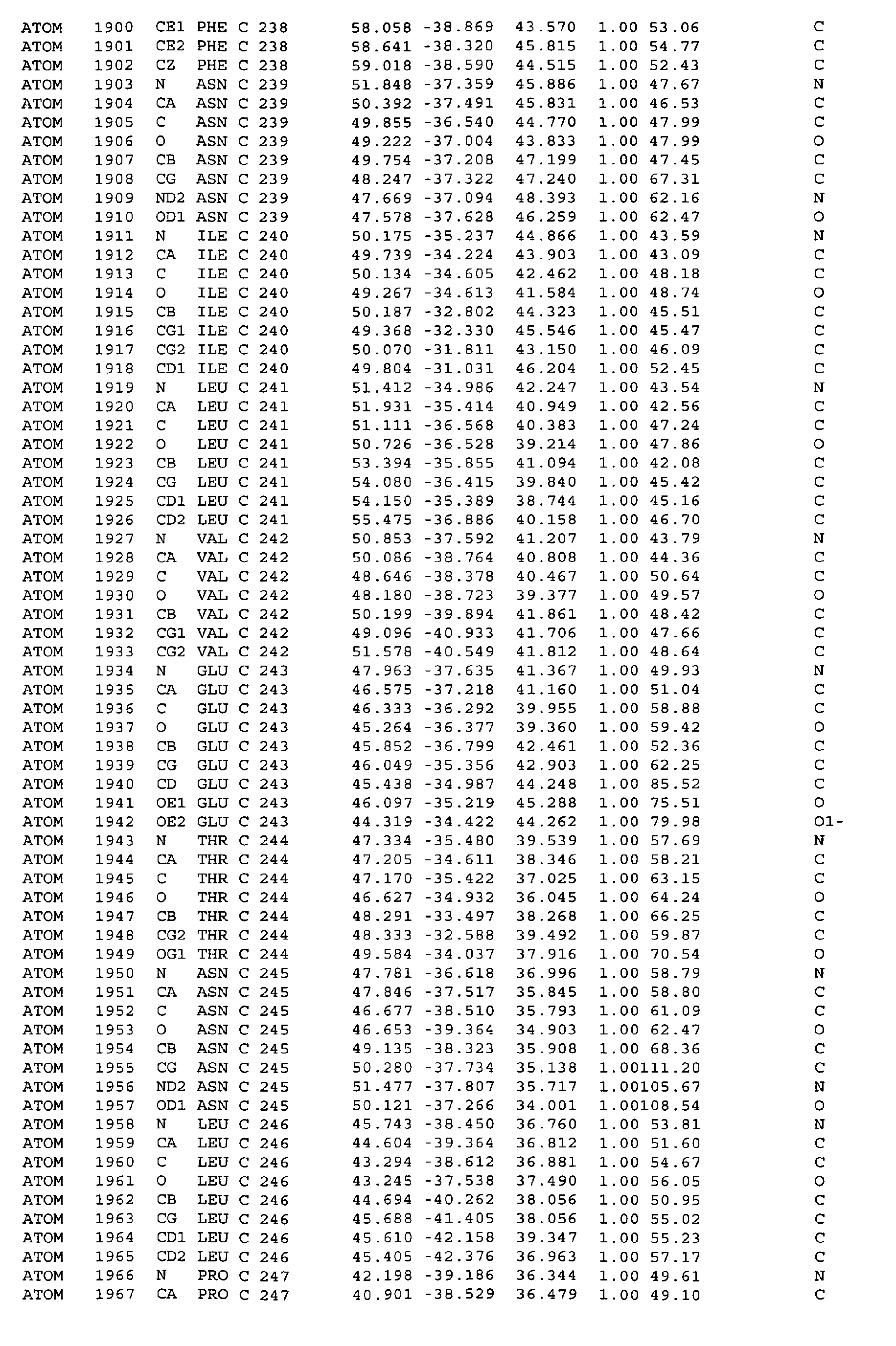

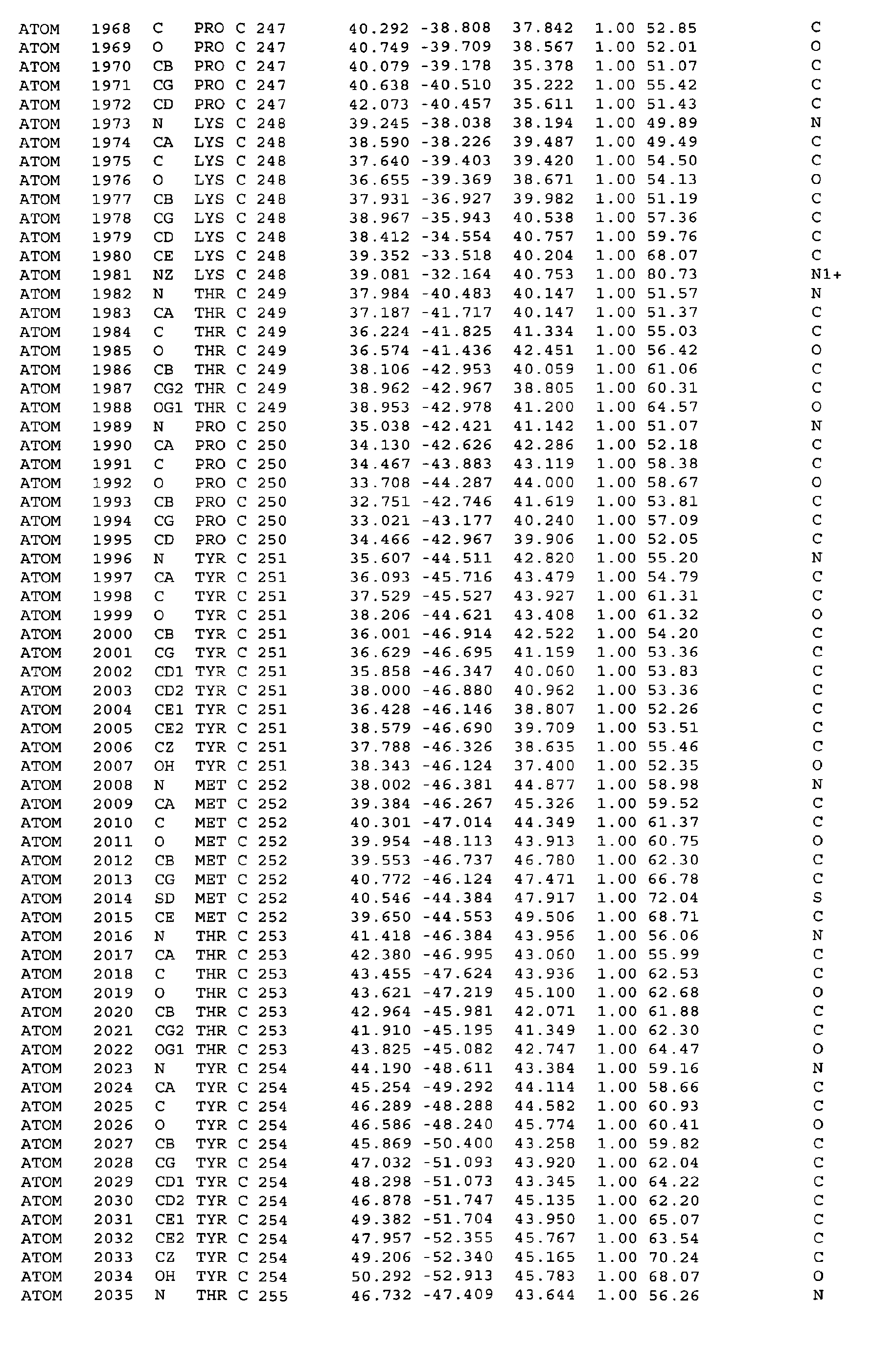

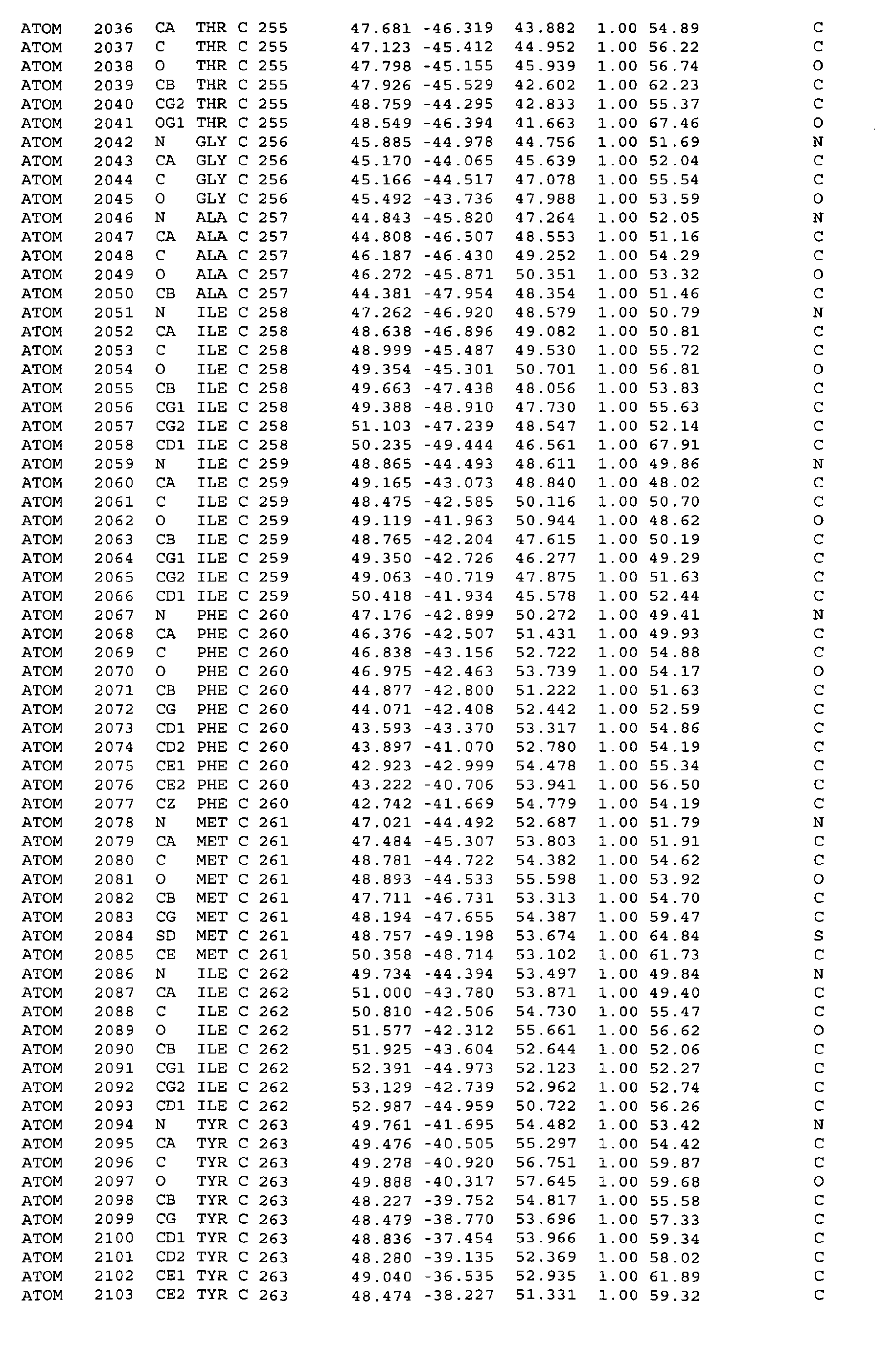

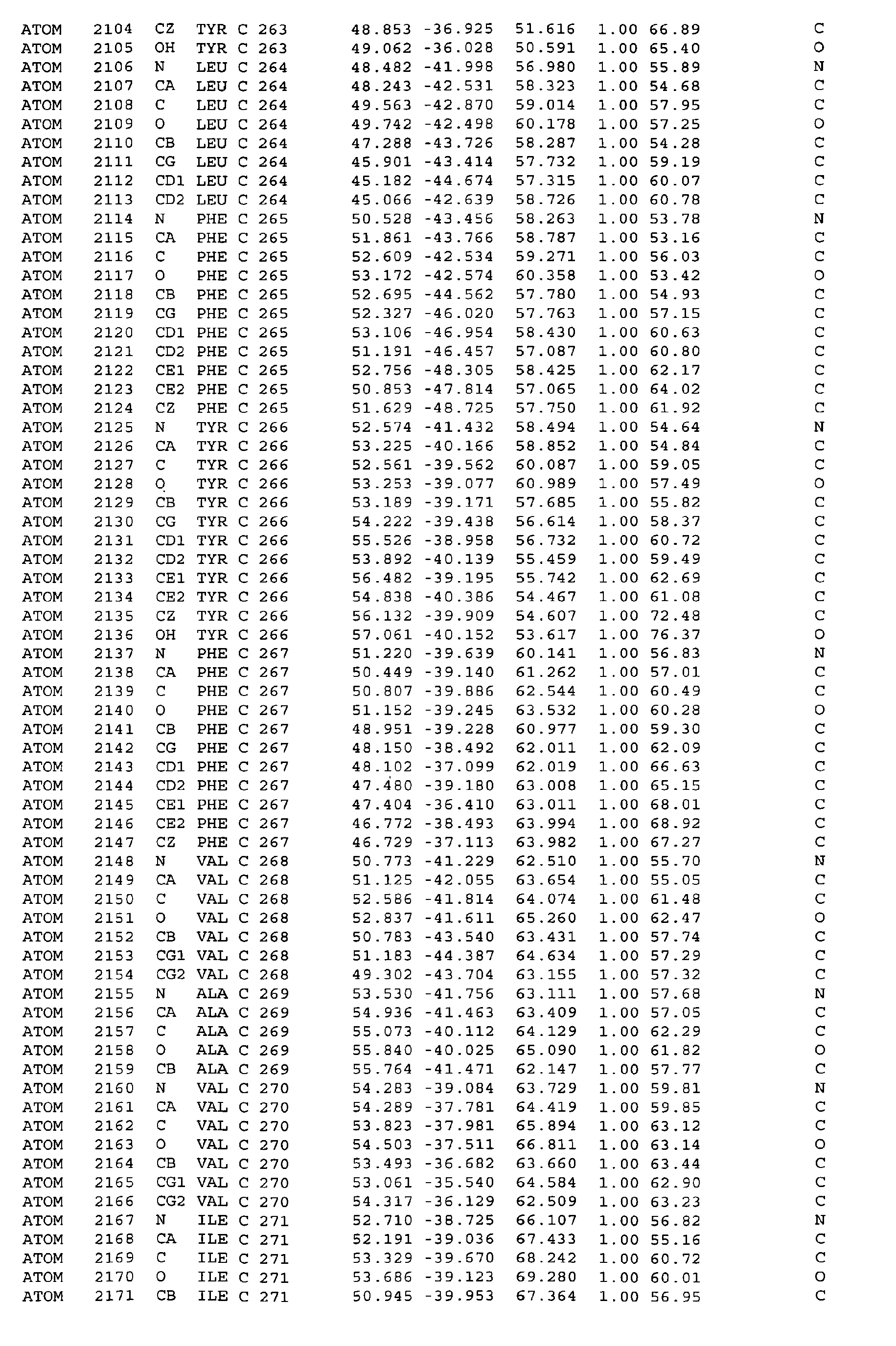

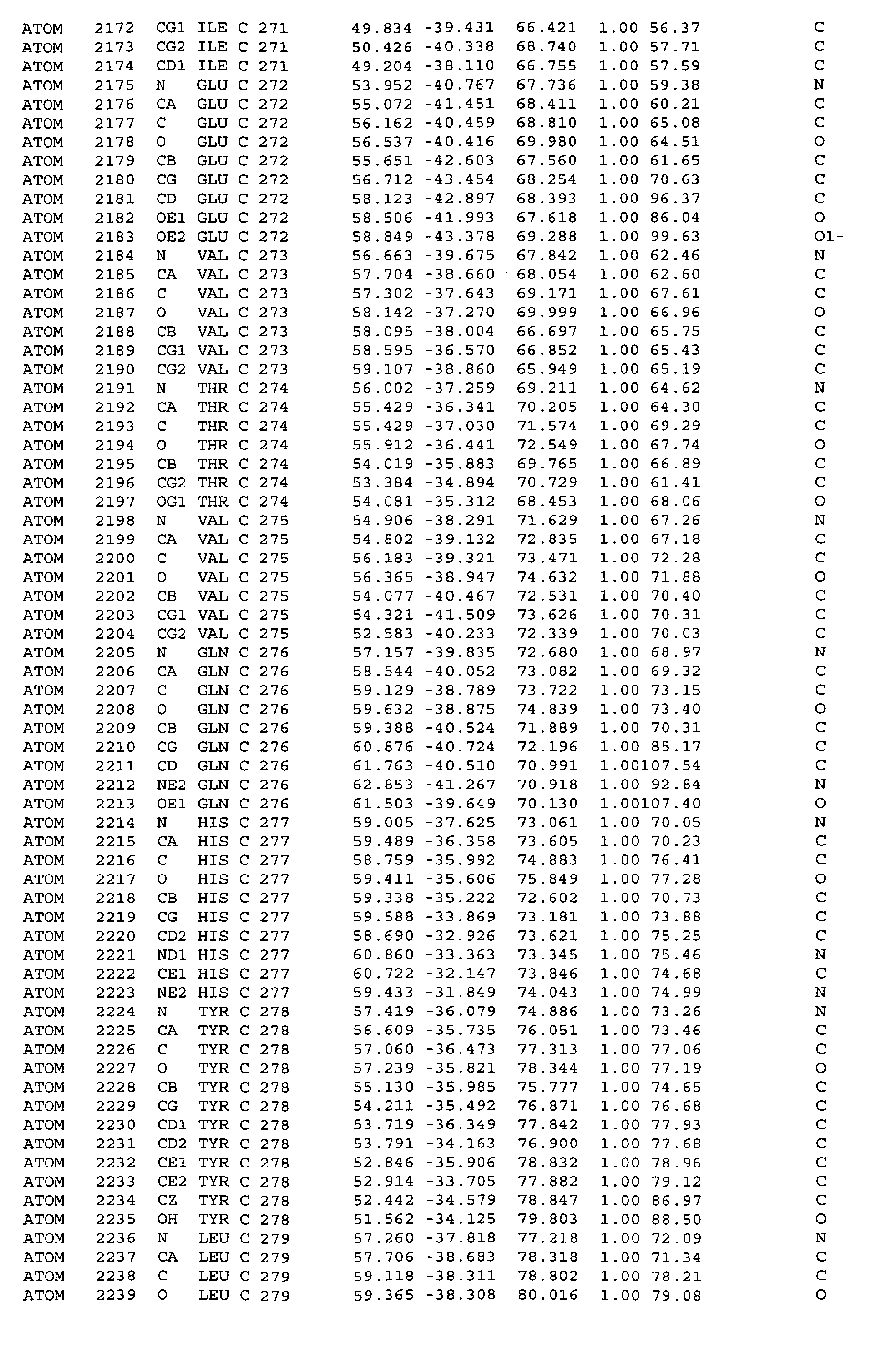

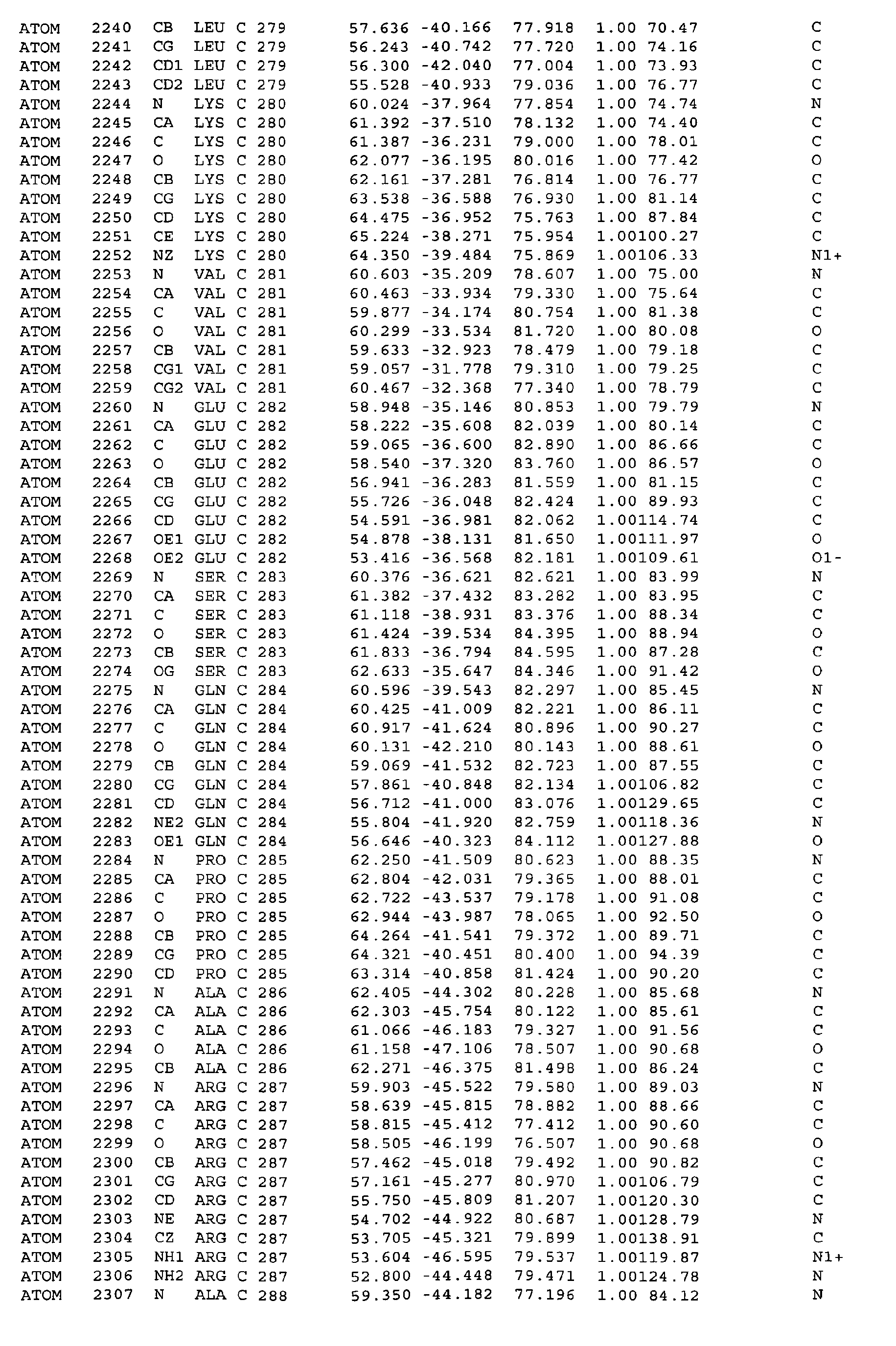

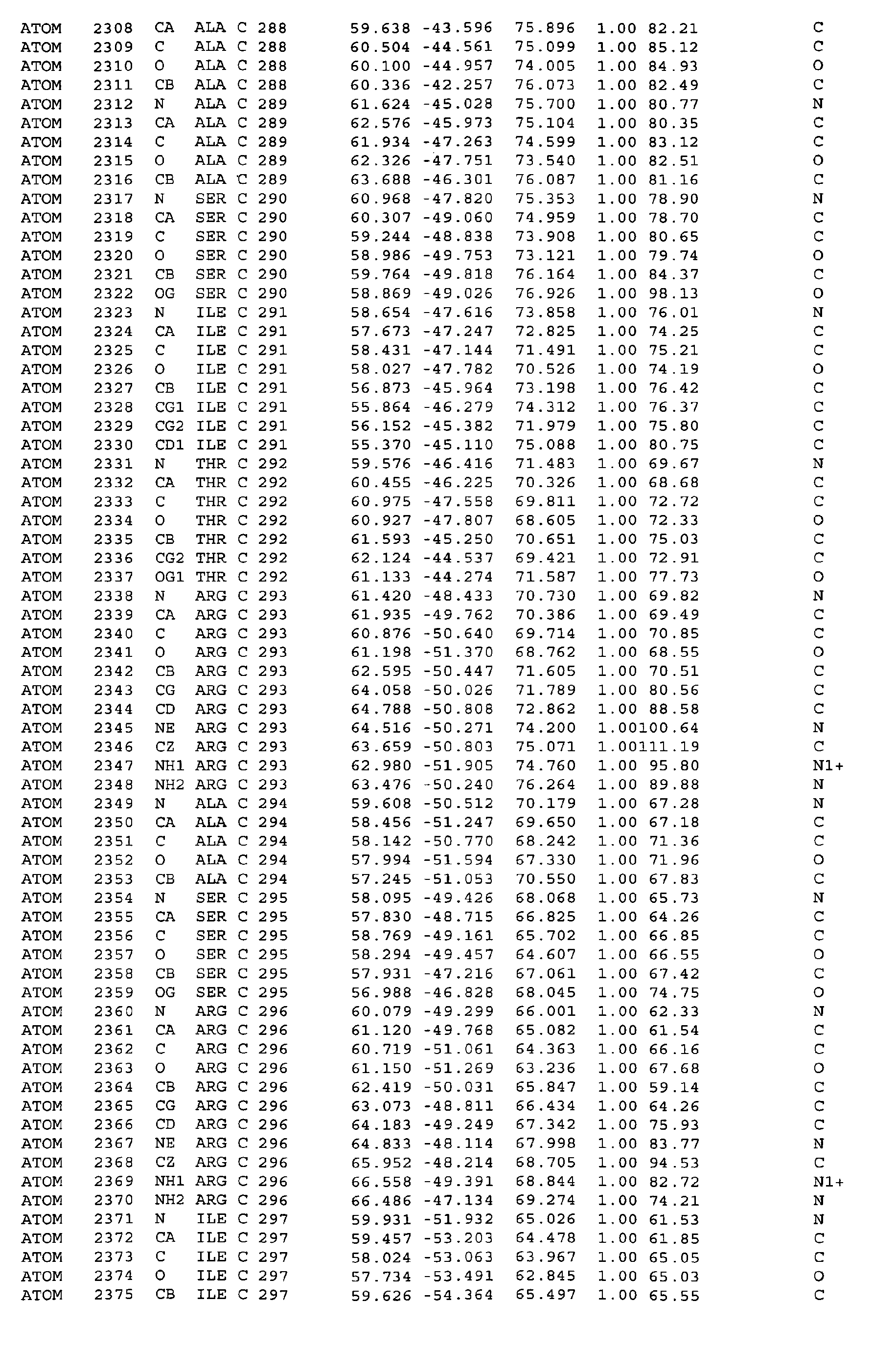

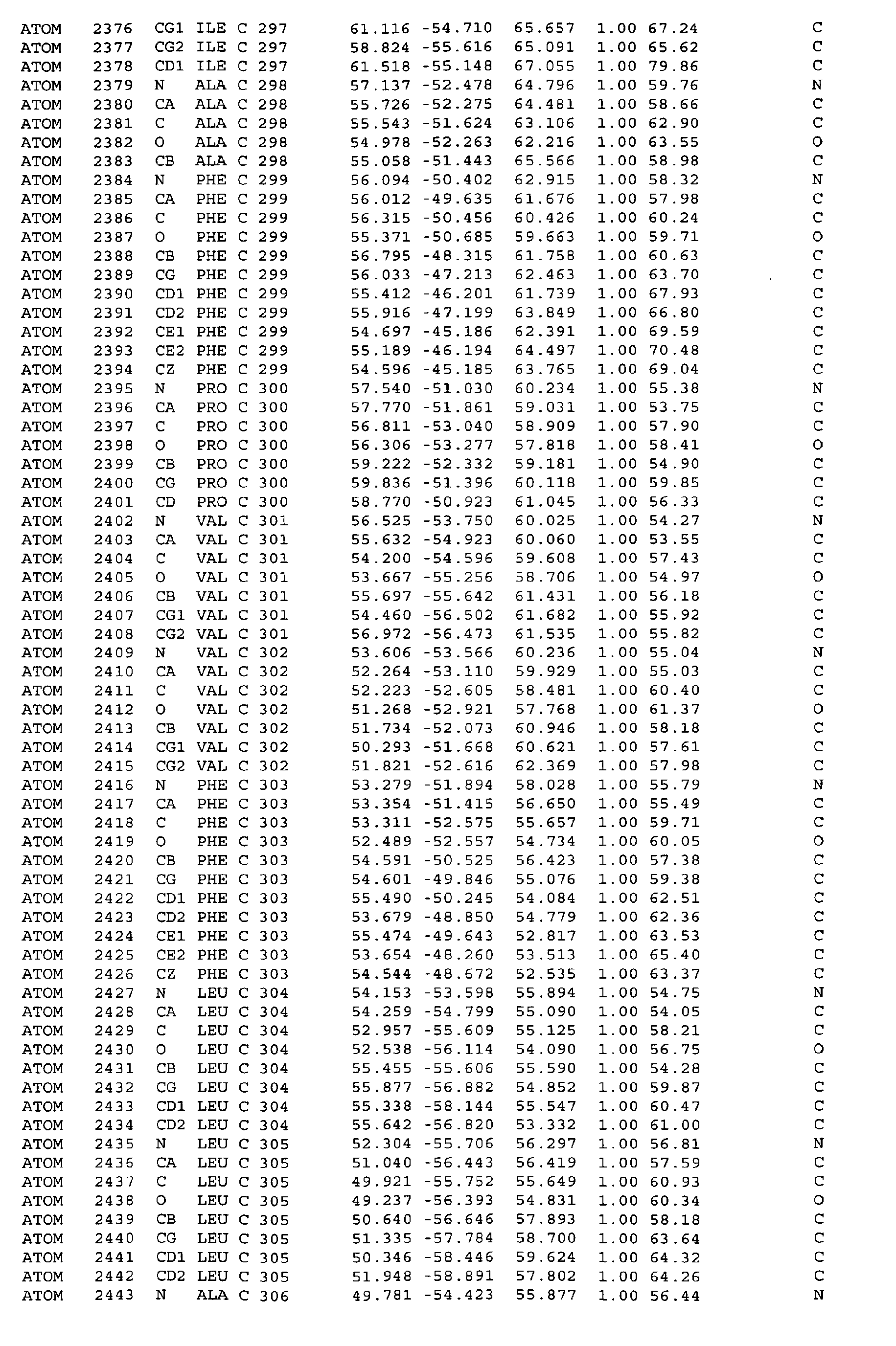

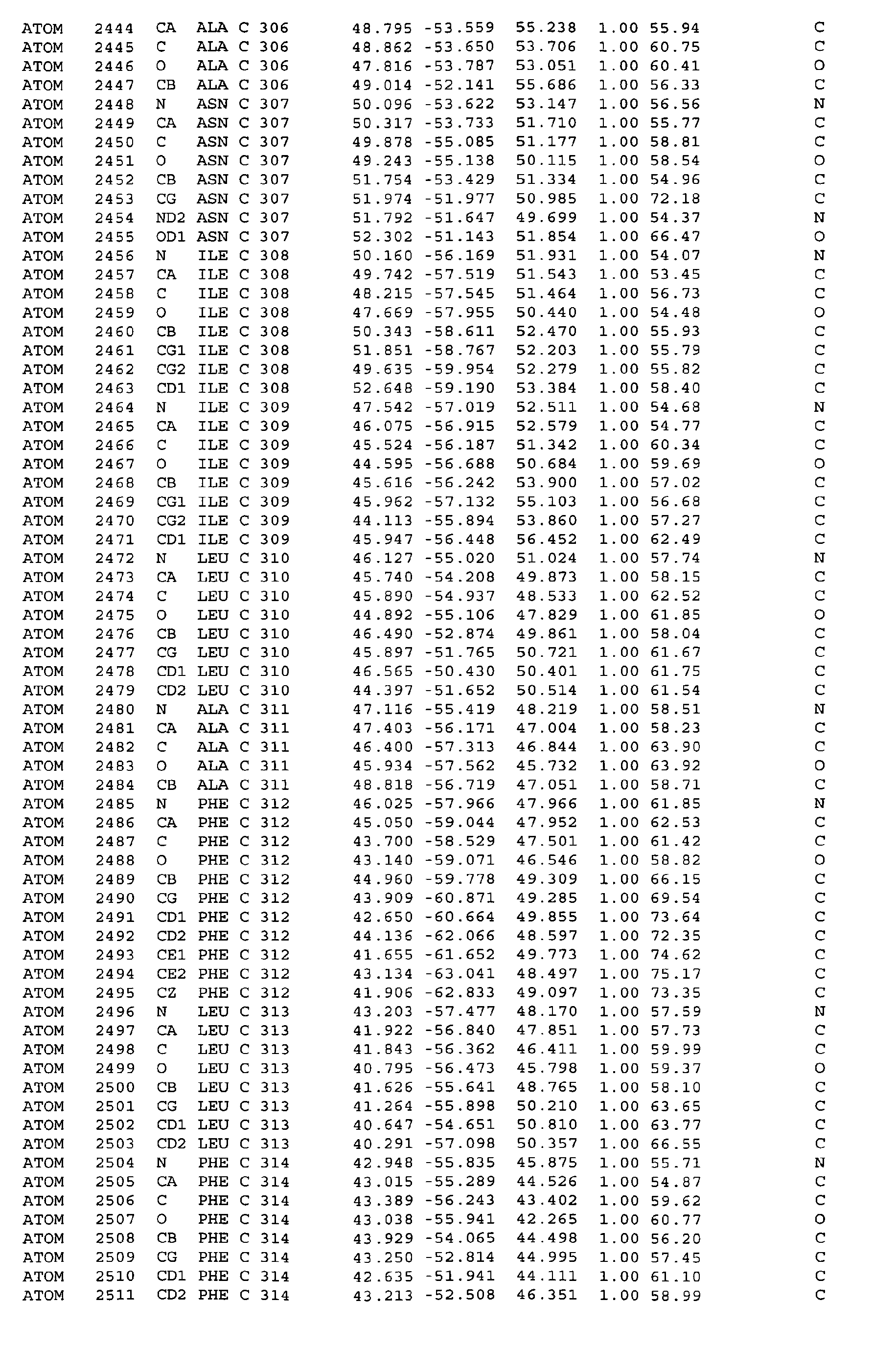

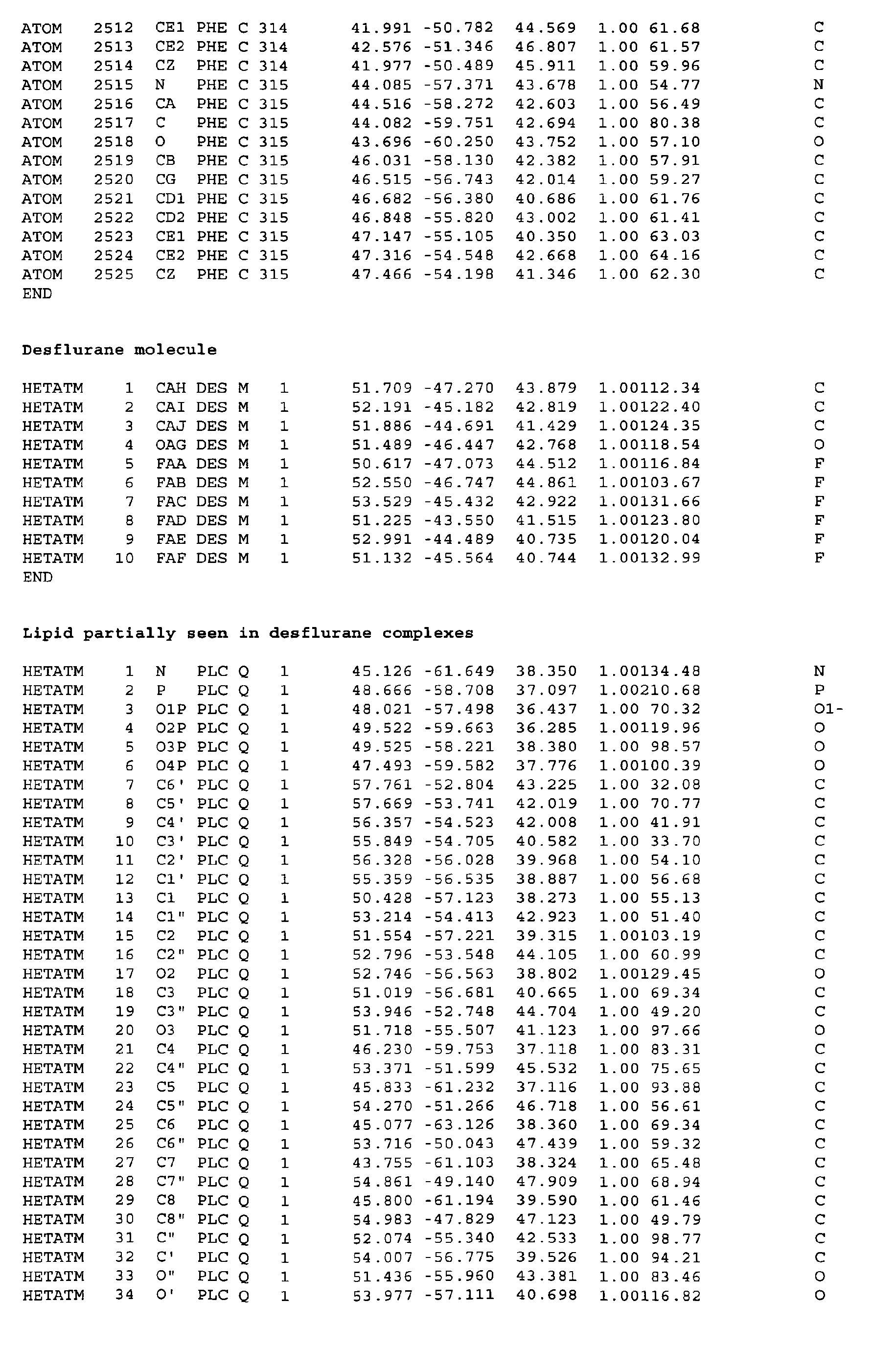

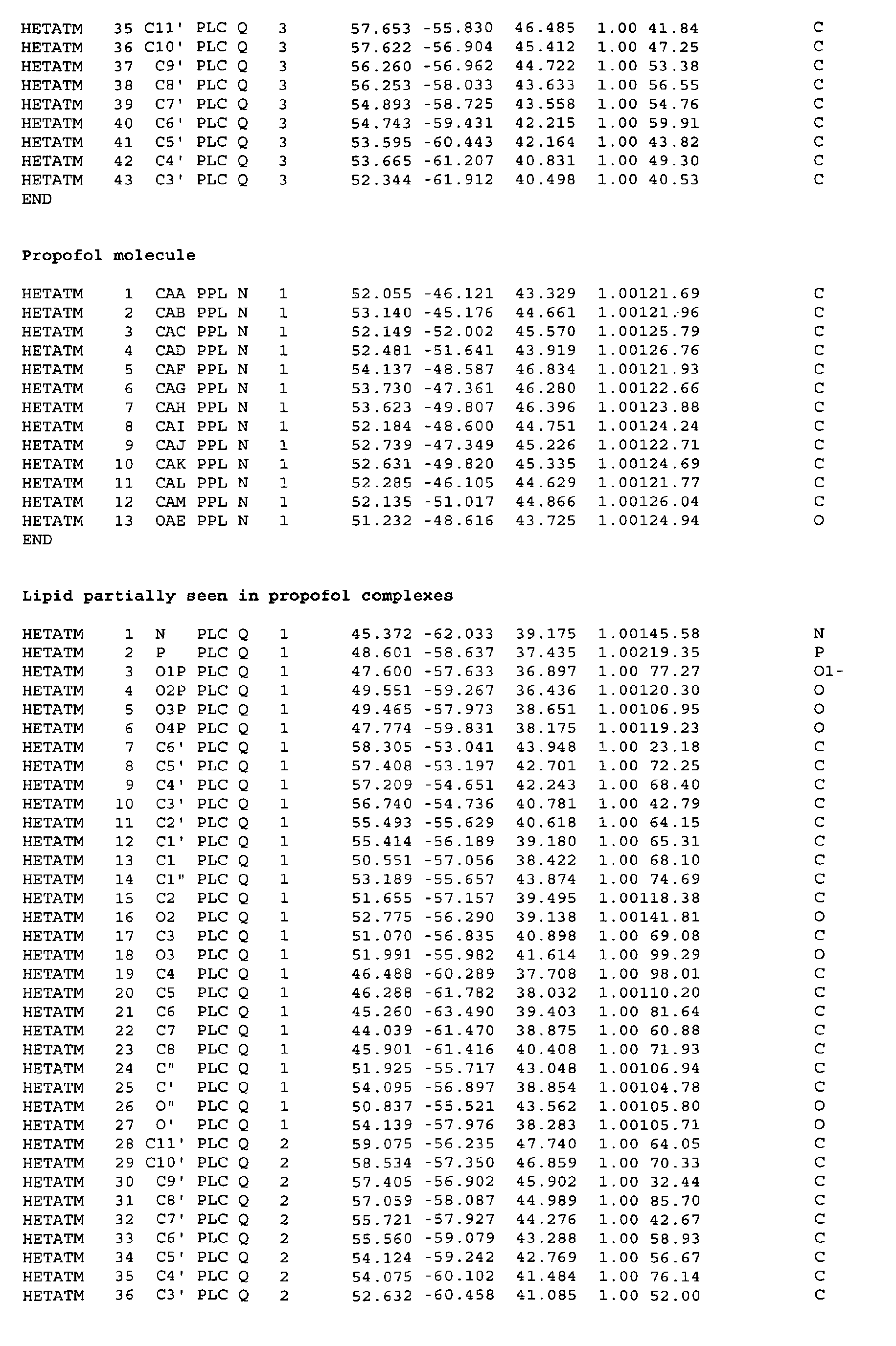

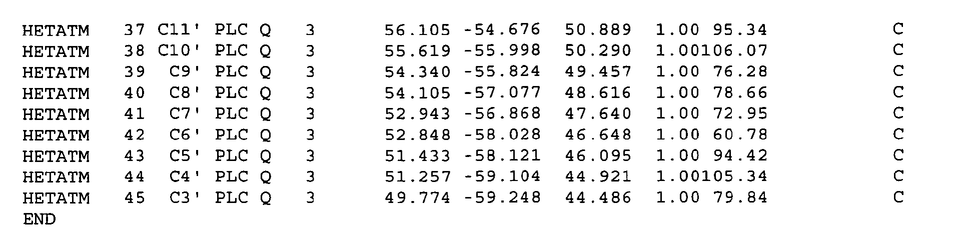

### EXAMPLES

General anesthetics are systematically used during surgery but their molecular mechanism of action remains poorly understood. These compounds are typically low molecular weight amphipatic molecules that are volatile or injected intravenously. Extensive work by molecular genetics indicates that they act by binding to specific protein targets, notably to ligand-gated ion channels.

The pentameric ligand-gated ion channel of *Gloebacter violaceus* (GLIC) is inhibited by most anesthetics at surgical concentrations. In the present experiments, GLIC was analyzed with the purpose of using it as a structural screen for the identification of neurotransmission inhibitors and stimulators.

The crystal structures of propofol/GLIC and desflurane/GLIC at 3.4-3.5 Å resolution, revealed a common anesthetics binding cavity, located inside the subunits that are contained in the transmembrane domain of each GLIC monomer chain (transmembrane segments M1, M2, M3, M4).

Said binding cavity is open to the cell membrane compartment, is close to the gate of the transmembrane channel on the backside of the M2 segment. The cavity is amphipatic and funnel shaped, compatible with accommodation of small molecules of different size, as illustrated by propofol which bound near the entrance, and the smaller desflurane which bound deeper inside.

Structures were solved at acidic pH with GLIC in an apparently open conformation, and therefore are not likely to correspond to the state stabilized by these negative allosteric modulators. However, two mutations (I202Y and T255A) within the cavity profoundly altered the gating transition of the channel, and two mutations (V242M and T255) differentially affected the sensitivity of GLIC for both anesthetics. These data point to a marked reorganization of the cavity in the course of the gating process, and support its contribution to both the intrinsic allosteric transition of the protein and the negative modulation by anesthetics.

The notorious complication to crystallize eukaryotic membrane protein has so far prevented their direct identification by X-ray crystallography.

The discovery of bacterial homologs of pLGICs (Tasneem et al. 2005, Bocquet et al, 2007) has allowed solving the first high resolution structures of two members of the family, namely the homolog from *Erwinia chrysanthemi* (ELIC, Hilf and Dutzler 2008) and from *Gloeobacter violaceus* (GLIC, Bocquet et al. 2009, Hilf and Dutzler 2009).

The GLIC protein functions as a proton-gated ion channel (Bocquet et al, 2007) and is robustly inhibited by most general anesthetics at clinical concentration, including volatile anesthetics such as desflurane, and also by propofol, etomidate and 3-hydroxybutyric acid. Here we present the crystal structures of GLIC in complex with two representative volatile and intravenous-administrated molecules widely used to induce and maintain anesthesia, *i.e*., desflurane and propofol.

### 1- X-ray structure of GLIC in complex with propofol and desflurane: identification of an intrasubunit transmembrane cavity that binds anesthetics.

The GLIC protein was produced as described in Bocquet et al. 2009 (fragment 43-359 of NP_927143).

Various general anesthetics assays for co-crystallization with GLIC were unsuccessful. Several hundreds of crystals (> 300) were tested that either did not diffract or did not contain the bound anesthetics, reflecting the difficulty in obtaining these structures. Desflurane is a liquid that quickly evaporates once the bottle is open (∼1 min) and propofol is a liquid that does not mix with water. The first issue we addressed was thus solubilizing the amphipatic drugs in buffers containing DMSO, detergents, alcohols that were compatible with the existing crystal conditions. Even though crystals still appeared in a mother solution supplemented with up to 2% DMSO and were shown to be highly diffracting, the drugs were not bound to the protein in any of these diverse solubilization conditions (range of non-polar additive added, mixtures...).

Two assays gave crystals with diffraction data unambiguously revealing a bound anesthetic.

These crystals were obtained with saturating concentrations of propofol or desflurane. GLIC was crystallized using the vapour diffusion method in hanging drops at 20°C. The concentrated (8-12 mg/mL) protein was mixed in a 1:1 ratio with the reservoir solution 12-16% PEG 4000, 400 nM (NH₄)SCN and 0.1M NaAc pH 4.0. The final pH was checked to be about 4.0 in the drop. Crystals were checked after 3-4 days, transferred in the mother solution supplemented with 20% glycerol for cryoprotection and with propofol or desflurane at saturating concentration, and flash-frozen in liquid nitrogen.

The conditions enabled both the persistence of the crystals and the presence of a high concentration of anesthetics:
- for propofol: mixing before crystallization 9/10 of a drop containing the mother solution and the protein solution and 1/10 of pure propofol. The smaller droplet is injected in the center of the bigger drop and very small crystals appear overnight close to the boundary between the two drops;
- for desflurane, it was better to grow crystals in the absence of anesthetics and then to rapidly add a huge amount of liquid desflurane in the mother liquor (and not directly in the protein drop); a two-day equilibration time allowed diffusion of the drop to the crystals.

The crystals were fished out of the drops and we incubated the freezing cryoprotectant solutions with anesthetics to minimize the risk of escape of the drugs out of their binding site during the process. All the freezing process was done as quickly as possible and we often fished only one crystal per drop.

The crystals were isomorphous and the solved structures showed a funnel-shaped ion channel with six molecules of bound detergent. The 3.4 Å resolution structure of desflurane-GLIC and the 3.5 Å resolution structure of propofol-GLIC present strong (>5 sigma) peaks in Fourier difference maps in all 5 subunits revealing the presence of anesthetics.

Desflurane and propofol were bound at the level of overlapping binding sites, within a cavity lying in the interior subunits in the upper half of the TMD (Figures 1A-1C, 2, 6A, 6B). The cavity had a mouth that opens to the outside where lipids are present in the apo structure. It was bordered on both sides by several residues from the M1 (I201, I202, M205 and L206) and M3 segments (Y254, T255, I258 and I259), M2 contributed a single residue at the back wall (V242), M4 contributed two residues near the mouth (N307 and F303), and three residues from the beta6-beta7 loop (Y119, P120, F121) constituted the "roof". The cavity was bordered by the side chains of 9 hydrophobic and 4 polar residues. In the present examples (and in the Figures), the amino acid positions are computed with the 43-359 fragment of the GLIC monomer amino acid sequence, *i.e*., with respect to SEQ ID NO: 2, whereas, in the body of the description, they are computed with respect to the full length GLIC sequence (*e.g*., with respect to the 359aa of SEQ ID NO: 1). For example, position 202 in the present examples and Figures is position 244 (202+42) in the body of the specification (*cf.* Table 2 above).

Desflurane was buried deep inside the cavity and interacted with M1 (1202, I203), M3 (T255 and I258) but also with the backwall M2 (V242). The electron density map did not allow assessing the exact orientation of the desflurane molecule at this resolution. Well-defined electron densities were observed that could be attributed to lipids. One alkyl chain is sterically blocking the cavity (Figures 1A, 1B, 6A, 6B).

Propofol was lying at the mouth of the cavity and protruded outward where it is expected to interact with lipids and/or detergents. Actually, it would clash with the capping lipids seen in the apo and desflurane structures, and consequently its presence must be associated with significant reorganization of the lipids surrounding the protein. Propofol is maintained in its position by a hydrophobic sandwich made up by side chain from M1 (notably I202) and M3 (notably T255 and 1258).

Altogether, these structures revealed that anesthetics bind at two locations within a continuous cavity that is open to the lipid medium and that progressively narrows toward the interior of the protein (Figures 2, 4A, 5A-5D). Such a cavity is expected to accommodate ligands of decreasing size when moving deeper inside the structure, a feature suggesting significant versatility of the pocket for small amphipatic molecules. The cavity ended up, 8 Å within the interior of the protein, with a narrow tunnel less than 3 Å in diameter, which communicates to another cavity located at the interface between subunits.

### 2- Mutational analysis of the anesthetics binding sites

Previous electrophysiological analysis had found that currents elicited by an EC20 concentration (pH 4.7-5.3) of protons on GLIC are inhibited by up to 90% by both propofol and desflurane, with IC50 and nH of respectively 0.6 microM (0.39) and 10 microM (0.24). The low Hill numbers suggest the occurrence of several anesthetics binding sites with different affinities. Thus these anesthetics preferentially bind and stabilize a closed-channel conformation of GLIC. It is noteworthy that the binding site unraveled by our X-ray analysis corresponded to an open form of the protein and it could thus be considered that it would not contribute to anesthetic inhibition. To check whether the binding sites contribute to anesthetics inhibition, we performed a set of mutations on residues directly contacting the anesthetics in the X-ray structures.

We monitored the inhibitory action of both anesthetics by two-electrode voltage clamp on oocytes expressing the GLIC channel. A typical experiment consists at activating the GLIC channel by an EC20 concentration of protons for 5 min, a period during which anesthetics are applied for 1min, 1 min after the onset of proton application. The current trace in figures 3A, 3B shows that a 1 min application of propofol at 10 micromolar produces a 28% inhibition of GLIC in a reversible manner. We found that a similar inhibition is observed when applying directly propofol with protons. In addition, we found that the inhibition by propofol is no longer observed at higher proton concentration (at the EC50).

Key residues contacting or neighboring propofol and desflurane in the 3D structure were mutated to alanine, or to more bulky residues in order to decrease the size of the binding pocket (mutants I202 A,Y,W, V242 A,M,W, T255A and I258A,M). All mutants yielded robust pH-elicited currents upon expression in the oocytes except the V242W for which no currents could be recorded and V242A which yielded small but significant currents. Mutants were first characterized by their trace currents and proton dose-response curve (figures 3C-3F). Reinvestigation of wild-type GLIC showed a proton pH₅₀ of 5.2 (nH=1.7). In addition, we found that activation by low proton concentrations elicits a typical slow activation followed by a stable plateau, whereas activation at very high concentrations, especially at supramaximal concentration (pH less than 4), elicited slow activation but a subsequent decrease of the response, a feature that suggests the occurrence of a desensitization process. Mutants I202A,W, V242A,M and I258A,M displayed trace currents and proton dose-response curves almost identical to that of the wild type. In contrast, I202Y and T255A displayed dose-response curves strongly shifted to the left, with pH₅₀ of respectively 6.1 (nH=2.1) and 6.0 (nH=1.1) pointing to a gain of function with increased sensitivity to proton. In addition, both mutations produced a slowing of the apparent kinetics of both activation and deactivation, pointing to a profound alteration of the gating process.

Screening the mutants for propofol inhibition by selecting activation at pH₂0 (pH 5.5 to 5.8 for all constructs except pH 6.5-6.8 for I202Y and T255A) revealed that I202A,W,Y and I258A,M were not significantly different from WT in these conditions, while V242A,M and T255A were more inhibited by propofol (figures 3C-3F). Comparison of propofol dose-inhibition curves of V242A,M, T255A and wt showed that the increased inhibition is the result of a shift of the dose-inhibition curve toward the left, pointing to an increased sensitivity for propofol.

For desflurane inhibition (figures 3C-3F), full dose-inhibition curves were measured at pH₂₀. It showed that I202A,Y,W and V242M did not alter significantly desflurane inhibition, while T255A produces a dramatic shift of the curve to the right, pointing to a decrease sensitivity of the mutant toward desflurane inhibition.

Altogether, this mutational analysis shows that, among the positions that were mutated within the anesthetics binding pocket, I202Y and T255A produce a marked gain of function phenotype with a one order of magnitude increased sensitivity for proton, V242M displays an increased sensitivity to propofol but not to desflurane, and T255A an increased sensitivity to propofol but a decreased sensitivity to desflurane.

### Discussion

### Identification of a transmembrane intrasubunit cavity that binds general anesthetics

Our work identifies for the first time an anesthetic binding pocket within the GLIC membrane protein. Anesthetics bind at the level of a cavity within the transmembrane domain, at the center of each subunit. The cavity is largely hydrophobic in nature but also contains several polar residues, a feature which fits the hydrophobic but partially polar character of anesthetics. The cavity is open to the membrane compartment, and progressively narrows when moving inside the protein. Such a shape is expected to accommodate ligands of decreasing size when moving deeper inside the structure, as exemplified by desflurane which is bound deep inside the cavity while the larger propofol molecule is apparently blocked at the mouth entrance. This feature suggests a versatility of the pocket for binding a wide range of small amphipatic molecules.

### The cavity undergoes structural reorganization i the course of channel opening

Our mutational analysis of selected residues from the cavity highlights two positions, the mutation of which strongly alters the gating process. I202Y and T255A produce gain of function phenotypes, characterized by a 10-fold increase in proton sensitivity.

These data point to a change in environment of positions 202 and 255 in the course of receptor gating, and thus to a reorganization of the cavity.

Strikingly, the cavity is close to the gate of the channel, i.e., the regions that narrows to impair ion diffusion in the closed conformation. This region is made up of three rings of hydrophobic residues at position 9', 13' and 16' of the M2 segment that lines the pore. The cavity is located on the back-side of the M2 segment out from the channel. Channel opening is thus expected to imply a large motion of this portion of the M2 segment, thereby producing a marked reorganization of the cavity.

### Contribution of the intrasubunit cavity to general anesthetics mediated inhibition

Most tested general anesthetics act as negative allosteric effectors on GLIC. They decrease the current elicited by an EC20 concentration of proton in a dose-dependence manner. This indicates that these compounds bind preferentially and thereby tend to stabilize a closed-channel conformation. Still, the present X-ray structures showed GLIC in an apparent open conformation, probably because the pH 4.6 used for crystallization is supramaximal and overcomes the inhibition of the anesthetics, even if they showed nearly maximal occupancy of the five binding sites. Therefore, we asked whether the intrasubunit cavity is also the binding site for these anesthetics in a closed channel conformation, and mediate their inhibitory property.

To test this idea, we mutated selected residues that directly contact propofol and/or desflurane in the 3-D structures. For desflurane, we found no effect when the relatively distant positions 202 and 242 are mutated. In contrast T255, which extensively contacts desflurane, produces a strong decrease in the sensitivity of GLIC for desflurane when mutated to alanine. These data provide evidence for a conserved binding site for desflurane in the closed conformation, where a local reorganization would increase its affinity for GLIC in the closed state. In the case of propofol, mutation T255A and V242A,M were found to increase the sensitivity of GLIC for propofol inhibition, while mutation of I202 has no effect. These data are difficult to reconcile with the X-ray structure, since position 202 and 255 do contact propofol, but not 242 which is distant from propofol by 4 Å. One possibility is that propofol, which is apparently too bulky to reach the interior of the cavity in the open conformation, would reach this level in the closed conformation to produce inhibition. In line with this idea, mutation that increase propofol inhibition all decrease the volume of residues and thus increase the volume of the cavity to accommodate the larger propofol.

Altogether, our data point to the intrasubunit cavity as mediating general anesthetics inhibition of GLIC:
1) it binds desflurane and propofol in X-ray experiments,
2) its mutation alters the sensitivity of these anesthetics for GLIC,
3) its mutation strongly alter the gating process and
4) It is expected to undergo a major structural reorganization in the course of gating.

Still, the X-ray data showed anesthetics bound to an open conformation, and the exact binding mode in the closed conformation implies significant reorganization of the cavity and possibly motion of the anesthetics molecules. Notably, our data suggest that the propofol binding site that mediates inhibition could be closer to the M2 segment than actually observed with the 3D structure.

### Conclusion

Overall, the emerging picture points to two neighboring cavities involved in anesthetics actions on pLGICs, an intra-subunit cavity identified herein on GLIC and an inter-subunit cavity identified on anionic eukaryotic receptors. Both cavities are located within the TMD close to the gate of the channel, on the back side of the M2 segments, and are thus expected to undergo major reorganization in the course of the opening-closing motions, consistent with their contribution to modulation by allosteric effector. The present experiments suggest that, in the case of GLIC, reorganization of the intrasubunit cavity in the course of channel closing is associated with significant increase in affinity for anesthetics, thereby providing stabilization of the closed channel conformation.

### BIBLIOGRAPHIC REFERENCES

Bocquet et al. 2007; A prokaryotic proton-gated ion channel from the nicotinic acetylcholine receptor family. Nature (2007) vol. 445 (7123) pp. 116-9
Bocquet et al. 2009; X-ray structure of a pentameric ligand-gated ion channel in an apparently open conformation. Nature (2009) vol. 457 (7225) pp. 111-4
Brejc et al. 2001; Crystal structure of an ACh-binding protein reveals the ligand-binding domain of nicotinic receptors. Nature (2001) vol. 411 (6835) pp. 269-76
Brunger et al. 1998; Crystallography & NMR system: a new software suite for macromolecular structure determination; Acta Crystallogr. D Biol. Crystallogr. 54, 905-921.
Hilf et al. 2008; X-ray structure of a prokaryotic pentameric ligand-gated ion channel. Nature (2008) vol. 452 (7185) pp. 375-9
Hilf et al. 2009; Structure of a potentially open state of a proton-activated pentameric ligand-gated ion channel. Nature (2009) vol. 457 (7225) pp. 115-8
Ho and Gruswitz 2008; Generating Accurate Representations of Channel and Interior Surfaces in Molecular Structures; BMC Structural Biology 8(49).
Mihic et al. 1997; Sites of alcohol and volatile anaesthetic action on GABA(A) and glycine receptors. Nature (1997) vol. 389 (6649) pp. 385-9
Miyazawa et al. 2003; Structure and gating mechanism of the acetylcholine receptor pore. Nature (2003) vol. 423 (6943) pp. 949-55
Tasneem et al. 2005; Identification of the prokaryotic ligand-gated ion channels and their implications for the mechanisms and origins of animal Cys-loop ion channels. Genome Biol (2005) vol. 6 (1) pp. R4

## Claims

1. **A crystal,** which comprises:
- **at least one monomer chain** of the pentameric ligand-gated ion channel of *Gloebacter violaceus* (GLIC), or at least one fragment thereof, which comprises a transmembrane sub-domain of said at least one GLIC monomer chain, wherein said transmembrane sub-domain is a fragment of the transmembrane domain of said GLIC monomer chain, which consists of, or comprises, the sequence extending from the first N-terminal amino acid of the segment M1 to the last C-terminal amino acid of the segment M4 of said GLIC transmembrane domain, for example at least one fragment of GLIC monomer chain, which comprises the sequence of SEQ ID NO: 8,
and which comprises
- **at least one compound bound** to said transmembrane sub-domain of said at least one GLIC monomer chain or fragment thereof,
wherein said at least one bound compound is in a cavity located **between the M1, M3 and M4 segments** of said at least one GLIC monomer chain or fragment thereof,
said at least one bound compound optionally being, or optionally comprising, at least one amphipatic or non-polar molecule, preferably at least one amphipatic molecule.

2. The crystal of claim 1, wherein said at least one bound compound is in a cavity located between:
- on a first side, the amino acids of said M1 segment, which are at **positions 243, 244, 247 and 248** in the full length amino acid sequence of said at least one GLIC monomer chain, and
- on a second side, the amino acids of said M3 segment, which are at **positions 296, 297, 300 and 301** in the full length amino acid sequence of said at least one GLIC monomer chain,
- on a third side, the amino acids of said M4 segment, which are at **positions 345 and 349** in the full length amino acid sequence of said at least one GLIC monomer chain,
and/or said at least one bound compound is in a cavity located between:
- on a first side, the atoms of said M1 segment, which have the **atomic coordinates** that are given under the ATOM field for residue sequence numbers 201, 202, 205 and 206 in **Table 1**, or coordinates which differ from these coordinates by a root mean square deviation (rmsd) of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and
- on a second side, the atoms of said M3 segment, which have the atomic coordinates that are given under the ATOM field for residue sequence numbers 254, 255, 258 and 259 in Table 1, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;
and
- on a third side, the atoms of said M4 segment, which have the atomic coordinates that are given under the ATOM field for residue sequence numbers 303 and 307 in Table 1, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or said at least one bound compound is in a cavity located between:
- on a first side, the atoms of said M1 segment, which have the **atomic coordinates** that are given under the ATOM field for residue sequence numbers 201, 202, 205 and 206 in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and
- on a second side, the atoms of said M3 segment, which have the atomic coordinates that are given under the ATOM field for residue sequence numbers 254, 255, 258 and 259 in chain A, B, C, D or E of the PDB file accessible under identity number 3EAM as last modified 9 June 2009, respectively, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;
and
- on a third side, the atoms of said M4 segment, which have the **atomic coordinates** that are given for chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or said at least one bound compound is in a cavity located between:
- on a first side, the atoms of said M 1 segment, which have the **atomic coordinates** that are given under the ATOM field for residue sequence numbers 200, 201, 204 and 205 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and
- on a second side, the atoms of said M3 segment, which have the atomic coordinates that are given under the ATOM field for residue sequence numbers 253, 254, 257 and 259 in chain A, B, C, D or E of the PDB file accessible under identity number 3EHZ as last modified 25 August 2009, respectively, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and
- on a third side, the atoms of said M4 segment, which have the **atomic coordinates** that are given under the ATOM field for residue sequence numbers 302 and 306 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Angstrom or less.

3. The crystal of any one of claims 1-2, wherein said at least one bound compound is in a cavity located **between:**
- **said M1 segment,**
- **said M3 segment,**
- **said M4 segment, and**
- **the M2 segment and/or the** β**₆-**β**₇ loop** of said at least one GLIC monomer chain.

4. The crystal of any one of claims 1-3, wherein said at least one bound compound is in a cavity located between said M1 segment on a first side, said M3 segment on a second side, said M4 segment on a third side, and, on a fourth and/or fifth side(s):
- the amino acid of said M2 segment, which is at **position 284** in the full length amino acid sequence of said at least one GLIC monomer chain, and/or
- the atoms of said M2 segment, which have the **atomic coordinates** that are given under the ATOM field for residue sequence number 242 in **Table 1** or in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atoms of said M2 segment, which have the **atomic coordinates** that are given under the ATOM field for residue sequence number 241 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;
and/or
- the amino acids of said β₆-β₇ loop, which are at **positions 161, 162 and 163** in the full length amino acid sequence of said at least one GLIC monomer chain, and/or
- the atoms of said β₆-β₇ loop, which have the **atomic coordinates** that are given under the ATOM field for residue sequence numbers 119, 120 and 121 in **Table 1** or in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less, and/or
- the atoms of said β₆-β₇ loop, which have the **atomic coordinates** that are given under the ATOM field for residue sequence numbers 118, 119 and 120 in chain A, B, C, D or E of the PDB file accessible under identity number **3EHZ** as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

5. The crystal of any one of claims 1-4, wherein said cavity is bordered by at least six, more particularly by at least seven, still more particularly by at least eight, even still more particularly by nine hydrophobic amino acids selected along the following group:
- hydrophic amino acids, which are at positions 120, 121, 201, 202, 206, 242, 258, 259 and 303 in the full length amino acid sequence of said at least one GLIC monomer chain (*e.g*., in the GLIC monomer chain of SEQ ID NO: 1),
- hydrophic amino acids, which have the atomic coordinates that are given under the ATOM field for residue sequence numbers 78, 79, 159, 160, 164, 200, 216, 217 and 261 in Table 1 or in chain A, B, C, D or E of the PDB file accessible under identity number **3EAM** as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
- hydrophic amino acids, which have the atomic coordinates that are given under the ATOM field for residue sequence numbers 77, 78, 158, 159, 163, 199, 215, 216 and 260 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

6. The crystal of any one of claims 1-5, wherein said at least one bound compound is a neurotransmission modulator, and/or a compound, which modulates neuronal cell depolarization, and/or a compound, which modulates neuronal cell hyperpolarization, and/or a compound, which modulates the ion conductance of GLIC.

7. The crystal of any one of claims 1-6, wherein said at least one compound is a compound, which modulates the ion conductance of a vertebrate anionic or cationic pentameric Ligand-Gated Ion Channel (pLGIC), more particularly of a gamma-aminobutyric acid receptor (GABA_{A}R) and/or a glycine receptor (GlyR) and/or a nicotinic acetylcholine receptor (AChR) and/or a serotonine receptor (5HT₃-R).

8. The crystal of any one of claims 1-7, wherein said at least one bound compound is, or comprises:
- a steroid (*e.g*., a hormone), or a benzodiazepine, or an azapirone, or a piperazine and/or diphenylmethane (such as hydroxyzine or (±)-2-(2-{4-[(4-chlorophenyl)-phenylmethyl]piperazin-1-yl}ethoxy)ethanol), or an halogenated ether (*e.g*., a tetrafluoroethane, such as desflurane or 2-(difluoromethoxy)-1,1,1,2-tetrafluoro-ethane), or a propylphenol (such as propofol or 2,6-diisopropylphenol), or a carboxylated imidazole derivative (such as etomidate or ethyl 3-[(1*R*)-1-phenylethyl]imidazole-4-carboxylate), or 3-hydroxybutyric acid, or
- donepezil ((*RS*)-2-[(1-benzyl-4-piperidyl)methyl]-5,6-dimethoxy-2,3-dihydroinden-1-one), or galantamine ((4a*S*,6*R*,8a*S*)-5,6,9,10,11,12-hexahydro-3-methoxy-11-methyl-4a*H-*[1]benzofuro[3a,3,2-*ef*] [2] benzazepin-6-ol), or rivastigmine ((*S*)-*N*-Ethyl-*N*-methyl-3-[1-(dimethylamino)ethyl]-phenylcarbamate), or memantine (3,5-dimethyladamantan-1-amine), or
- an alcohol (ethanol, propanol, butanol, pentanol, hexanol, heptanol, octanol, nonanol, decanol), or
- a barbiturate (more particularly, pentobarbital).

9. The crystal of any one of claims 1-8, wherein said at least one bound compound is, or comprises the active principle (or one the active principles) of:
- a medicament for use in the treatment and/or prevention and/or palliation of a disease or disorder of the nervous system, more particularly the CNS and/or the PNS, still more particularly the CNS, even still more particularly the brain, or
- a drug for use in inducing a medically-desired temporary disorder of the nervous system.

10. The crystal of any one of claims 1-9, wherein said at least one bound compound is, or comprises the active principle (or one of the active principles) of:
- an anesthetic, and/or
- a muscle relaxant, and/or
- a sedative, and/or
- a hypnotic, and/or
- an anxiolytic, and/or
- an antipsychotic, and/or
- an antidepressant, and/or
- an anticonvulsant, and/or
- an anti-epileptic, and/or
- an anti-emetic (*e.g*., an anti-emetic intended for the treatment and/or prevention and/or palliation of motion sickness and/or of the side-effects of medications such as opioids and chemotherapy), and/or
- an amnesic, and/or
- a medicament intended for the treatment and/or prevention and/or palliation of a chemical addiction (*e.g*., nicotine addiction and/or cocaine addiction), and/or
- a nootropic drug, and/or
- an euphorisant, and/or
- a medicament intended for the treatment and/or prevention and/or palliation of toxic and/or metabolic encephalopathy(ies) (*e.g*., mercury or lead intoxication), and/or
- a medicament intended for the treatment and/or prevention and/or palliation of neurodegenerative disease(s) (*e.g*., Alzheimer's disease and/or Parkinson's disease), and/or
- a medicament intended for the treatment and/or prevention and/or palliation of diseases involving a cognitive deficit and/or the inability to focus attention (*e.g*., schizophrenia, more particularly the first stages of schizophrenia, and/or ADHD).

11. The crystal of any one of claims 1-10, wherein said at least one bound compound is **bound to:**
- the amino acid of said at least one GLIC monomer chain or fragment thereof, which is at **position 244 in the full length amino acid sequence** of said GLIC monomer chain,
and/or
- the atoms, which have the atomic coordinates that are given under the ATOM field for residue sequence number 202 in Table 1 or in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- the atoms, which have the atomic coordinates that are given under the ATOM field for residue sequence number 201 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;
**and/or is bound to:**
- the amino acid of said at least one GLIC monomer chain or fragment thereof, which is at **position 297 in the full length amino acid sequence** of said at least one GLIC monomer chain,
and/or
- the atoms, which have the atomic coordinates that are given under the ATOM field for residue sequence number 255 in Table 1 or in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- the atoms, which have the atomic coordinates that are given under the ATOM field for residue sequence number 254 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;
**and/or is bound to:**
- the amino acid of said at least one GLIC monomer chain or fragment thereof, which is at **position 300 in the full length amino acid sequence** of said at least one GLIC monomer chain,
and/or
- the atoms, which have the atomic coordinates that are given under the ATOM field for residue sequence number 258 in Table 1 or in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- the atoms, which have the atomic coordinates that are given under the ATOM field for residue sequence number 257 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;
said at least one bound compound **being optionally further bound to:**
- the amino acid of said at least one GLIC monomer chain or fragment thereof, which is at **position 243 in the full length amino acid sequence** of said at least one GLIC monomer chain,
and/or
- the atoms, which have the atomic coordinates that are given under the ATOM field for residue sequence number 201 in Table 1 or in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- the atoms, which have the atomic coordinates that are given under the ATOM field for residue sequence number 200 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less;
**and/or is optionally further bound to:**
- the amino acid of said at least one GLIC monomer chain or fragment thereof, which is at **position 284 in the full length amino acid sequence** of said at least one GLIC monomer chain,
and/or
- the atoms, which have the atomic coordinates that are given under the ATOM field for residue sequence number 242 in Table 1 or in chain A, B, C, D or E of the PDB file accessible under identity number 3EAM as last modified 9 June 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less,
and/or
- the atoms, which have the atomic coordinates that are given under the ATOM field for residue sequence number 241 in chain A, B, C, D or E of the PDB file accessible under identity number 3EHZ as last modified 25 August 2009, or coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

12. The crystal of any one of claims 1-11, wherein said at least one bound compound comprises, or consists of, atoms, which have the atomic coordinates that are given in the HETATM field of Table 1 for the residue named DES in said Table 1 or for the residue named PPL in said Table 1, or atomic coordinates which differ from these coordinates by a rmsd of the C-alpha atoms of 6 Ångstrom or less, preferably of 5 Ångstrom or less, more preferably of 4 Ångstrom or less, even more preferably of 3 Ångstrom or less, still even more preferably of 2 Ångstrom or less, most preferably of 1.5 Ångstrom or less.

13. **A method for identifying and/or developing a compound,** which is a neurotransmission modulator, and/or which (allosterically) modulates neuronal cell hyperpolarization, and/or which (allosterically) modulates neuronal cell depolarization, said method comprising:
- identifying and/or developing a ligand of the transmembrane domain of a GLIC monomer chain or a ligand of a transmembrane sub-domain thereof, which, when bound to said GLIC transmembrane domain or sub-domain:
- locates in the cavity as defined in any one of claims 1-12, and/or
- locates at the same location as the at least one bound compound as defined in any one of claims 1-12, and/or
- binds to said GLIC transmembrane domain or sub-domain at the same binding site(s) as the at least one bound compound as defined in any one of claims 1-12,
wherein said GLIC transmembrane sub-domain is a fragment of said GLIC transmembrane domain, which consists of, or comprises, the sequence extending from the first N-terminal amino acid of the segment M1 to the last C-terminal amino acid of the segment M4 of said GLIC transmembrane domain,
said identification and/or development optionally comprising, for each one of said ligands, co-crystallizing said ligand and at least one GLIC monomer chain (or a fragment thereof, which has retained said GLIC transmembrane domain or sub-domain), and determining the location of said ligand in said at least one GLIC monomer chain (or fragment thereof), and comparing the ligand location thus determined with the location of the at least one bound compound in the crystal of any one of claims 1-12,
- optionally selecting those ligands, which allosterically modulates the ion conductance of a vertebrate anionic or cationic pentameric Ligand-Gated Ion Channel (pLGIC), more particularly of a gamma-aminobutyric acid receptor (GABA_{A}R) and/or a glycine receptor (GlyR) and/or a nicotinic acetylcholine receptor (AChR) and/or a serotonine receptor (5HT₃-R), and/or
- optionally selecting those ligands, which compete with propofol and/or desflurane for binding to said GLIC transmembrane domain or sub-domain, said optional selection optionally comprising, for each one of said ligands, co-crystallizing said ligand and at least one GLIC monomer chain (or a fragment thereof, which has retained said GLIC transmembrane domain or sub-domain), determining the atomic coordinates of said ligand in the crystal thus formed, and comparing it with the atomic coordinates that are given in the HETATM field of Table 1 for the residue named DES and/or for the residue named PPL.

14. **A computer system device** for identifying or developing a compound, which is a neurotransmission modulator, and/or a compound, which (allosterically) modulates neuronal cell hyperpolarization, and/or a compound, which (allosterically) modulates neuronal cell depolarization, wherein said computer system device comprises:
- storage means storing:
- the atomic coordinates, which said at least one GLIC monomer chain, or a fragment thereof, has in the crystal of any one of claims 1-12, and storing
- the location of the GLIC cavity as defined in any one of claims 1-12 and/or the location of the at least one bound compound as defined in any one of claims 1-12, more particularly the atomic coordinates thereof, and
- software for identifying or developing, based on said coordinates and location(s), ligand(s) of the GLIC transmembrane domain or ligand(s) of a transmembrane sub-domain thereof, which, when bound to said GLIC transmembrane domain or sub-domain, locates(locate) in said cavity and/or locates at the same location as said at least one bound compound, wherein said GLIC transmembrane sub-domain is a fragment of said GLIC transmembrane domain, which consists of, or comprises, the sequence extending from the first N-terminal amino acid of the segment M1 to the last C-terminal amino acid of the segment M4 of said GLIC transmembrane domain;
said storage means optionally storing the atomic coordinates that are given in the HETATM field of Table 1 for the residue named DES and/or for the residue named PPL; said computer system device optionally further comprising:
- means displaying representations of the candidate ligand(s) or a portion thereof, and displaying representations of said GLIC monomer chain or fragment thereof, or a portion thereof; and/or
- processing means for calculating binding affinities between the candidate ligand(s), and those portion(s) of said GLIC transmembrane domain or sub-domain where said ligand(s) is(are) located in said crystal.

15. **A method for producing a medicament,** more particularly a medicament for use in the treatment and/or prevention and/or palliation of a disease or disorder of the nervous system, more particularly the CNS and/or the PNS, still more particularly the CNS, and/or a drug for use in inducing a medically-desired temporary disorder of the nervous system, said method comprising:
- identifying or developing at least one compound by the method of the invention for identifying and/or developing a compound of claim 13;
- optionally, modifying said at least one identified and/or developed compound to increase its specificity with respect to neuronal cells and/or its lipophilicity;
- optionally, testing the neurotransmission modulation effect(s) induced by said at least one identified and/or developed compound in one or several *in vitro* and/or *in vivo* assays in the presence of neuronal cells;
- formulating said at least one identified and/or developed, optionally modified, compound with a pharmaceutically acceptable carrier or diluent.

16. The method for producing a medicament or drug of claim 15, wherein said medicament or drug is:
- an anesthetic, and/or
- a muscle relaxant, and/or
- a sedative, and/or
- a hypnotic, and/or
- an anxiolytic, and/or
- an antipsychotic, and/or
- an antidepressant, and/or
- an anticonvulsant, and/or
- an anti-epileptic, and/or
- an anti-emetic (*e.g*., an anti-emetic intended for the treatment and/or prevention and/or palliation of motion sickness and/or of the side-effects of medications such as opioids and chemotherapy), and/or
- an amnesic, and/or
- a medicament intended for the treatment and/or prevention and/or palliation of a chemical addiction (*e.g*., nicotine addiction and/or cocaine addiction),
or wherein said medicament or drug is:
- a nootropic drug, and/or
- an euphorisant, and/or
- a medicament intended for the treatment and/or prevention and/or palliation of toxic and/or metabolic encephalopathy(ies) (*e.g*., mercury or lead intoxication), and/or
- a medicament intended for the treatment and/or prevention and/or palliation of neurodegenerative disease(s) (*e.g*., Alzheimer's disease and/or Parkinson's disease), and/or
- a medicament intended for the treatment and/or prevention and/or palliation of diseases involving a cognitive deficit and/or the inability to focus attention (*e.g*., schizophrenia, more particularly the first stages of schizophrenia, and/or ADHD).
